# EUROPEAN PATENT SPECIFICATION

(11) **EP 2 741 782 B1**
(45) Date of publication and mention of the grant of the patent: **06.05.2020**
(21) Application number: 12748422.8
(22) Date of filing: 10.08.2012
(51) Int. Cl.: A61K 47/64, A61K 38/22, A61K 38/25, A61K 38/27

(54) **PROTEIN CARRIER-LINKED PRODRUGS**
PROTEINTRÄGER-VERKNÜPFTE PRODRUGS
PRODROGUES LIÉS À UN SUPPORT PROTÉINIQUE

(30) Priority: 12.08.2011 EP 11177408
(43) Date of publication of application: 18.06.2014
(73) Proprietor: Ascendis Pharma A/S, 2900 Hellerup (DK)
(72) Inventor: HERSEL, Ulrich, 69121 Heidelberg (DE); VETTER, Dirk, 69121 Heidelberg (DE)
(74) Representative: Büchel, Edwin
(86) International application number: PCT/EP2012/065740
(87) International publication number: WO 2013/024049

(56) References cited:
- WO-A1-2008/155134
- WO-A1-2010/091122
- WO-A1-2010/144508
- WO-A1-2011/144756
- WO-A2-2005/099768
- SCHELLENBERGER V ET AL: "A recombinant polypeptide extends the in vivo half-life of peptides and proteins in a tunable manner", NATURE BIOTECHNOLOGY 2009 NATURE PUBLISHING GROUP USA LNKD- DOI:10.1038/NBT.1588, vol. 27, no. 12, December 2009 (2009-12), pages 1186-1190, XP002665891, ISSN: 1087-0156
- MEAGHER R J ET AL: "Sequencing of DNA by free-solution capillary electrophoresis using a genetically engineered protein polymer drag-tag", ANALYTICAL CHEMISTRY 20080415 US LNKD- DOI:10.1021/AC702591T, vol. 80, no. 8, 15 April 2008 (2008-04-15) , pages 2842-2848, XP002685541, ISSN: 0003-2700
- KUANGSHI WU ET AL: "Drug-Free Macromolecular Therapeutics: Induction of Apoptosis by Coiled-Coil-Mediated Cross-Linking of Antigens on the Cell Surface", ANGEWANDTE CHEMIE INTERNATIONAL EDITION, vol. 49, no. 8, 15 February 2010 (2010-02-15), pages 1451-1455, XP55041531, ISSN: 1433-7851, DOI: 10.1002/anie.200906232

## Description

Drugs frequently exhibit short plasma half-life due to renal and receptor-mediated clearance, aggregation, proteolytic degradation, poor bioavailability and physical properties which preclude efficient formulations. This is highly undesirable as it leads to the need for frequent and repeated administration of the drug, resulting in increased costs and inconvenience for the patient.

One mechanism for enhancing the availability of drugs is by conjugating them with derivatizing compounds, which include, for example, poly(ethylene glycol) and poly(propylene glycol). Some of the benefits recognized include lowered immunogenicity and antigenicity, increased duration of action, and altered pharmacokinetic properties (Veronese, F.M. "Enzymes for Human Therapy: Surface Structure Modifications," Chimica Oggi, 7:53-56, 1989).

To enhance physicochemical or pharmacokinetic properties of a drug *in vivo,* drugs can be bound to carriers in a non-covalent way, using physicochemical formulations of drug-solvent-carrier mixtures. However, the non-covalent approach requires a highly efficient drug encapsulation to prevent uncontrolled, burst-type release of the drug. Restraining the diffusion of an unbound, water soluble drug molecule requires strong van der Waals contacts, frequently mediated through hydrophobic moieties. Many conformationally sensitive drugs, such as proteins or peptides, are rendered dysfunctional during the encapsulation process and/or during subsequent storage of the encapsulated drug. In addition, such amino-containing drugs readily undergo side reactions with carrier degradation products. Furthermore, dependence of the release mechanism of the drug upon biodegradation may cause interpatient variability.

US2009/0298762A2 describes stable fusions of protein drugs with peptide/protein carriers comprised mainly of glycine, asparagine and glutamine. Similarly, patent EP2173890B1 describes the use of a protein sequence comprised of random coil conformations which is stably connected to protein drug moieties. However, as such conjugates do not release the protein drug moiety such conjugates need to retain sufficient activity.

Alternatively, drugs may be conjugated to a carrier via a reversible linker molecule, resulting in carrier-linked prodrugs. This approach has been applied to various classes of molecules, from so-called small molecules, through natural products up to larger peptides and proteins.

Prodrug activation may occur by enzymatic or non-enzymatic cleavage of the bond between the carrier and the drug molecule, or a sequential combination of both, i.e. an enzymatic step followed by a non-enzymatic rearrangement.

Enzymatically induced prodrug activation is characterized in that the cleavage in enzyme-free *in vitro* environment such as an aqueous buffer solution, of, e.g., an ester or amide may occur, but the corresponding rate of hydrolysis may be much too slow and not therapeutically useful. In an in-vivo environment, esterases or amidases are typically present and the esterases and amidases may cause significant catalytic acceleration of the kinetics of hydrolysis from twofold up to several orders of magnitude. Therefore, the cleavage is predominantly controlled by the enzymatic reaction.

A major drawback of predominantly enzymatic cleavage is interpatient variability. Enzyme levels may differ significantly between individuals resulting in biological variation of prodrug activation by the enzymatic cleavage. The enzyme levels may also vary depending on the site of administration. For instance it is known that in the case of subcutaneous injection, certain areas of the body yield more predictable therapeutic effects than others. To reduce this unpredictable effect, non-enzymatic cleavage or intramolecular catalysis is of particular interest.

Therefore, enzyme-independent autocatalytic cleavage of carrier and drug is preferred. In most cases this is achieved by an appropriately designed linker moiety between the carrier and the drug, which is directly attached to the functional group of a drug via a covalent bond.

A number of such enzyme-independent prodrugs suitable for different classes of biologically active moieties are known in the art. Examples can be found in the international patent applications WO-A 2005/099768, WO-A 2006/13565869, WO-A 2009/095479, and WO-A 2011/012722.

Various carrier-moieties are known to the person skilled in the art. Protein carriers have the advantage that they are - among other aspects - biodegradable and do not accumulate over time.

However, protein carriers always pose the risk of inducing undesired and harmful immune responses. The protein carriers disclosed in EP2173890B1 are not immunogenic due to their random coil confirmation, but this patent only discloses stable fusion proteins, thus restricting the use of the carrier to peptide and protein drugs and as the drug is not released in this case such fusion proteins need to retain sufficient activity to be therapeutically effective when administered to a patient in need of a corresponding drug treatment.

Therefore, it was an object of the present invention to provide novel protein carrier-linked prodrugs that at least partially overcome the above-mentioned shortcomings. This object is achieved with a protein carrier reagent of formula (A) or (B):

PC-SP-LG (A),

(PC)ₚ-SP-LG (B),

wherein
each PC individually is a protein carrier moiety,
   wherein a protein carrier moiety comprises, preferably consists of an amino acid sequence consisting of at least 100 amino acid residues, and
   wherein the amino acid sequence of at least 100 amino acid residues, preferably the protein carrier moiety, is in random coil conformation as determined by circular dichroism measurements, and,
   wherein the amino acid sequence of at least 100 amino acid residues, preferably the protein carrier moiety, comprises alanine, serine and proline residues,
SP is a spacer moiety comprising q branching points BP,
p is an integer of from 1 to 32, preferably of from 1 to 16, more preferably of from 1 to 10, even more preferably p is selected from 1, 2, 3, 4, 5, 6, 7, 8, 9, and 10,
q is an integer of from 0 to 32, preferably of from 0 to 16, more preferably of from 2 to 8, most preferably q is 3,
LG is a leaving group or an electrophile selected from formulas (a) to (f): 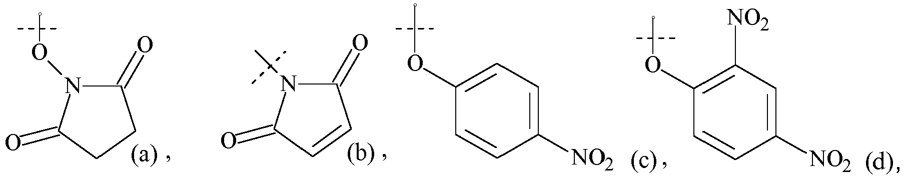 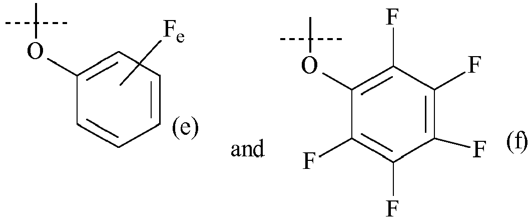 wherein
   dashed lines indicate attachment to the rest of the molecule, and
   e is 1, 2, 3 or 4.

Also disclosed are water-soluble protein carrier-linked prodrugs, wherein the prodrug is of formula (1) or (2): 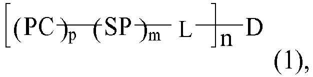 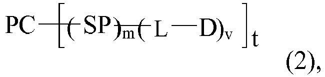 wherein
each PC individually is a protein carrier moiety,
   wherein a protein carrier moiety comprises, preferably consists of an amino acid sequence consisting of at least 100 amino acid residues, and
   wherein the amino acid sequence of at least 100 amino acid residues, preferably the protein carrier moiety, is in random coil conformation, and,
   wherein the amino acid sequence of at least 100 amino acid residues, preferably the protein carrier moiety, comprises alanine, serine and proline residues,
each SP independently is a spacer moiety comprising q branching points BP,
each L independently is a reversible prodrug linker moiety,
each D independently is a biologically active moiety,
each m is independently 0 or 1,
each n, p and v are independently an integer of from 1 to 32, preferably of from 1 to 16, more preferably of from 1 to 10, even more preferably each n, p and v are independently selected from 1, 2, 3, 4, 5, 6, 7, 8, 9, and 10,
each q is independently an integer of from 0 to 32, preferably of from 0 to 16, more preferably of from 2 to 8, most preferably q is 3,
t is an integer of from 1 to 200, preferably of from 1 to 100, more preferably of from 1 to 25, even more preferably t is 1, 2, 3, 4, 5, 6, 7, 8, 9, or 10,
or a pharmaceutically acceptable salt thereof.

It is understood that p is at least 2, if m is 1.

It was now surprisingly found that such water-soluble protein carrier-linked prodrugs can be used as sustained-release dosage forms of biologically active moieties. In addition, the polymeric moiety allows for increased water-solubility and the random coil conformation of the protein carrier reduces the risk of undesired immune responses.

Within the present invention the terms are used having the meaning as follows.

The terms "drug", "biologically active molecule", "biologically active moiety", "biologically active agent", "active agent", and "active substance" mean any substance which can affect any physical or biochemical properties of a biological organism, including viruses, bacteria, fungi, plants, animals, and humans. In particular, as used herein, the terms include any substance intended for diagnosis, cure, mitigation, treatment, or prevention of disease in organisms, in particular humans or animals, or to otherwise enhance physical or mental well-being of organisms, in particular humans or animals.

"Biologically active moiety D" means the part of the biologically active moiety-reversible prodrug linker conjugate or the part of the biologically active moiety-reversible prodrug linker-carrier conjugate, which results after cleavage in a drug D-H of known biological activity.

"Amine-containing biologically active moiety" or "hydroxyl-containing biologically active moiety" means the part (moiety or fragment) of the biologically active moiety-reversible prodrug linker conjugate or the part of the biologically active moiety-reversible prodrug linker-carrier conjugate (active agent) of (known) biological activity, and which part of the drug comprises at least one amine or hydroxyl group, respectively.

In addition, the subterm "aromatic amine-containing" means that the respective biologically active moiety D and analogously the corresponding drug D-H contain at least one aromatic fragment, which is substituted with at least one amino group. The subterm "aliphatic amine-containing" means that the amine group of the respective biologically active moiety D and analogously the corresponding drug D-H is connected to an aliphatic fragment. Without further specification the term "amine-containing" is used generically and refers to aliphatic and aromatic amine moieties.

The subterm "aromatic hydroxyl-containing" means that the respective biologically active moiety D and analogously the corresponding drug D-H contains at least one aromatic fragment, which is substituted with at least one hydroxyl group. The subterm "aliphatic hydroxyl-containing" means that the hydroxyl group of the respective biologically active moiety D and analogously the corresponding drug D-H is connected to an aliphatic fragment. Without further specification the term "hydroxyl-containing" is used generically and refers to aliphatic and aromatic hydroxyl moieties.

The term "hyperbranched" or "hyperbranched moiety" refers to a spacer moiety comprising at least one branching point. Such branching point comprises, for example, an at least 3-fold substituted carbocycle, an at least 3-fold substituted heterocycle, a tertiary carbon atom, a quaternary carbon atom or a tertiary nitrogen atom.

A carbocycle and heterocycle may be substituted by C₁₋₂₀ alkyl, optionally interrupted or terminated by heteroatoms or functional groups selected from the group consisting of -O-, -S-, N(R), C(O), C(O)N(R), and N(R)C(O), wherein R is hydrogen or a C₁₋₁₀ alkyl chain, which is optionally interrupted or terminated by one or more of the above mentioned atoms or groups which further have a hydrogen as terminal atom.

"Free form" of a drug refers to the drug in its unmodified, pharmacologically active form, such as after being released from a carrier-linked prodrug.

The phrases "in bound form" or "moiety" refer to sub-structures which are part of a molecule. The phrase "in bound form" is used to simplify reference to moieties by naming or listing reagents, starting materials or hypothetical starting materials well known in the art, and whereby "in bound form" means that for example one or more hydrogen radical(s) (-H), or one or more activating or protecting group(s) present in the reagent(s) or starting material(s) is/are not present in the moiety when part of a molecule.

To enhance physicochemical or pharmacokinetic properties of a drug *in vivo,* such drug can be conjugated with a carrier, as in the present invention. If the drug is reversibly bound to a carrier and/or a linker, as in the present invention such system is commonly assigned as "carrier-linked prodrug". According to the definitions provided by IUPAC (as given under http://www.chem.qmul.ac.uk/iupac/medchem/ah.html, accessed on March 7, 2011), a carrier-linked prodrug is a prodrug that contains a temporary linkage of a given active substance with a reversible carrier group that produces improved physicochemical or pharmacokinetic properties and that can be easily removed *in vivo,* usually by a hydrolytic cleavage.

The term "promoiety" refers to the part of the prodrug which is not the drug, thus meaning for example protein carrier(s) PC, as well as reversible prodrug linker(s) L, and/or one or more spacer moiety/moieties SP, if present.

The term "reversible prodrug linker" or "transient prodrug linkers" refers to a moiety which on its one end is attached to the biologically active moiety D through a reversible linkage and at another end is attached through a permanent bond to either a spacer moiety permanently attached to a protein carrier PC or is directly attached through a permanent bond to a a protein carrier PC. Such reversible prodrug linkers are non-enzymatically hydrolytically degradable, i.e. cleavable, under physiological conditions (aqueous buffer at pH 7.4, 37°C) with half-lives ranging from, for example, one hour to six months. Reversible linkages are, for example, aconityls, acetals, amides, carboxylic anhydrides, esters, imines, hydrazones, maleamic acid amides, ortho esters, phosphamides, phosphoesters, phosphosilyl esters, silyl esters, sulfonic esters, aromatic carbamates, and combinations thereof.

Permanent linkages are non-enzymatically hydrolytically degradable under physiological conditions (aqueous buffer at pH 7.4, 37°C) with half-lives of six months or longer, such as, for example, amides.

"Free form" of a drug refers to the drug in its unmodified, pharmacologically active form, such as after being released from a carrier-linked prodrug.

The phrases "in bound form", "connected to" or "moiety" refer to sub-structures which are part of a molecule. The phrases "in bound form" and "connected to" are used to simplify reference to moieties by naming or listing reagents, starting materials or hypothetical starting materials well known in the art, and whereby "in bound form" and "connected to" mean that for example one or more hydrogen radicals (-H), or one or more activating or protecting groups present in the reagents or starting materials are not present in the moiety when part of a molecule.

The term "functional group" refers to specific groups of atoms within molecules that can undergo characteristic chemical reactions. Examples of functional groups are hydroxyl, carbonyl, aldehyde, carboxyl, ester, ketal, hemiketal, acetal, hemiacetal, primary/secondary/tertiary amine, cyanate, disulfide, sulfhydryl, sulfonyl, phosphate.

If a functional group is coupled to another functional group, the resulting chemical structure is referred to as "linkage". For example, the reaction of an amine functional group with a carboxyl functional group results in an amide linkage. Further examples for linkages are ester, ether, ketal, acetal, secondary/tertiary amine, carboxamide, sulfide, and disulfide.

A "therapeutically effective amount" of carrier-linked prodrug as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician.

The term "branch" refers to those moieties of a branched spacer moiety that are connecting branching points or that are terminally connected to branching points.

A "traceless prodrug linker" refers to a reversible prodrug linker from which a drug is released in its free form, meaning that upon release from the promoiety the drug does not contain any traces of the promoiety.

"Non-biologically active linker" means a linker which does not show the pharmacological effects of the drug (D-H) derived from the biologically active moiety.

The term "polymer" describes a molecule comprising, in particular consisting of repeating structural units connected by chemical bonds in a linear, circular, branched, crosslinked or dendrimeric way or a combination thereof, which can be of synthetic or biological origin or a combination of both. In case of polypeptide or protein polymers, it is understood, that a polypeptide or protein polymers consists of the same or different natural or non-natural amino acids, wherein the sequence itself need not be repetetive. It is understood, that e.g. capping moieties may be present in a polymer. For example, proteins may be capped by C-terminal amidation.

The term "polymeric" refers to a moiety comprising one or more polymer(s).

A "peptide" is a single linear polymer chain of up to about 100 amino acids, preferably up to about 50 amino acids, more preferably up to about 25 amino acids bonded together by peptide bonds between the carboxyl and amino groups of adjacent amino acid residues and may be linear or branched. Preferably, a peptide is a single linear polymer chain of at least about 4 amino acids, more preferably of at least about 6 amino acids. A "protein" or "polypeptide" is a single linear polymer chain of more than about 100 amino acids bonded together by peptide bonds between the carboxyl and amino groups of adjacent amino acid residues. Proteins or polypeptides may comprise modifications, for example by C-terminal amidation.

The term "peptide fragment" as used herein refers to a polypeptide moiety or peptide moiety comprising at least 3 amino acids and comprising at least one alanine, and/or one serine and/or one proline.

The term "polymer cassette" relates to peptides of defined, individual amino acid stretches. Polymer cassettes may be used to form the protein carrier PC. Thus, a protein carrier PC comprises, preferably consists of one or more, in particular of 1, 2, 3, 4, 5, 6, 7, 8, 9, 10, 11, 12, 13, 14, 15, 16, 17, 18, 19 or 20 polymer cassette(s), which may be of the same or of different sequence.

As used herein, the term "random coil" relates to any conformation of a polymeric molecule, including proteins, in which the individual monomeric elements that form said polymeric structure are essentially randomly oriented towards the adjacent monomeric elements while still being chemically bound to said adjacent monomeric elements. In particular, a polypeptide or protein having random coil conformation substantially lacks a defined secondary and tertiary structure. The nature of polypeptide random coils and their methods of experimental identification are known to the person skilled in the art. In particular, the lack of secondary and tertiary structure of a protein may be determined by circular dichroism (CD) measurements. CD spectroscopy represents a light absorption spectroscopy method in which the difference in absorbance of right- and left-circularly polarized light by a substance is measured. The secondary structure of a protein can be determined by CD spectroscopy using far-ultraviolet spectra with a wavelength between approximately 190 and 250 nm. At these wavelengths the different secondary structures commonly found in conformations each give rise to a characteristic shape and magnitude of the CD spectrum. Accordingly, by using CD spectrometry the skilled artisan is readily capable of determining whether an amino acid polymer adopts random coil conformation at physiological conditions.

When determining whether a peptide or protein adopts random coil conformation under experimental conditions using the methods as described herein, the biophysical parameters such as temperature, pH, osmolarity and protein content may be different to the physiological conditions normally found in vivo. Temperatures between 1 °C and 42 °C or preferably 4 °C to 25 °C may be considered useful to test and/or verify the biophysical properties and biological activity of a peptide or protein under physiological conditions in vitro.

Several buffers, in particular in experimental settings (for example in the determination of protein structures, in particular in circular dichroism (CD) measurements and other methods that allow the person skilled in the art to determine the structural properties of a protein/polypeptide or peptide stretch) or in buffers, solvents and/or excipients for pharmaceutical compositions, are considered to represent "physiological solutions" or "physiological conditions" in vitro. Examples of such buffers are, e.g. phosphate-buffered saline (PBS: 115 mM NaCl, 4 mM KH₂PO₄, 16 mM Na₂HPO₄ pH 7.4), Tris buffers, acetate buffers, citrate buffers or similar buffers such as those used in the appended examples. Generally, the pH of a buffer representing physiological conditions should lie in a range from 6.5 to 8.5, preferably in a range from 7.0 to 8.0, most preferably in a range from 7.2 to 7.7 and the osmolarity should lie in a range from 10 to 1000 mmol/kg H₂O, more preferably in a range from 50 to 500 mmol/kg H₂O and most preferably in a range from 200 to 350 mmol/kg H₂O. Optionally, the protein content of a buffer representing physiological conditions may lie in a range from 0 to 100 g/l, neglecting the protein with biological activity itself, whereby typical stabilizing proteins may be used, for example human or bovine serum albumin.

Other established biophysical methods for determining random coil conformation include nuclear magnetic resonance (NMR) spectroscopy, absorption spectrometry, infrared and Raman spectroscopy, measurement of the hydrodynamic volume via size exclusion chromatography, analytical ultracentrifugation and dynamic/static light scattering as well as measurements of the frictional coefficient or intrinsic viscosity.

"Pharmaceutical composition" or "composition" means a composition containing one or more water-soluble protein carrier-linked prodrug, and optionally one or more excipients, as well as any product which results, directly or indirectly, from combination, complexation and/or aggregation of any two or more of the excipients, or from dissociation of one or more of the excipients, or from other types of reactions or interactions of one or more of the excipients.

Accordingly, the pharmaceutical compositions encompass any composition obtainable by admixing a water-soluble protein carrier-linked prodrug and at least one pharmaceutically acceptable excipient. Optionally, the pharmaceutical composition may comprise one or more other drug(s) and/or prodrug(s).

The term "excipient" refers to a diluent, adjuvant, or vehicle with which the water-soluble protein carrier-linked prodrug is administered. Such pharmaceutical excipients can be sterile liquids, such as water and oils, including those of petroleum, animal, vegetable or synthetic origin, including peanut oil, soybean oil, mineral oil, and sesame oil. Water is a preferred excipient when the pharmaceutical composition is administered orally. Saline and aqueous dextrose are preferred excipients when the pharmaceutical composition is administered intravenously. Saline solutions and aqueous dextrose and glycerol solutions are preferably employed as liquid excipients for injectable solutions. Suitable pharmaceutical excipients include starch, glucose, lactose, sucrose, mannitol, trehalose, gelatin, malt, rice, flour, chalk, silica gel, sodium stearate, glycerol monostearate, talc, sodium chloride, dried skim milk, glycerol, propylene, glycol, water, and ethanol. The composition, if desired, can also contain minor amounts of wetting or emulsifying agents, pH buffering agents, like, for example, acetate, succinate, tris, carbonate, phosphate, HEPES (4-(2-hydroxyethyl)-1-piperazineethanesulfonic acid), MES (2-(*N*-morpholino)ethanesulfonic acid), or can contain detergents, like Tween, poloxamers, poloxamines, CHAPS, Igepal, or amino acids like, for example, glycine, lysine, or histidine. These compositions can take the form of solutions, suspensions, emulsions, tablets, pills, capsules, powders, and sustained-release formulations. The composition can be formulated as a suppository, with traditional binders and excipients such as triglycerides. Oral formulation can include standard excipients such as pharmaceutical grades of mannitol, lactose, starch, magnesium stearate, sodium saccharine, cellulose, magnesium carbonate, etc. Examples of suitable pharmaceutical excipients are described in "Remington's Pharmaceutical Sciences" by E.W. Martin. Such compositions will contain a diagnostically and/or therapeutically effective amount of the water-soluble protein carrier-linked prodrug, preferably in purified form, together with a suitable amount of excipient so as to provide the form for proper administration to the patient. The formulation should suit the mode of administration.

The term "pharmaceutically acceptable" means approved by a regulatory agency such as the EMEA (Europe) and/or the FDA (US) and/or any other national regulatory agency for use in animals, preferably in humans.

"Dry composition" means that the pharmaceutical composition comprising water-soluble protein carrier-linked prodrug is provided in a dry form in a container. Suitable methods for drying are spray-drying and lyophilization (freeze-drying). Such dry composition of water-soluble protein carrier-linked prodrug has a residual water content of a maximum of 10 %, preferably less than 5% and more preferably less than 2% (determined according to Karl Fischer). The preferred method of drying is lyophilization.

"Lyophilized composition" means that the pharmaceutical composition comprising water-soluble protein carrier-linked prodrug was first frozen and subsequently subjected to water reduction by means of reduced pressure. This terminology does not exclude additional drying steps which may occur in the manufacturing process prior to filling the composition into the final container.

"Lyophilization" (freeze-drying) is a dehydration process, characterized by freezing a composition and then reducing the surrounding pressure and, optionally, adding heat to allow the frozen water in the composition to sublime directly from the solid phase to gas. Typically, the sublimed water is collected by desublimation.

"Alkyl" means a straight-chain (linear, unbranched) or branched carbon chain (unsubstituted alkyl). Optionally, one or more hydrogen atom(s) of an alkyl carbon may be replaced by a substituent as indicated herein. In general, a preferred alkyl is C₁₋₆ alkyl.

"C₁₋₄ alkyl" means an alkyl chain having 1 to 4 carbon atoms (unsubstituted C₁₋₄ alkyl), e.g. if present at the end of a molecule: methyl, ethyl, n-propyl, isopropyl, n-butyl, isobutyl, sec-butyl tert-butyl, or e.g. -CH₂-, -CH₂-CH₂-, -CH(CH₃)-, -CH₂-CH₂-CH₂-, -CH(C₂H₅)-, -C(CH₃)₂-, when two moieties of a molecule are linked by the alkyl group (also referred to as C₁₋₄ alkylene). Optionally, one or more hydrogen atom(s) of a C₁₋₄ alkyl carbon may be replaced by a substituent as indicated herein. Accordingly, "C₁₋₅₀ alkyl" means an alkyl chain having 1 to 50 carbon atoms.

"C₁₋₆ alkyl" means an alkyl chain having 1 - 6 carbon atoms, e.g. if present at the end of a molecule: C₁₋₄ alkyl, methyl, ethyl, n-propyl, isopropyl, n-butyl, isobutyl, sec-butyl, tert-butyl, n-pentyl, n-hexyl, or e.g. -CH₂-, -CH₂-CH₂-, -CH(CH₃)-, -C(CH₂)-, -CH₂-CH₂-CH₂-, -CH(C₂H₅)-, -C(CH₃)₂-, when two moieties of a molecule are linked by the alkyl group (also referred to as C₁₋₆ alkylene). One or more hydrogen atom(s) of a C₁₋₆ alkyl carbon may be replaced by a substituent as indicated herein. The terms C₁₋₁₅ alkyl or C₁₋₁₅ alkylene are defined accordingly.

"C₂₋₆ alkenyl" means an alkenyl chain having 2 to 6 carbon atoms, e.g. if present at the end of a molecule: -CH=CH₂, -CH=CH-CH₃, -CH₂-CH=CH₂, -CH=CH-CH₂-CH₃, -CH=CH-CH=CH₂, or e.g. -CH=CH-, when two moieties of a molecule are linked by the alkenyl group. One or more hydrogen atom(s) of a C₂₋₆ alkenyl carbon may be replaced by a substituent as indicated herein.

The term C₂₋₄ alkenyl is defined accordingly.

"C₂₋₆ alkynyl" means an alkynyl chain having 2 to 6 carbon atoms, e.g. if present at the end of a molecule: -C≡CH, -CH₂-C≡CH, CH₂-CH₂-C≡CH, CH₂-C≡C-CH₃, or e.g. -C≡C- when two moieties of a molecule are linked by the alkynyl group. One or more hydrogen atom(s) of a C₂₋₆ alkynyl carbon may be replaced by a substituent as indicated herein. The terms "C₂₋₄ alkynyl" and "C₂₋₁₀" are defined accordingly.

"C₂₋₅₀ alkenyl" means a branched or unbranched alkenyl chain having 2 to 50 carbon atoms (unsubstituted C₂₋₅₀ alkenyl), e.g. if present at the end of a molecule: -CH=CH₂, -CH=CH-CH₃, -CH₂-CH=CH₂, -CH=CH-CH₂-CH₃, -CH=CH-CH=CH₂, or e.g. -CH=CH-, when two moieties of a molecule are linked by the alkenyl group. Optionally, one or more hydrogen atom(s) of a C₂₋₅₀ alkenyl carbon may be replaced by a substituent as further specified. Accordingly, the term "alkenyl" relates to a carbon chain with at least one carbon carbon double bond. Optionally, one or more triple bonds may occur. The term "C₂₋₁₅ alkenyl" is defined accordingly.

"C₂₋₅₀ alkynyl" means a branched or unbranched alkynyl chain having 2 to 50 carbon atoms (unsubstituted C₂₋₅₀ alkynyl), e.g. if present at the end of a molecule: -C=CH, -CH₂-C≡CH, CH₂-CH₂-C≡CH, CH₂-C≡C-CH₃, or e.g. -C=C- when two moieties of a molecule are linked by the alkynyl group. Optionally, one or more hydrogen atom(s) of a C₂₋₅₀ alkynyl carbon may be replaced by a substituent as further specified. Accordingly, the term "alkynyl" relates to a carbon chain with at least one carbon triple bond. Optionally, one or more double bonds may occur. The term "C₂₋₆ alkenyl" is defined accordingly.

"C₃₋₇ cycloalkyl" or "C₃₋₇ cycloalkyl ring" means a cyclic alkyl chain having 3 to 7 carbon atoms, which may have carbon-carbon double bonds being at least partially saturated (unsubstituted C₃₋₇ cycloalkyl), e.g. cyclopropyl, cyclobutyl, cyclopentyl, cyclohexyl, cyclohexenyl, cycloheptyl. Optionally, one or more hydrogen atom(s) of a cycloalkyl carbon may be replaced by a substituent as indicated herein. The term "C₃₋₇ cycloalkyl" or "C₃₋₇ cycloalkyl ring" also includes bridged bicycles like norbonane (norbonanyl) or norbonene (norbonenyl). Accordingly, "C₃₋₅ cycloalkyl" means a cycloalkyl having 3 to 5 carbon atoms. Accordingly, "C₃₋₁₀ cycloalkyl" means a cycloalkyl having 3 to 10 carbon atoms.

"Halogen" means fluoro, chloro, bromo or iodo. It is generally preferred that halogen is fluoro or chloro.

"4 to 7 membered heterocyclyl" or "4 to 7 membered heterocycle" means a ring with 4, 5, 6 or 7 ring atoms that may contain up to the maximum number of double bonds (aromatic or nonaromatic ring which is fully, partially or un-saturated) wherein at least one ring atom up to 4 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom (unsubstituted 4 to 7 membered heterocyclyl). For the sake of completeness it is indicated that in some embodiments of the present invention, 4 to 7 membered heterocyclyl has to fulfill additional requirements. Examples for a 4 to 7 membered heterocycles are azetidine, oxetane, thietane, furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, diazepane, azepine or homopiperazine. Optionally, one or more hydrogen atom(s) of a 4 to 7 membered heterocyclyl may be replaced by a substituent.

"8 to 11 membered heterobicyclyl" or "8 to 11 membered heterobicycle" means a heterocyclic system of two rings with 8 to 11 ring atoms, where at least one ring atom is shared by both rings and that may contain up to the maximum number of double bonds (aromatic or nonaromatic ring which is fully, partially or un-saturated) wherein at least one ring atom up to 6 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom (unsubstituted 8 to 11 membered heterobicyclyl). Examples for a 8 to 11 membered heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine or pteridine. The term 8 to 11 membered heterobicycle also includes spiro structures of two rings like 1,4-dioxa-8-azaspiro[4.5]decane or bridged heterocycles like 8-aza-bicyclo[3.2.1]octane. The term "9 to 11 membered heterobicyclyl" or "9 to 11 membered heterobicycle" is defined accordingly.

The term "aliphatic" means fully saturated.

The term "interrupted" means that between two carbon atoms of, for example, a linker or a spacer or at the respective end of the carbon chain between the respective carbon atom and the hydrogen atom a group (such a -O- or -NH-) is inserted.

In general the term "substituted" preferably refers to substituents, which are the same or different and which are independently selected from the group consisting of halogen, CN, COOR^{b9}, OR^{b9}, C(O)R^{b9}, C(O)N(R^{b9}R^{b9a}), S(O)₂N(R^{b9}R^{b9a}), S(O)N(R^{b9}R^{b9a}), S(O)₂R^{b9}, S(O)R^{b9}, N(R^{b9})S(O)₂N(R^{b9a}R^{b9b}), SR^{b9}, N(R^{b9}R^{b9a}), NO₂, OC(O)R^{b9}, N(R^{b9})C(O)R^{b9a}, N(R^{b9})S(O)₂R^{b9a}, N(R^{b9})S(O)R^{b9a}, N(R^{b9})C(O)OR^{b9a}, N(R^{b9})C(O)N(R^{b9a}R^{b9b}), OC(O)N(R^{b9}R^{b9a}), T^{b}, C₁₋₅₀ alkyl, C₂₋₅₀ alkenyl, and C₂₋₅₀ alkynyl,
wherein T^{b}, C₁₋₅₀ alkyl, C₂₋₅₀ alkenyl, and C₂₋₅₀ alkynyl are optionally substituted with one or more R^{b10}, which are the same or different, and wherein C₁₋₅₀ alkyl; C₂₋₅₀ alkenyl; and C₂₋₅₀ alkynyl are optionally interrupted by one or more groups selected from the group consisting of T^{b}, -C(O)O-; -O-; -C(O)-; -C(O)N(R^{b11})-; -S(O)₂N(R^{b11})-; -S(O)N(R^{b11})-; -S(O)₂-; -S(O)-; -N(R^{b11})S(O)₂N(R^{b11a})-; -S-; -N(R^{b11})-; -OC(O)R^{b11}; -N(R^{b11})C(O)-; -N(R^{b11})S(O)₂-; -N(R^{b11})S(O)-; -N(R^{b11})C(O)O-; -N(R^{b11})C(O)N(R^{b11a})-, and -OC(O)N(R^{b11}R^{b11a}),
R^{b9}, R^{b9a}, R^{b9b} are independently selected from the group consisting of H; T^{b}; and C₁₋₅₀ alkyl; C₂₋₅₀ alkenyl; and C₂₋₅₀ alkynyl,
   wherein T^{b}, C₁₋₅₀ alkyl, C₂₋₅₀ alkenyl, and C₂₋₅₀ alkynyl are optionally substituted with one or more R^{b10}, which are the same or different, and wherein C₁₋₅₀ alkyl; C₂₋₅₀ alkenyl; and C₂₋₅₀ alkynyl are optionally interrupted by one or more groups selected from the group consisting of T^{b}, -C(O)O-, -O-, -C(O)-, -C(O)N(R^{b11})-, -S(O)₂N(R^{b11})-, -S(O)N(R^{b11})-, -S(O)₂-, -S(O)-, -N(R^{b11})S(O)₂N(R^{b11a})-, -S-, -N(R^{b11})-, -OC(O)R^{b11}, -N(R^{b11})C(O)-, -N(R^{b11})S(O)₂-, -N(R^{b11})S(O)-, -N(R^{b11})C(O)O-, -N(R^{b11})C(O)N(R^{b11a})-, and -OC(O)N(R^{b11}R^{b11a}),
   T^{b} is selected from the group consisting of phenyl, naphthyl, indenyl, indanyl, tetralinyl, C₃₋₁₀ cycloalkyl, 4- to 7-membered heterocyclyl, and 9- to 11-membered heterobicyclyl, wherein T^{b} is optionally substituted with one or more R^{b10}, which are the same or different,
   R^{b10} is halogen, CN, oxo (=O), COOR^{b12}, OR^{b12}, C(O)R^{b12}, C(O)N(R^{b12}R^{b12a}), S(O)₂N(R^{b12}R^{b12a}), S(O)N(R^{b12}R^{b12a}), S(O)₂R^{b12}, S(O)R^{b12}, N(R^{b12})S(O)₂N(R^{b12a}R^{b12b}), SR^{b12}, N(R^{b12}R^{b12a}), NO₂, OC(O)R^{b12}, N(R^{b12})C(O)R^{b12a}, N(R^{b12})S(O)₂R^{b12a}, N(R^{b12})S(O)R^{b12a}, N(R^{b12})C(O)OR^{b12a}, N(R^{b12})C(O)N(R^{b12a}R^{b12b}), OC(O)N(R^{b12}R^{b12a}), or C₁₋₆ alkyl, wherein C₁₋₆ alkyl is optionally substituted with one or more halogen, which are the same or different,
   R^{b11}, R^{b11a}, R^{b12}, R^{b12a}, R^{b12b} are independently selected from the group consisting of H; or C₁₋₆ alkyl, wherein C₁₋₆ alkyl is optionally substituted with one or more halogen, which are the same or different.

The term "interrupted" means that between two carbons a group is inserted or that at the end of the carbon chain between the carbon and hydrogen.

In general the term "comprise" or "comprising" also encompasses "consist of' or "consisting of".

In the following section the invention is described in further detail.

Also disclosed are water-soluble protein carrier-linked prodrugs, wherein the prodrug is of formula (1) or (2): 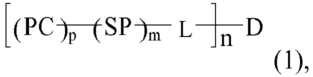 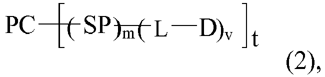 wherein
each PC individually is a protein carrier moiety,
   wherein a protein carrier moiety comprises, preferably consists of an amino acid sequence consisting of at least 100 amino acid residues, and
   wherein the amino acid sequence of at least 100 amino acid residues, preferably the protein carrier moiety, is in random coil conformation, and,
   wherein the amino acid sequence of at least 100 amino acid residues, preferably the protein carrier moiety, comprises alanine, serine and proline residues,
each SP independently is a spacer moiety comprising q branching points BP,
each L independently is a reversible prodrug linker moiety,
each D independently is a biologically active moiety,
each m is independently 0 or 1,
each n, p and v are independently an integer of from 1 to 32, preferably of from 1 to 16, more preferably of from 1 to 10, even more preferably each n, p and v are independently selected from 1, 2, 3, 4, 5, 6, 7, 8, 9, and 10,
each q is independently an integer of from 0 to 32, preferably of from 0 to 16, more preferably of from 2 to 8, most preferably q is 3,
t is an integer of from 1 to 200, preferably of from 1 to 100, more preferably of from 1 to 25, even more preferably t is 1, 2, 3, 4, 5, 6, 7, 8, 9, or 10,
or a pharmaceutically acceptable salt thereof.

It is understood that p is at least 2, if m is 1.
In a preferred embodiment m is 1.

The terms "spacer", "spacer group", "spacer molecule", and "spacer moiety" are used interchangeably and refer to any moiety suitable for connecting two moieties. Preferably, a spacer moiety is selected from C₁₋₅₀ alkyl, C₂₋₅₀ alkenyl and C₂₋₅₀ alkynyl, which moiety is optionally interrupted by one or more groups selected from -NH-, -N(C₁₋₄ alkyl)-, -O-, -S-, -C(O)-, -C(O)NH-, -C(O)N(C₁₋₄ alkyl)-, -O-C(O)-, -S(O)-, -S(O)₂-, 4- to 7-membered heterocyclyl, phenyl, naphthyl, at least 3-fold substituted carbocycles, at least 3-fold substituted heterocycles, a tertiary carbon atom, a quaternary carbon atom and a tertiary nitrogen atom, and which moiety is optionally substituted. In one preferred embodiment, the moiety is substituted and/or interrupted in a way to generate one or more branching points. Suitable interrupting groups for generating one or more branching points are, for example, at least 3-fold substituted carbocycles, at least 3-fold substituted heterocycles, a tertiary carbon atom, a quaternary carbon atom and a tertiary nitrogen atom. Such spacer moieties are preferred spacer moieties SP.

In an even more preferred embodiment a moiety SP comprises, more preferably consists of an amino acid, peptide, or polypeptide, even more preferably consists of lysine, dilysine, trilysine, tetralysine, pentalysine, hexalysine, heptalysine, octalysine, nonalysine, decalysine, undecalysine, dodecalysine, tridecalysine, tetradecalysine, pentadecalysine, hexadecalysine, heptadecalysine, octadecalysine, nonadecalysine, di(glutamic acid), tri(glutamic acid), tetra(glutamic acid), penta(glutamic acid), hexa(glutamic acid), hepta(glutamic acid), octa(glutamic acid), nona(glutamic acid), deca(glutamic acid), undeca(glutamic acid), dodeca(glutamic acid), trideca(glutamic acid), tetradeca(glutamic acid), pentadeca(glutamic acid), hexadeca(glutamic acid), heptadeca(glutamic acid), octadeca(glutamic acid), nonadeca(glutamic acid), di(aspartic acid), tri(aspartic acid), tetra(aspartic acid), penta(aspartic acid), hexa(aspartic acid), hepta(aspartic acid), octa(aspartic acid), nona(aspartic acid), deca(aspartic acid), undeca(aspartic acid), dodeca(aspartic acid), trideca(aspartic acid), tetradeca(aspartic acid), pentadeca(aspartic acid), hexadeca(aspartic acid), heptadeca(aspartic acid), octadeca(aspartic acid), nonadeca(aspartic acid), all in bound form.

In one embodiment, a spacer moiety SP comprises, in particular has, q branching points BP, wherein q is an integer from 0 to 32, preferably from 0 to 16, more preferably from 2 to 8, and most preferably q is 3. Each branching point BP may be the same or different and preferably all branching points BP are the same. From each branching point BP extend r branches, wherein r is 2, 3, or 4, preferably r is 3 or 4.

In one embodiment, q is not 0. In such embodiment, the spacer moiety SP in a water-soluble protein carrier-linked prodrug is branched. A branching point BP may be any chemical entity which allows the branching of a chain. Preferably, the q branching points in a spacer moiety are independently selected from the group consisting of at least 3-fold substituted carbocycles, at least 3-fold substituted heterocycles, a tertiary carbon atom, a quaternary carbon atom and a tertiary nitrogen atom.

A carbocycle and heterocycle may be substituted by C₁₋₂₀ alkyl, optionally interrupted or terminated by heteroatoms or functional groups selected from the group consisting of -O-, -S-, N(R), C(O), C(O)N(R), and N(R)C(O), wherein R is hydrogen or a C₁₋₁₀ alkyl chain, which is optionally interrupted or terminated by one or more of the above mentioned atoms or groups which further have a hydrogen as terminal atom.

In another embodiment, q in formula (1) is 0. In a further embodiment, q in formula (2) is 0. In such embodiments the spacer moiety is not branched.

The branches of a moiety SP are suitable for connecting p protein carrier moieties PC in formula (1) to the biologically active moiety D, via a reversible prodrug linker moiety L.

In a preferred embodiment, a moiety PC comprises, preferably consists of, an amino acid sequence of at least 100 amino acid residues.

In another preferred embodiment, a moiety PC is in random coil conformation.

In another preferred embodiment, a moiety PC comprises, in particular consists of alanine, serine and proline residues.
A moiety PC comprises, preferably consists of, an amino acid sequence of at least 100 amino acid residues, and
wherein the amino acid sequence of at least 100 amino acid residues, preferably a moiety PC, is in random coil conformation as determined by circular dichroism measurements, and,
wherein the amino acid sequence of at least 100 amino acid residues, preferably a moiety PC, comprises alanine, serine and proline residues.

A moiety PC is composed of an amino acid sequence comprising at least 100 amino acid residues, consisting of alanine, serine and proline residues which have a random coil conformation at physiological conditions. It is understood a moiety PC may transiently or temporarily not form a random coil, for example when present in a lyophilisate or dried composition.

In one embodiment a moiety PC has a random coil conformation with an amino acid sequence of maximally 3000 amino acid residues, preferably of maximally 1500 amino acid residues, more preferably of maximally 900 amino acid residues, even more preferably of maximally 700 amino acid residues, particularly preferably of maximally 600 amino acid residues. Thus, the amino acid sequence forming random coil conformation is maximally 500 amino acid residues or of maximally 450 amino acid residues in length.

Accordingly, a moiety PC, in particular the amino acid sequence forming random coil conformation of the protein carrier moiety is 100 to 3000 amino acid residues in length.

In particular embodiments said amino acid sequence forming random coil conformation of 100 to 1000 amino acid residues is as characterized herein, i.e. comprising alanine, serine and proline as main or unique residues as defined below.

A moiety PC consists mainly of the three amino acid residues alanine, serine and proline, and wherein all three amino acids are present in a moiety PC, whereby proline residues constitute more than 4 % and less than 40 % of a moiety PC. The alanine and serine residues preferably comprise the remaining at least 60 % to 96 % of a moiety PC. However, as will be detailed herein below said a moiety PC may also comprise further amino acids differing from alanine, serine, and proline, i.e. as minor constituents.

The term "minor constituent" as used herein means that maximally 10 % (i.e. maximally 10 of 100 amino acids) may be different from alanine, serine and proline, preferably maximally 8 % (i.e. maximally 8 of 100 amino acids) may be different than alanine, serine and proline, more preferably maximally 6 % (i.e. maximally 6 of 100 amino acids) may be different from alanine, serine and proline, even more preferably maximally 5 % (i.e. maximally 5 of 100 amino acids) may be different from alanine, serine and proline, particularly preferably maximally 4 % (i.e. maximally 4 of 100 amino acids) may be different from alanine, serine and proline, more particularly preferably maximally 3 % (i.e. maximally 3 of 100 amino acids) may be different from alanine, serine and proline, even more particularly preferably maximally 2 % (i.e. maximally 2 of 100 amino acids) may be different from alanine, serine and proline and most preferably maximally 1 % (i.e. maximally 1 of 100 of the amino acids) that encode the protein carrier PC may be different from alanine, serine and proline. Said amino acids different from alanine, serine and proline may be selected from the group of natural or proteinogenic amino-acids consisting of Arg, Asn, Asp, Cys, Gln, Glu, Gly, His, Ile, Leu, Lys, Met, Phe, Thr, Trp, Tyr, and Val. Minor constituents may also be selected from non-naturally occurring amino acids, such as, for example, hydroxyproline or selenomethionine or other modified natural amino acids.

The term "at least about 100/150/200/250/300/300/350 (etc) amino acid residues" is not limited to the concise number of amino acid residues but also comprises amino acid stretches that comprise an additional 10 % to 20 % or comprise 10 % to 20 % less residues. For example "at least about 100 amino acid residues" may also comprise 80 to 100 and about 100 to 120 amino acid residues without deferring from the gist of the present invention.

In one embodiment, a moiety PC comprises a plurality of amino acids repeats, wherein said repeats consist of Ala, Ser, and Pro residues, and wherein no more than 6 consecutive amino acid residues of the carrier moiety PC are identical and wherein said proline residues constitute more than 4 % and less than 40 % of the amino acids of said moiety PC.

Also disclosed is a water-soluble protein carrier-linked prodrug or pharmaceutically acceptable salt thereof, wherein a moiety PC comprises, preferably consists of a plurality of amino acid repeats,
wherein said repeats consist of Ala, Ser, and Pro residues,
and wherein no more than 6 consecutive amino acid residues of a moiety PC are identical.

In a preferred embodiment, said proline residues constitute more than 4 % and less than 40 % of the amino acids of a moiety PC.

In a further preferred embodiment, a moiety PC comprises, in particular consists of an amino acid sequence of about 100 to 3000 amino acid residues forming random coil conformation.

A moiety PC may comprise a plurality of identical polymer cassettes or a plurality of non-identical polymer casettes. Non-limiting examples of polymer cassettes consisting of Ala, Ser and/or Pro residues are provided herein below; see SEQ ID NO:9, SEQ ID NO:10, SEQ ID NO:11, SEQ ID NO:12, SEQ ID NO:13 and SEQ ID NO:14 or peptide fragments or multimers of these sequences. A polymer cassette may consist of at least 3, 4, 5, 6, 7, 8, 9, 10, 11, 12, 13, 14, 15, 16, 17, 18, 19, 20, 21, 22, 23, 24, 25, 26, 27, 28, 29, 30 or more amino acid residues, wherein each polymer cassette comprises (an) Ala, Ser, and/or Pro residue(s), preferably (an) Ala, Ser, and Pro residue(s).

In one embodiment, the polymer cassette according to the present invention does not comprise more than 100 amino acid residues. Preferably, a polymer cassette as defined herein comprises more than about 4 %, preferably more than about 5 %, even more preferably more than about 6%, particularly preferably more than about 8 %, more particularly preferably more than about 10 %, even more particularly preferably more than about 15 % and most preferably more than about 20 % proline residues. Such polymer cassette as defined herein preferably comprises less than about 40 % or less than about 35 % proline residues.

In one embodiment a PC is of formula (a):

Serₓ[Ala_{y} Ser_{z}]ᵥ (a),

which formula further comprises proline residues as defined herein and wherein
x is independently selected from integer 0 to 6,
each y is independently selected from integer ranging of from 1 to 6,
each z is independently selected from integer ranging of from 1 to 6.
v is any integer so that a moiety PC consists of at least about 100 amino acid residues, and in particular of at least about 100 to about 3000 amino acid residues, preferably to about 2000 and more preferably to about 1000 amino acid residues.

In one embodiment, all y and z of the v Ala_{y} Ser_{z} monomer moieties are identical. In another embodiment, the y and z of the v Ala_{y} Ser_{z} monomer moieties are different.

In preferred embodiments, a moiety PC comprises no more than 5 identical consecutive amino acid residues, more preferably no more than 4 identical consecutive amino acid residues and most preferably no more than 3 identical consecutive amino acid residues.

As already indicated herein above, a moiety PC comprises proline residues, wherein said proline residues constitute more than about 4 %, preferably more than about 5 %, even more preferably more than about 6 %, particularly preferably more than about 8 %, more particularly preferably more than about 10 %, even more particularly preferably more than about 15 % and most preferably more than about 20 % of the amino acids constituting a moiety PC. Such proline residues may be introduced at any position in formula (a). Preferably, the proline residues may be present in one or more of the v Ala_{y} Ser_{z} monomers of formula (a), and they may be present at the same or at different positions.

In another preferred embodiment, a moiety PC comprises more than 4 % but less than 50 %, preferably more than 10 % but less than 50 % and most preferably more than 20 % but less than 50 % alanine residues of the amino acids constituting a moiety PC.

In a further preferred embodiment, a moiety PC comprises more than 4 % and less than 50 %, preferably more than 10 % but less than 50 % and most preferably more than 20 % but less than 50 % serine residues of the amino acids constituting a moiety PC.

Accordingly, a moiety PC comprises 35 % proline residues, 50 % alanine residues and 15 % serine residues of the amino acids constituting a moiety PC. Alternatively, a moiety PC may comprise 35 % proline residues, 15 % alanine residues and 50 % serine residues of the amino acids constituting a moiety PC.

Preferably, a moiety PC is comprises one or more of the following alanine-serine polymer cassettes:
SEQ ID NO:1
   AAAASSASSASSSSSAAASA
SEQ ID NO:2
   AASAAASSAAASAAAASASS
SEQ ID NO:3
   ASASASASASASSAASAASA
SEQ ID NO:4
   SAASSSASSSSAASSASAAA
SEQ ID NO:5
   SSSSAASAASAAAAASSSAS
SEQ ID NO:6
   SSASSSAASSSASSSSASAA
SEQ ID NO:7
   SASASASASASAASSASSAS
   and
SEQ ID NO:8
   ASSAAASAAAASSAASASSS
provided that a moiety PC further comprises proline residues as described herein.

The multimers of these alanine-serine polymer cassettes may form random coil conformation in case the resulting amino acid sequence further comprises proline residues as defined herein above.

In a preferred embodiment, a moiety PC comprises, preferably consists of, one or more of the following polymer cassettes:
SEQ ID NO:9
   ASPAAPAPASPAAPAPSAPA
SEQ ID NO: 10
   AAPASPAPAAPSAPAPAAPS
SEQ ID No:11
   APSSPSPSAPSSPSPASPSS
   and
SEQ ID NO: 15
   SAPSSPSPSAPSSPSPASPS.

SEQ ID NO:15 corresponds to the herein provided SEQ ID No:11 in a circularly permuted form, wherein the last serine was removed and another serine was appended as starting amino acid. As a consequence, multimers of this modified sequence possess essentially the same internal repeating unit as multimers of the non-modified sequence, except for the very first and the very last residue. Accordingly, SEQ ID NO:15 may be considered as an example of a further polymer cassette for a moiety PC. It is clear for the person skilled in the art that also other polymer cassettes and (shorter) peptide fragments or circularly permuted versions of the herein provided amino acid polymers may be used as polymer cassettes for a moiety PC.

Yet, even further and illustrative amino acid polymers forming random coil conformation may comprise amino acid sequences that may be selected from the group consisting of:
SEQ ID NO:12
   SSPSAPSPSSPASPSPSSPA,
SEQ ID NO: 13
   AASPAAPSAPPAAASPAAPSAPPA, and
SEQ ID NO: 14
   ASAAAPAAASAAASAPSAAA.

Therefore, preferred polymer cassettes for PC are selected from the following sequences:
ASPAAPAPASPAAPAPSAPA (SEQ ID NO:9),
AAPASPAPAAPSAPAPAAPS (SEQ ID NO:10),
APSSPSPSAPSSPSPASPSS (SEQ ID NO:11),
SSPSAPSPSSPASPSPSSPA (SEQ ID NO:12),
AASPAAPSAPPAAASPAAPSAPPA (SEQ ID NO:13), and
ASAAAPAAASAAASAPSAAA (SEQ ID NO:14);
or circular permuted versions or (a) multimer(s) of these sequences as a whole or parts of these sequences.

In one embodiment, a moiety PC comprises at least one amino acid sequence selected from the group consisting of:
ASPAAPAPASPAAPAPSAPA (SEQ ID NO:9),
AAPASPAPAAPSAPAPAAPS (SEQ ID NO:10),
APSSPSPSAPSSPSPASPSS (SEQ ID NO:11),
SSPSAPSPSSPASPSPSSPA (SEQ ID NO:12),
AASPAAPSAPPAAASPAAPSAPPA (SEQ ID NO:13), and
ASAAAPAAASAAASAPSAAA (SEQ ID NO:14);
and circular permuted versions or (a) multimer(s) of these sequences as a whole or parts of these sequences.

Again, also (a) peptide fragment(s) or (a) multimer(s) or circularly permuted versions of these sequences and the sequences provided herein above may be employed in context of the present invention as polymer cassettes for a moiety PC.

Accordingly, the exemplified polymer cassettes may also provide for individual peptide fragments which may be newly combined to form further polymer cassettes.

In accordance with the above, a moiety PC may comprise a multimer consisting of either one of the amino acid sequences with SEQ ID NO:9, 10, 11, 12, 13 or 14 as disclosed herein above or may comprise a multimer consisting of more than one of amino acid sequences SEQ ID NO:9, 10, 11, 12, 13 and 14. Furthermore, it is envisaged that also peptide fragments or circularly permuted versions of these exemplified sequences may be used to build up further polymer cassettes of a moiety PC.

In another embodiment, a moiety PC may comprise a multimer comprising, preferably consisting of a (circular) permutation of the amino acid sequence selected from the group consisting of SEQ ID NOs:9, 10, 11, 12, 13, 14, 15 and (a) multimers(s) of these (circular) permutated sequences.

In yet another embodiment, a moiety PC may comprise, preferably consist of a multimer consisting of a peptide fragment/part of the amino acid sequence selected from the group consisting of SEQ ID NO: 9, 10, 12, 13, 14, 15 and (a) multimers(s) of these exemplified polymer cassettes.

Peptide fragments of these sequences to be employed for the generation of a moiety PC may consist of at least 3, preferably of at least 4, more preferably of at least 5, even more preferably of at least 6, still more preferably of at least 8, particularly preferably of at least 10, more particularly preferably of at least 12, even more particularly preferably of at least 14, preferably of at least 6, still more preferably of at least 8, particularly preferably of at least 10, more particularly preferably of at least 12, even more particularly preferably of at least 14, even more particularly preferably of at least 16, and most preferably of at least 18 consecutive amino acids of the amino acid sequence selected from the group consisting of said SEQ ID NOs: 9, 10, 11, 12, 13 and 14.

For example, individual peptide fragments of the inventive polymer cassettes may be combined to further individual polymer cassettes as long as the above-identified rules for the overall distribution and amount of alanine, serine and proline are respected. Again, these polymer cassettes may also comprise further amino acid residues, however only as minimal or minor constituents, i. e. maximally 10 %, preferably maximally 2 % of the individual polymer cassette. Said individual polymer cassettes consist, in accordance with this invention, of at least about 3 amino acid residues. Individual polymer cassettes may be combined in order to form longer random coil forming amino acid polymers, whereby a maximal length of a moiety PC is about 3000 amino acids. A preferred minor constituent is lysine.

Preferably, a linkage between a moiety PC and a moiety SP or - in the case that m of formula (1) or (2) is 0 - a moiety L is a permanent linkage, more preferably an amide linkage. Preferably, a moiety PC is linked to a moiety SP or - in the case that m is 0 - to moiety L through an amine group, hydroxyl group, thiol group or carboxyl group of a moiety PC, more preferably through the N-terminal amine group or through a hydroxyl group of a serine residue or through the C-terminal carboxyl group. Even more preferably, PC is linked to a spacer moiety SP or to a moiety L through the N-terminal amine group or through the C-terminal carboxyl group. Most preferably, if t of formula (2) is 1, the moiety PC is linked to a moiety SP or - in the case that m is 0 - to a moiety L through the N-terminal amine group of the moiety PC.

In another preferred embodiment, if t of formula (2) is 2, then the 2 moieties (SP)ₘ-(L-D)ᵥ are preferably connected to the protein carrier PC through the N- and C-terminus of PC, respectively.

A moiety L may be chosen for the water-soluble protein carrier-linked prodrugs depending on the one or more functional groups present in the drug used as biologically active moiety D. Such moieties are known to the person skilled in the art.
In a preferred embodiment, a moiety L is a traceless prodrug linker.

Examples for preferred moieties L are given in the following sections.

A preferred reversible prodrug linker moiety for amine-comprising drugs is described in WO-A 2005/099768. Therefore, the general formulas (IIa) and (IIIa) are preferred embodiments for the water-soluble carrier-linked prodrug according to formula (1), in which m is 1: 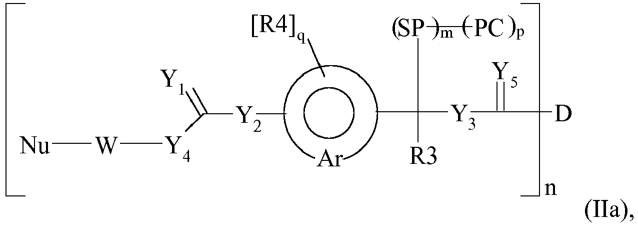 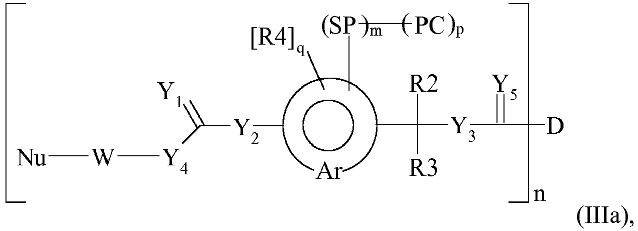 wherein
SP, m, PC, p, n, Y₁, Y₂, Y₃, Y₄, Y₅, R2, R3, R4, Nu, W, q, and D of formulas (IIa) and (IIIa) have the following meaning:
D is an amine-comprising biologically active moiety D of formula (1) which is attached to the rest of the sub-structure shown in formula (IIa) or (IIIa) by forming a -O-(C=O)-N-; -O-(C=S)-N-; -S-(C=O)-N-; or -S-(C=S)-N- linkage,
SP, m, PC, p, and n are used as defined in formula (1),
Y₁ and Y₂ are each independently O, S or NR6,
Y₃ is O or S,
Y₄ is O, NR6, or -C(R7)(R8)-,
Y₅ is O or S,
each of R2 and R3 is a moiety selected from the group consisting of hydrogen, substituted or unsubstituted linear, branched or cyclical alkyl or heteroalkyl groups, aryls, substituted aryls, substituted or unsubstituted heteroaryls, cyano groups, nitro groups, halogens, carboxy groups, carboxyalkyl groups, alkylcarbonyl groups and carboxamidoalkyl groups,
R4 is selected from the group consisting of hydrogen, substituted or unsubstituted linear, branched or cyclical alkyls or heteroalkyls, aryls, substituted aryls, substituted or unsubstituted heteroaryl, substituted or unsubstituted linear, branched or cyclical alkoxys, substituted or unsubstituted linear, branched or cyclical heteroalkyloxys, aryloxys or heteroaryloxys, cyano groups and halogens,
R6 is selected from hydrogen, substituted or unsubstituted linear, branched or cyclical alkyls or heteroalkyls, aryls, substituted aryls and substituted or unsubstituted heteroaryls,
R7 and R8 are each independently selected from the group consisting of hydrogen, substituted or unsubstituted linear, branched or cyclical alkyls or heteroalkyls, aryls, substituted aryls, substituted or unsubstituted heteroaryls, carboxyalkyl groups, alkylcarbonyl groups, carboxamidoalkyl groups, cyano groups, and halogens,
W is selected from substituted or unsubstituted linear, branched or cyclical alkyls, aryls, substituted aryls, substituted or unsubstituted linear, branched or cyclical heteroalkyls, substituted or unsubstituted heteroaryls,
Nu is a nucleophile,
q is zero or a positive integer, and
Ar is a multi-substituted aromatic hydrocarbon or multi-substituted aromatic heterocycle.

A preferred reversible prodrug linker moiety for amine-comprising drugs is described in WO-A 2005/099768. Therefore, the following sub-structures selected from the general formulas (IIb) and (IIIb) are preferred embodiments for a sub-structure 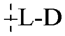 for the water-soluble carrier-linked prodrug according to formula (2): 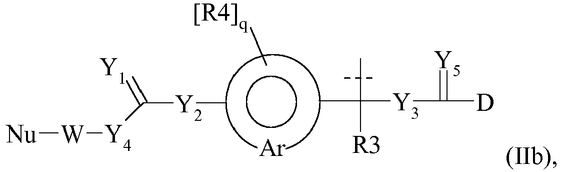 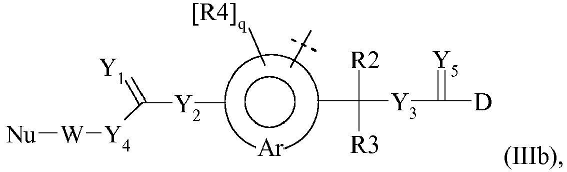
wherein the dashed line indicates attachment to SP (if m=1) or PC (if m=0) of formula (2), and
Y₁, Y₂, Y₃, Y₄, Y₅, R2, R3, R4, Nu, W, q, and D of formulas (IIb) and (IIIb) have the following meaning:
   D is an amine-comprising biologically active moiety D of formula (1) which is attached to the rest of the sub-structure shown in formula (IIb) or (IIIb) by forming a -O-(C=O)-N-; -O-(C=S)-N-; -S-(C=O)-N-; or -S-(C=S)-N- linkage,
   Y₁ and Y₂ are each independently O, S or NR6,
   Y₃ is O or S,
   Y₄ is O, NR6, or -C(R7)(R8)-,
   Y₅ is O or S,
   each of R2 and R3 is a moiety selected from the group consisting of hydrogen, substituted or unsubstituted linear, branched or cyclical alkyl or heteroalkyl groups, aryls, substituted aryls, substituted or unsubstituted heteroaryls, cyano groups, nitro groups, halogens, carboxy groups, carboxyalkyl groups, alkylcarbonyl groups and carboxamidoalkyl groups,
   R4 is selected from the group consisting of hydrogen, substituted or unsubstituted linear, branched or cyclical alkyls or heteroalkyls, aryls, substituted aryls, substituted or unsubstituted heteroaryl, substituted or unsubstituted linear, branched or cyclical alkoxys, substituted or unsubstituted linear, branched or cyclical heteroalkyloxys, aryloxys or heteroaryloxys, cyano groups and halogens,
   R6 is selected from hydrogen, substituted or unsubstituted linear, branched or cyclical alkyls or heteroalkyls, aryls, substituted aryls and substituted or unsubstituted heteroaryls,
   R7 and R8 are each independently selected from the group consisting of hydrogen, substituted or unsubstituted linear, branched or cyclical alkyls or heteroalkyls, aryls, substituted aryls, substituted or unsubstituted heteroaryls, carboxyalkyl groups, alkylcarbonyl groups, carboxamidoalkyl groups, cyano groups, and halogens,
   W is selected from substituted or unsubstituted linear, branched or cyclical alkyls, aryls, substituted aryls, substituted or unsubstituted linear, branched or cyclical heteroalkyls, substituted or unsubstituted heteroaryls,
   Nu is a nucleophile,
   q is zero or a positive integer, and
   Ar is a multi-substituted aromatic hydrocarbon or multi-substituted aromatic heterocycle.

If in formula (2) m is 1, then SP of formula (2) is connected to v sub-structures of formula(s) (IIb) and/or (IIIb). If in formula (2) m is 0, then PC of formula (2) is connected to t sub-structures of formula(s) (IIb) and/or (IIIb).

A preferred reversible prodrug linker moiety for amine-comprising drugs is described in WO-A 2006/136586. Therefore, the general formulas (IVa), (Va), or (VIa) are preferred embodiments for the water-soluble carrier-linked prodrug according to formula (1), in which m is 1: 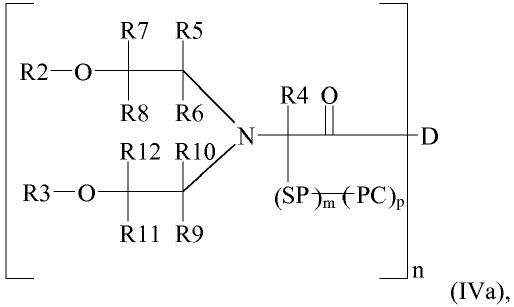 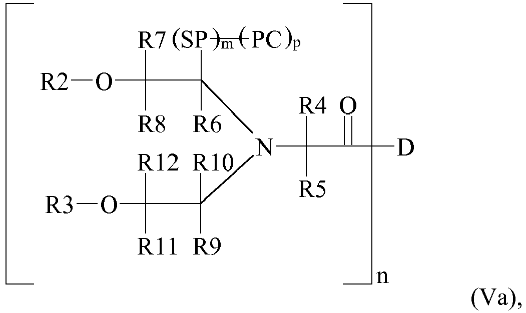 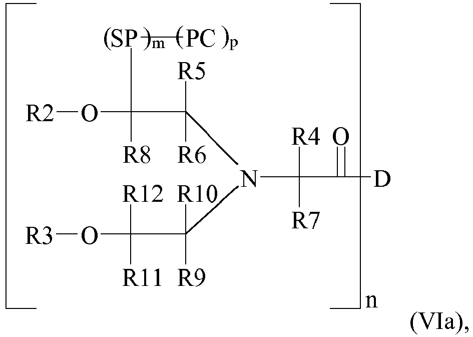 wherein
SP, m, PC, p, n, R2, R3, R4, R5, R6, R7, R8, R9, R10, R11, R12 and D of formulas (IVa), (Va) and (VIa) have the following meaning:
D is an amine-comprising biologically active moiety D of formula (I),
SP, m, PC, p, and n are used as defined in formula (1),
Y1 is O, S, NR6, succinimide, maleimide, an unsaturated carbon-carbon bond, or any heteroatom-containing a free electron pair or Y1 is absent,
R2 and R3 are selected independently from hydrogen, acyl groups, and protecting groups for hydroxyl groups;
R4 to R12 are selected independently from hydrogen, substituted or non-substituted linear, branched or cyclical alkyl or heteroalkyl, aryls, substituted aryls, substituted or non-substituted heteroaryls, cyano, nitro, halogen, carboxy, and carboxamide.

A preferred reversible prodrug linker moiety for amine-comprising drugs is described in WO-A 2006/136586. Therefore, the general formulas (IVb), (Vb), or (VIb) are preferred embodiments for a sub-structure 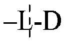 for the water-soluble carrier-linked prodrug according to formula (2): 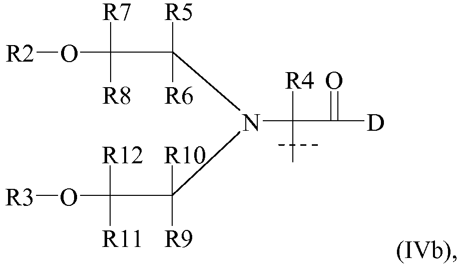 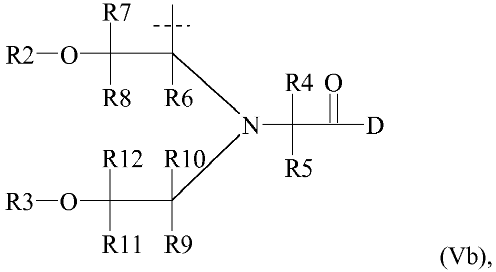 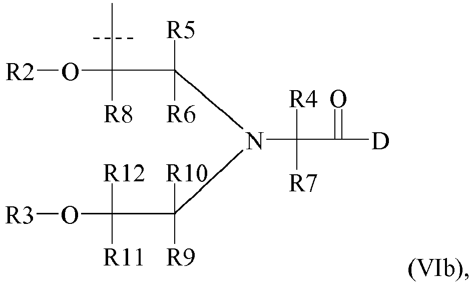 wherein
the dashed line indicates attachment to SP (if m=1) or PC (if m=0) of formula (2), and
R2, R3, R4, R5, R6, R7, R8, R9, R10, R11, and R12 of formulas (IVb), (Vb) and (VIb) have the following meaning:
   R2 and R3 are selected independently from hydrogen, acyl groups, and protecting groups for hydroxyl groups;
   R4 to R12 are selected independently from hydrogen, substituted or non-substituted linear, branched or cyclical alkyl or heteroalkyl, aryls, substituted aryls, substituted or non-substituted heteroaryls, cyano, nitro, halogen, carboxy, and carboxamide.

If in formula (2) m = 1, then SP of formula (2) is connected to v sub-structures of formula(s) (IIb) and/or (IIIb). If in formula (2) m = 0, then PC of formula (2) is connected to t sub-structures of formula(s) (IIb) and/or (IIIb).

In yet another preferred embodiment, a reversible prodrug linker moiety L suitable for biologically active moieties comprising a primary or secondary amine group is described in WO-A 2009/095479. Therefore, a preferred sub-structure (PC)ₚ-(SP)ₘ-L- in formula (1) for the water-soluble protein carrier-linked prodrug has the structure as given in formula (VIIa): 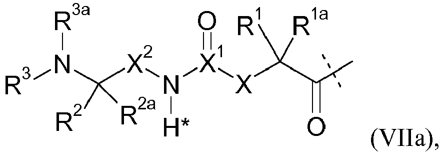 wherein the dashed line indicates the attachment to a primary or secondary amino group of an amine-containing biologically active moiety D by forming an amide bond; and wherein X, X¹, X², R¹, R^{1a}, R², R^{2a}, R³, and R^{3a} of formula (VIIa) have the following meaning:
X is C(R⁴R^{4a}); N(R⁴); O; C(R⁴R^{4a})-C(R⁵R^{5a}); C(R⁵R^{5a})-C(R⁴R^{4a}); C(R⁴R^{4a})-N(R⁶); N(R⁶)-C(R⁴R^{4a}); C(R⁴R^{4a})-O; or O-C(R⁴R^{4a});
X¹ is C; or S(O);
X² is C(R⁷, R^{7a}); or C(R⁷, R^{7a})-C(R⁸, R^{8a});
R¹, R^{1a}, R², R^{2a}, R³, R^{3a}, R⁴, R^{4a}, R⁵, R^{5a}, R⁶, R⁷, R^{7a}, R⁸, R^{8a} are independently selected from the group consisting of H; and C₁₋₄ alkyl; or
Optionally, one or more of the pairs R^{1a}/R^{4a}, R^{1a}/R^{5a}, R^{4a}/R^{5a}, R^{7a}/R^{8a} form a chemical bond;
Optionally, one or more of the pairs R¹/R^{1a}, R²/R^{2a}, R⁴/R^{4a}, R⁵/R^{5a}, R⁷/R^{7a}, R⁸/R^{8a} are joined together with the atom to which they are attached to form a C₃₋₇ cycloalkyl; or 4- to 7-membered heterocyclyl;
Optionally, one or more of the pairs R¹/R⁴, R¹/R⁵, R¹/R⁶, R⁴/R⁵, R⁷/R⁸, R²/R³ are joined together with the atoms to which they are attached to form a ring A;
Optionally, R³/R^{3a} are joined together with the nitrogen atom to which they are attached to form a 4- to 7-membered heterocycle;
A is selected from the group consisting of phenyl; naphthyl; indenyl; indanyl; tetralinyl; C₃₋₁₀ cycloalkyl; 4- to 7-membered heterocyclyl; and 9- to 11-membered heterobicyclyl; and
wherein the structure given in (VIIa) is substituted with p protein carrier moieties PC, either directly or through a spacer moiety SP (m=0 or 1), and wherein the structure given in (VIIa) is optionally further substituted, provided that the hydrogen marked with the asterisk in formula (VIIa) is not replaced by a substituent, and wherein p, PC, SP, and m have the meaning as defined in formula (1).

In yet another preferred embodiment, a reversible prodrug linker moiety L suitable for biologically active moieties comprising a primary or secondary amine group is described in WO-A 2009/095479. Therefore, a preferred sub-structure L in formula (2) for the water-soluble protein carrier-linked prodrug has the structure as given in formula (VIIb): 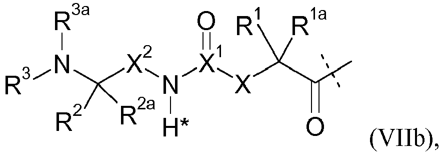 wherein the dashed line indicates the attachment to a primary or secondary amino group of an amine-containing biologically active moiety D by forming an amide bond; and wherein X, X¹, X², R¹, R^{1a}, R², R^{2a}, R³, and R^{3a} of formula (VIIb) have the following meaning:
X is C(R⁴R^{4a}); N(R⁴); O; C(R⁴R^{4a})-C(R⁵R^{5a}); C(R⁵R^{5a})-C(R⁴R^{4a}); C(R⁴R^{4a})-N(R⁶); N(R⁶)-C(R⁴R^{4a}); C(R⁴R^{4a})-O; or O-C(R⁴R^{4a});
X¹ is C; or S(O);
X² is C(R⁷, R^{7a}); or C(R⁷, R^{7a})-C(R⁸, R^{8a});
R¹, R^{1a}, R², R^{2a}, R³, R^{3a}, R⁴, R^{4a}, R⁵, R^{5a}, R⁶, R⁷, R^{7a}, R⁸, R^{8a} are independently selected from the group consisting of H; and C₁₋₄ alkyl; or
Optionally, one or more of the pairs R^{1a}/R^{4a}, R^{1a}/R^{5a}, R^{4a}/R^{5a}, R^{4a}/R^{5a}, R^{7a}/R^{8a} form a chemical bond;
Optionally, one or more of the pairs R¹/R^{1a}, R²/R^{2a}, R⁴/R^{4a}, R⁵/R^{5a}, R⁷/R^{7a}, R⁸/R^{8a} are joined together with the atom to which they are attached to form a C₃₋₇ cycloalkyl; or 4- to 7-membered heterocyclyl;
Optionally, one or more of the pairs R¹/R⁴, R¹/R⁵, R¹/R⁶, R⁴/R⁵, R⁷/R⁸, R²/R³ are joined together with the atoms to which they are attached to form a ring A;
Optionally, R³/R^{3a} are joined together with the nitrogen atom to which they are attached to form a 4- to 7-membered heterocycle;
A is selected from the group consisting of phenyl; naphthyl; indenyl; indanyl; tetralinyl; C₃₋₁₀ cycloalkyl; 4- to 7-membered heterocyclyl; and 9- to 11-membered heterobicyclyl; and
wherein in the structure given in (VIIb) a hydrogen is replaced by a bond for the attachment to the protein carrier PC (m=0) or to the spacer moiety SP (m=1), and wherein the structure given in (VIIb) is optionally further substituted, provided that the hydrogen marked with the asterisk in formula (VIIb) is not replaced by a substituent, and wherein PC, SP, and m have the meaning as defined in formula (2).

Preferably, the one or more further optional substituent(s) of formula (VIIa) and (VIIb) are independently selected from the group consisting of halogen; CN; COOR⁹; OR⁹; C(O)R⁹; C(O)N(R⁹R^{9a}); S(O)₂N(R⁹R^{9a}); S(O)N(R⁹R^{9a}); S(O)₂R⁹; S(O)R⁹; N(R⁹)S(O)₂N(R^{9a}R^{9b}); SR⁹; N(R⁹R^{9a}); NO₂; OC(O)R⁹; N(R⁹)C(O)R^{9a}; N(R⁹)S(O)₂R^{9a}; N(R⁹)S(O)R^{9a}; N(R⁹)C(O)OR^{9a}; N(R⁹)C(O)N(R^{9a}R^{9b}); OC(O)N(R⁹R^{9a}); T; C₁₋₅₀ alkyl; C₂₋₅₀ alkenyl; and C₂₋₅₀ alkynyl,
wherein T, C₁₋₅₀ alkyl, C₂₋₅₀ alkenyl, and C₂₋₅₀ alkynyl are optionally substituted with one or more R¹⁰, which are the same or different,
and wherein C₁₋₅₀ alkyl; C₂₋₅₀ alkenyl; and C₂₋₅₀ alkynyl are optionally interrupted by one or more groups selected from the group consisting of T, -C(O)O-; -O-; -C(O)-; -C(O)N(R¹¹)-; -S(O)₂N(R¹¹)-; -S(O)N(R¹¹)-; -S(O)₂-; -S(O)-; -N(R¹¹)S(O)₂N(R^{11a})-; -S-; -N(R¹¹)-; -OC(O)R¹¹; -N(R¹¹)C(O)-; -N(R¹¹)S(O)₂-; -N(R¹¹)S(O)-; -N(R¹¹)C(O)O-; -N(R¹¹)C(O)N(R^{11a})-, and -OC(O)N(R¹¹R^{11a}),
R⁹, R^{9a}, R^{9b} are independently selected from the group consisting of H; T; and C₁₋₅₀ alkyl; C₂₋₅₀ alkenyl; and C₂₋₅₀ alkynyl,
wherein T, C₁₋₅₀ alkyl, C₂₋₅₀ alkenyl, and C₂₋₅₀ alkynyl are optionally substituted with one or more R¹⁰, which are the same or different,
and wherein C₁₋₅₀ alkyl; C₂₋₅₀ alkenyl; and C₂₋₅₀ alkynyl are optionally interrupted by one or more groups selected from the group consisting of T, -C(O)O-; -O-; -C(O)-; -C(O)N(R¹¹)-; -S(O)₂N(R¹¹)-; -S(O)N(R¹¹)-; -S(O)₂-; -S(O)-; -N(R¹¹)S(O)₂N(R^{11a})-; -S-; -N(R¹¹)-; -OC(O)R¹¹; -N(R¹¹)C(O)-; -N(R¹¹)S(O)₂-; -N(R¹¹)S(O)-; -N(R¹¹)C(O)O-; -N(R¹¹)C(O)N(R^{11a})-, and -OC(O)N(R¹¹R^{11a});
T is selected from the group consisting of phenyl; naphthyl; indenyl; indanyl; tetralinyl; C₃₋₁₀ cycloalkyl; 4- to 7-membered heterocyclyl; and 9- to 11-membered heterobicyclyl, wherein T is optionally substituted with one or more R¹⁰, which are the same or different;
R¹⁰ is halogen; CN; oxo (=O); COOR¹²; OR¹²; C(O)R¹²; C(O)N(R¹²R^{12a}); S(O)₂N(R¹²R^{12a}); S(O)N(R¹²R^{12a}); S(O)₂R¹²; S(O)R¹²; N(R¹²)S(O)₂N(R^{12a}R^{12b}); SR¹²; N(R¹²R^{12a}); NO₂; OC(O)R¹²; N(R¹²)C(O)R^{12a}; N(R¹²)S(O)₂R^{12a}; N(R¹²)S(O)R^{12a}; N(R¹²)C(O)OR^{12a}; N(R¹²)C(O)N(R^{12a}R^{12b}); OC(O)N(R¹²R^{12a}); or C₁₋₆ alkyl, wherein C₁₋₆ alkyl is optionally substituted with one or more halogen, which are the same or different;
R¹¹, R^{11a}, R¹², R^{12a}, R^{12b} are independently selected from the group consisting of H; and C₁₋₆ alkyl, wherein C₁₋₆ alkyl is optionally substituted with one or more halogen, which are the same or different.

The term "interrupted" means that between two carbons a group is inserted or at the end of the carbon chain between the carbon and hydrogen.

Preferred sub-structures according to formula (VIIa) and (VIIb) are selected from the group consisting of 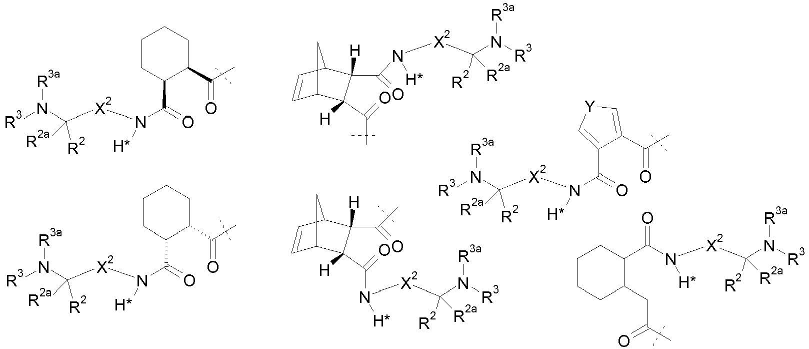 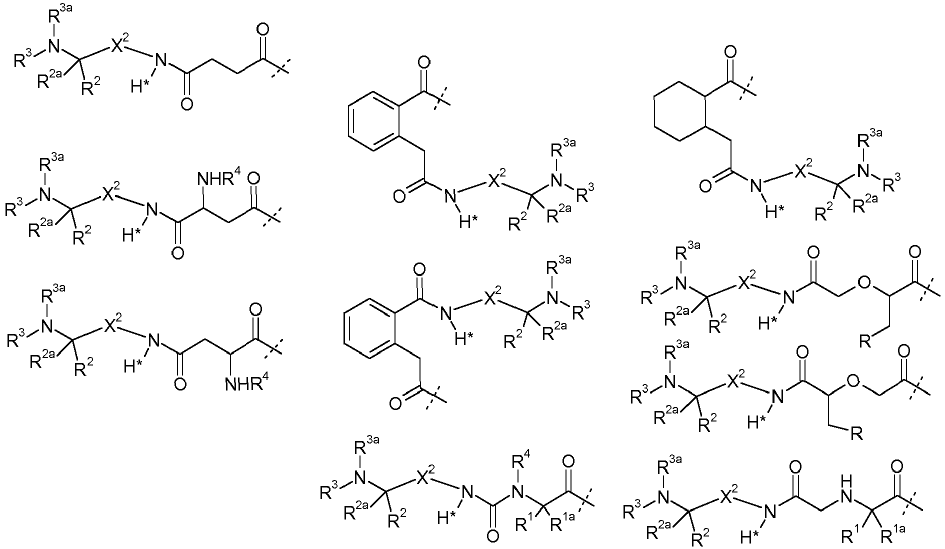 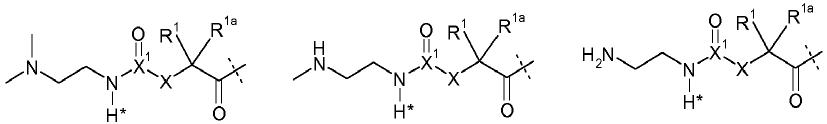 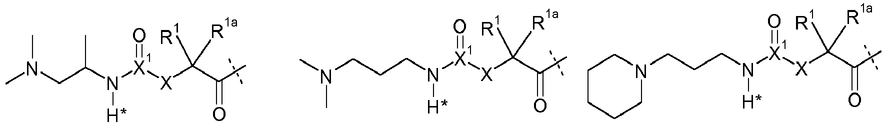 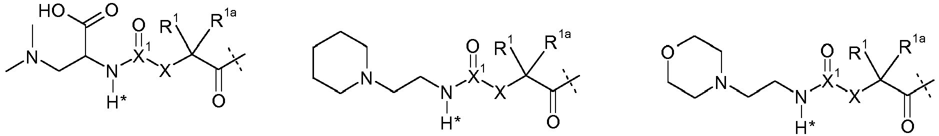 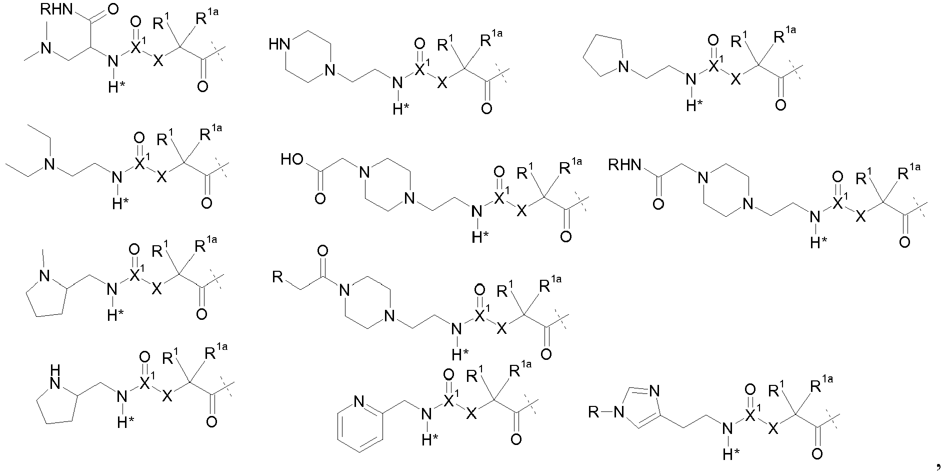
wherein R is H; or C₁₋₄ alkyl;
Y is NH; O; or S;
and R¹, R^{1a}, R², R^{2a}, R³, R^{3a}, R⁴, X, X¹, X² have the meaning as indicated above.

Even more preferred sub-structures according formula (VIIa) and (VIIb) are selected from the group consisting of 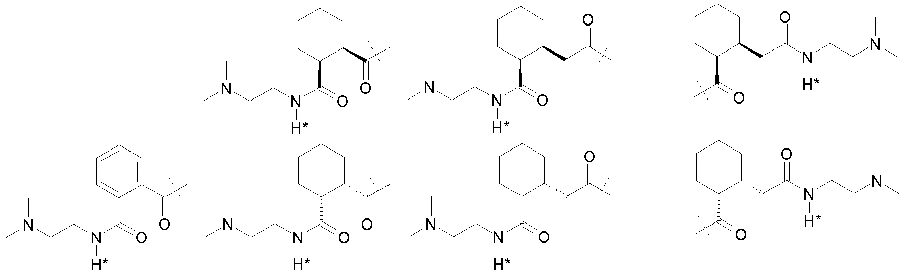 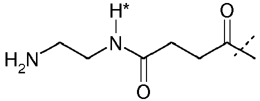 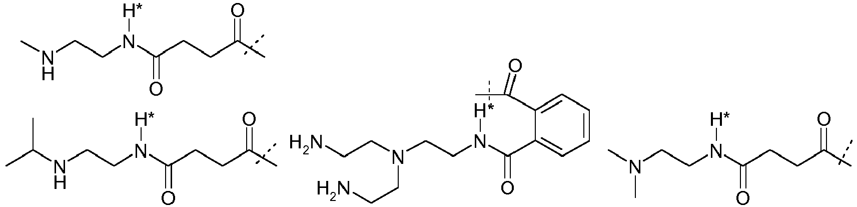 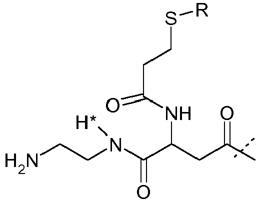 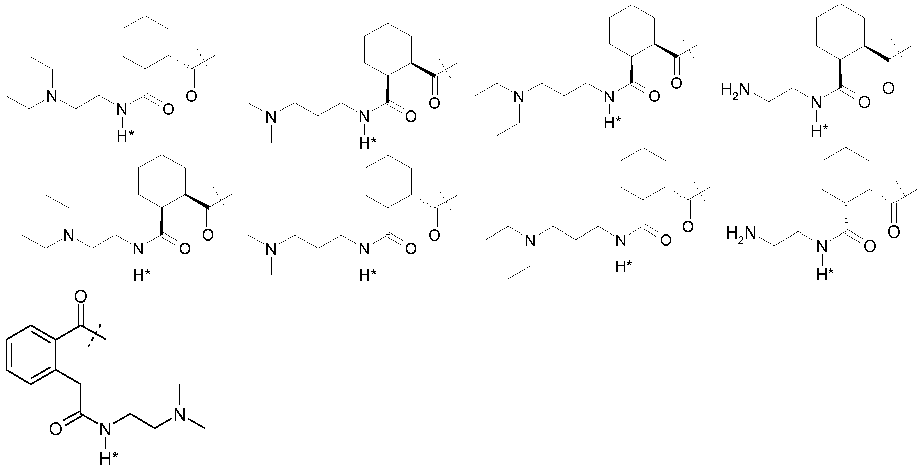 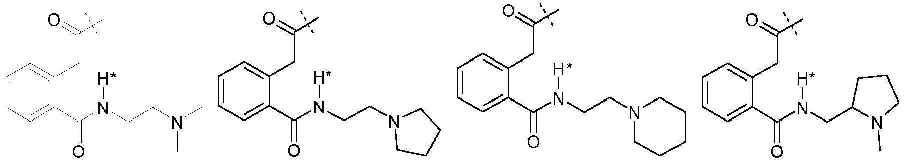 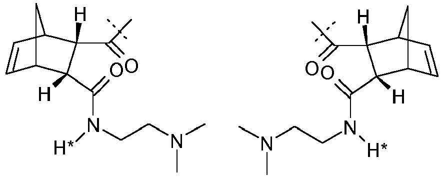 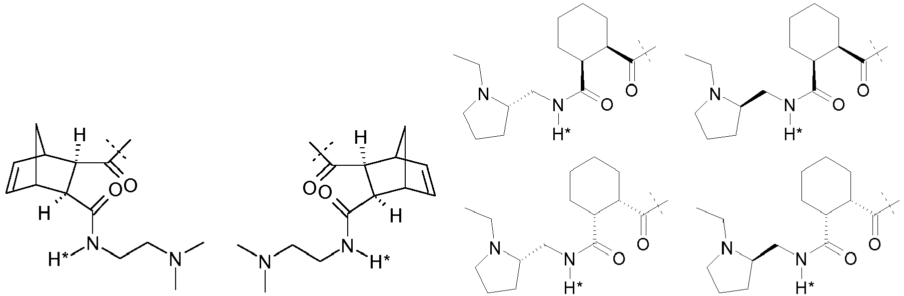 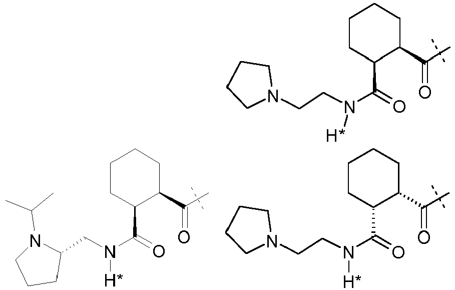 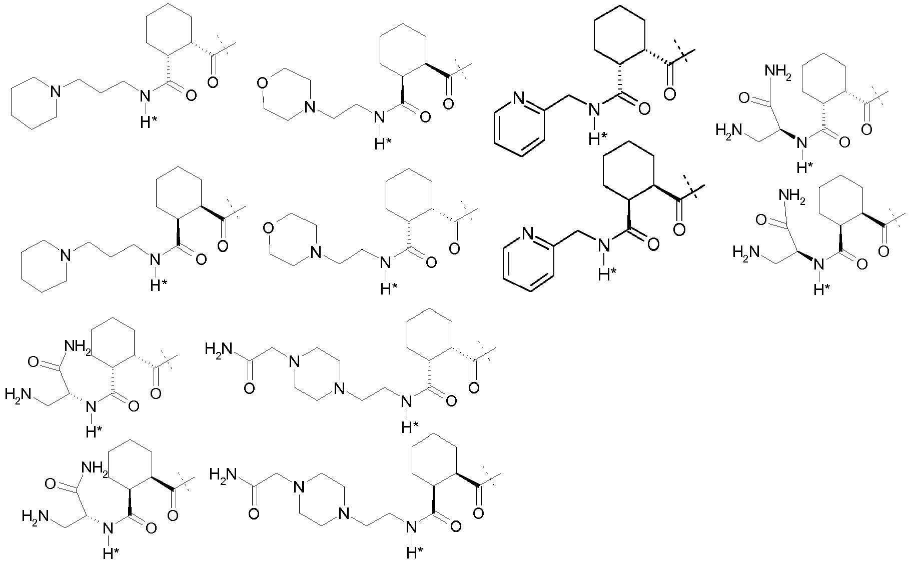 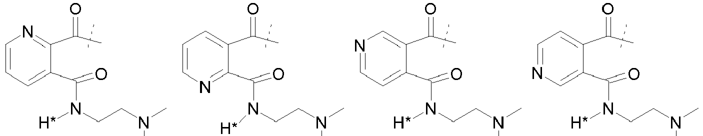 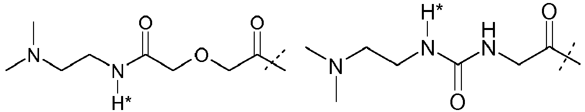 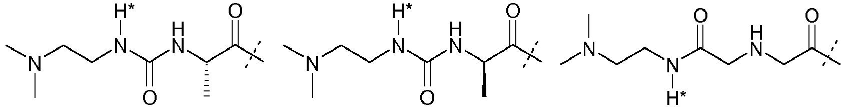 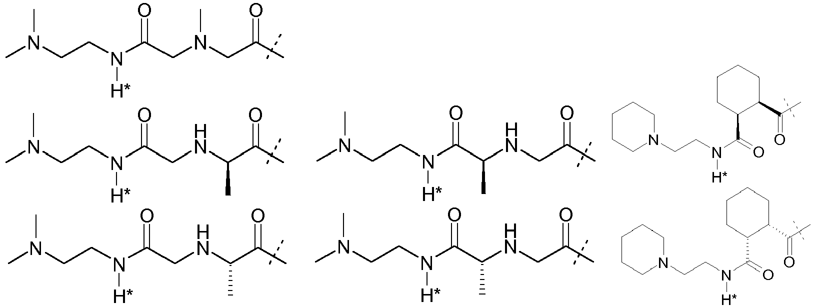 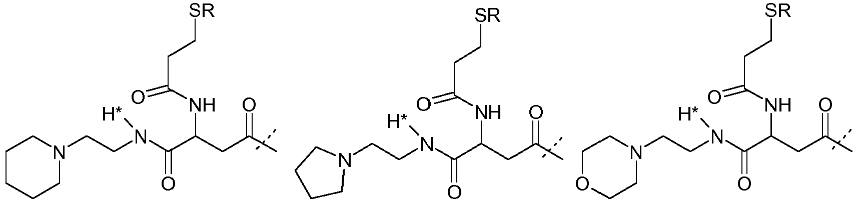 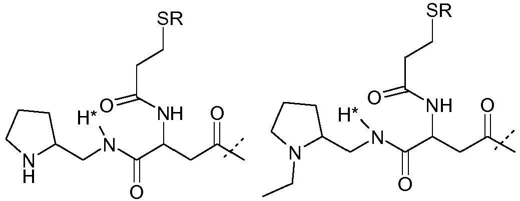 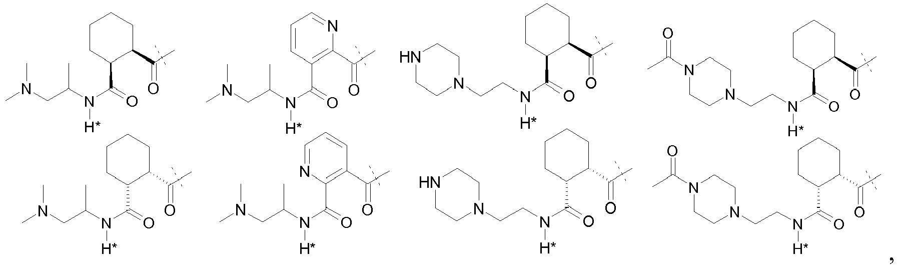 wherein R has the meaning as indicated above.

A further preferred sub-structure (PC)ₚ-(SP)ₘ-L- in formula (1) for the water-soluble protein carrier-linked prodrug has the structure as given in formula (VIIIa): 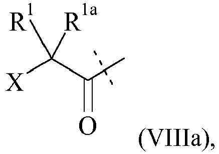 wherein the dashed line indicates the attachment of the linker moiety to an aromatic amino group of the biologically active moiety D by forming an amide bond; and wherein X, R¹, and R^{1a} of formula (VIIIa) have the following meaning:
X is H or C₁₋₅₀ alkyl optionally interrupted by one or more groups selected from -NH-, -C(C₁₋₄ alkyl)-, -O-, -C(O)- and -C(O)NH-;
R¹ and R^{1a} are independently selected from the group consisting of H and C₁-C₄ alkyl;
and wherein the structure given in (VIIIa) is substituted with p protein carrier moieties PC, either directly or through a spacer moiety SP (m=0 or 1), and wherein the structure given in (VIIIa) is optionally further substituted, and wherein p, PC, SP, and m have the meaning as defined in formula (1).

In another embodiment a preferred sub-structure L in formula (2) for the water-soluble protein carrier-linked prodrug has the structure as given in formula (VIIIb): 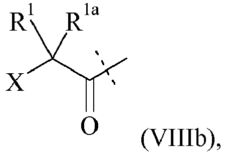 wherein the dashed line indicates the attachment to an aromatic amino group of the biologically active moiety D by forming an amide bond; and wherein X, R¹, and R^{1a} of formula (VIIIb) have the following meaning:
X is H or C₁₋₅₀ alkyl optionally interrupted by one or more groups selected from -NH-, -C(C₁₋₄ alkyl)-, -O-, -C(O)- and -C(O)NH-;
R¹ and R^{1a} are independently selected from the group consisting of H and C₁-C₄ alkyl; and
wherein in the structure given in (VIIIb) a hydrogen is replaced by a bond for the attachment to the protein carrier PC (m=0) or to the spacer moiety SP (m=1), and wherein the structure given in (VIIIb) is optionally further substituted, and wherein PC, SP, and m have the meaning as defined in formula (2).

More preferably, formula (VIIIa) or (VIIIb) comprises one of the fragments of formulas (VIIIc) and (VIIId), wherein the dashed line marked with an asterisk indicates attachment to D by forming an amide bond with the aromatic amino group of biologically active moiety and the unmarked dashed line indicates attachment to the rest of the molecule and wherein the linker fragments of formulae (VIIIc) and (VIIId) are optionally further substituted:

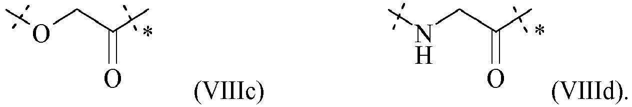

More preferably, formula (VIIIa) or (VIIIb) comprises one of the following fragments, wherein the dashed lines marked with an asterisk indicates attachment to D by forming an amide bond with the aromatic amino group of D and the dashed line on the left hand side indicates attachment to the rest of the molecule:

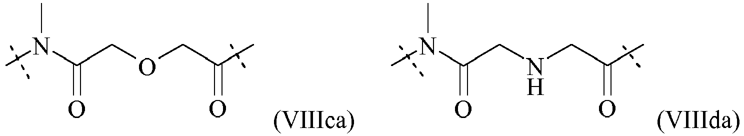

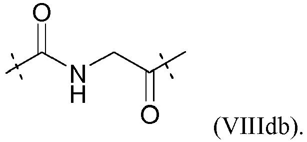

More preferably, the sub-structure (PC)ₚ-(SP)ₘ-L- in formula (1) for the water-soluble protein carrier-linked prodrug has the structure as given in formula of formula (IXa), as disclosed in WO 2011/012721: 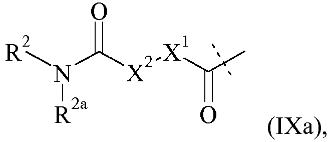 wherein the dashed line indicates the attachment to an aromatic amine group of the biologically active moiety D by forming an amide bond; and wherein X¹, X², R², and R^{2a} in formula (IXa) have the following meaning:
X¹ is C(R¹R^{1a}) or a cyclic fragment selected from C₃₋₇ cycloalkyl, 4- to 7-membered heterocyclyl, phenyl, naphthyl, indenyl, indanyl, tetralinyl, and 9- to 11-membered heterobicyclyl;
X² is a chemical bond or selected from C(R³R^{3a}), N(R³), O, C(R³R^{3a})-C(R⁴R^{4a}), C(R³R^{3a})-N(R⁴), N(R³)-C(R⁴R^{4a}), C(R³R^{3a})-O, and O-C(R³R^{3a}),
wherein in case X¹ is a cyclic fragment, X² is a chemical bond, C(R³R^{3a}), N(R³) or O;
optionally, in case X¹ is a cyclic fragment and X² is C(R³R^{3a}), the order of the X¹ fragment and the X² fragment within the linker moiety shown in formula (IXa) may be changed;
R¹, R³ and R⁴ are independently selected from the group consisting of H, C₁₋₄ alkyl and -N(R⁵R^{5a});
R^{1a}, R², R^{2a}, R^{3a}, R^{4a} and R^{5a} are independently selected from the group consisting of H, and C₁₋₄ alkyl;
optionally, one of the pairs R^{2a}/R², R^{2a}/R^{3a}, R^{2a}/R^{4a} are joined to form a 4- to 7-membered at least partially saturated heterocycle;
R⁵ is C(O)R⁶;
R⁶ is C₁₋₄ alkyl;
optionally, one of the pairs R^{1a}/R^{4a}, R^{3a}/R^{4a} or R^{1a}/R^{3a} form a chemical bond;
and wherein the structure given in (IXa) is substituted with p protein carrier moieties PC, either directly or through a spacer moiety SP (m=0 or 1), and wherein the structure given in (IXa) is optionally further substituted, and wherein p, PC, SP, and m have the meaning as defined in formula (1).

In yet another preferred embodiment, a reversible prodrug linker moiety L suitable for biologically active moieties comprising an aromatic group is described in WO 2011/012721. Therefore, a preferred sub-structure L in formula (2) for the water-soluble protein carrier-linked prodrug has the structure as given in formula (IXb): 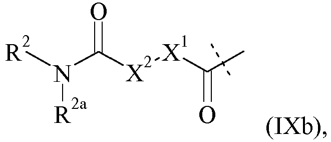 wherein the dashed line indicates the attachment to an aromatic amine group of the biologically active moiety D by forming an amide bond; and wherein X¹, X², R², and R^{2a} in formula (IXb) have the following meaning:
X¹ is C(R¹R^{1a}) or a cyclic fragment selected from C₃₋₇ cycloalkyl, 4- to 7-membered heterocyclyl, phenyl, naphthyl, indenyl, indanyl, tetralinyl, and 9- to 11-membered heterobicyclyl;
X² is a chemical bond or selected from C(R³R^{3a}), N(R³), O, C(R³R^{3a})-C(R⁴R^{4a}), C(R³R^{3a})-N(R⁴), N(R³)-C(R⁴R^{4a}), C(R³R^{3a})-O, and O-C(R³R^{3a}),
wherein in case X¹ is a cyclic fragment, X² is a chemical bond, C(R³R^{3a}), N(R³) or O;
optionally, in case X¹ is a cyclic fragment and X² is C(R³R^{3a}), the order of the X¹ fragment and the X² fragment within the linker moiety shown in formula (IXb) may be changed;
R¹, R³ and R⁴ are independently selected from the group consisting of H, C₁₋₄ alkyl and -N(R⁵R^{5a});
R^{1a}, R², R^{2a}, R^{3a}, R^{4a} and R^{5a} are independently selected from the group consisting of H, and C₁₋₄ alkyl;
optionally, one of the pairs R^{2a}/R², R^{2a}/R^{3a}, R^{2a}/R^{4a} are joined to form a 4- to 7-membered at least partially saturated heterocycle;
R⁵ is C(O)R⁶;
R⁶ is C₁₋₄ alkyl;
optionally, one of the pairs R^{1a}/R^{4a}, R^{3a}/R^{4a} or R^{1a}/R^{3a} form a chemical bond;
and wherein in the structure given in (IXb) a hydrogen is replaced by a bond for the attachment to the protein carrier PC (m=0) or to the spacer moiety SP (m=1), and wherein the structure given in (IXb) is optionally further substituted, and wherein PC, SP, and m have the meaning as defined in formula (2).

More preferably, a sub-structure according to formula (IXa) or (IXb) is selected from the following formulas:

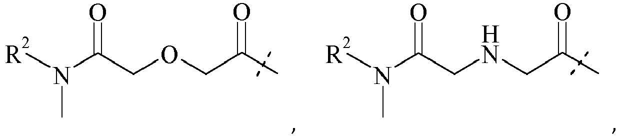

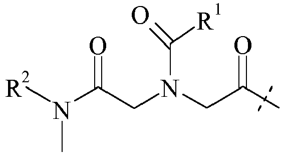

Preferably, in formula (IXa) and (IXb) R^{1a}, R², R^{2a}, R^{3a}, R^{4a} and R^{5a} are independently selected from the group consisting of H, and C₁₋₄ alkyl.

In yet another preferred embodiment, a reversible prodrug linker moiety L suitable for biologically active moieties comprising an aromatic amine group is described in WO 2011/012722. Therefore, a preferred sub-structure (PC)ₚ-(SP)ₘ-L- in formula (1) for the water-soluble protein carrier-linked prodrug has the structure as given in formula (Xa): 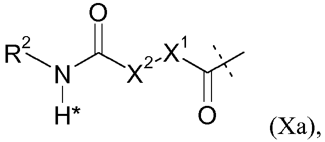 wherein the dashed line indicates the attachment to an aromatic amino group of the biologically active moiety D by forming an amide bond; and wherein X¹, X², and R² of formula (Xa) have the following meaning
X¹ is C(R¹R^{1a}) or a cyclic fragment selected from C₃₋₇ cycloalkyl, 4 to 7 membered heterocyclyl, phenyl, naphthyl, indenyl, indanyl, tetralinyl, and 9 to 11 membered heterobicyclyl,
wherein in case X¹ is a cyclic fragment, said cyclic fragment is incorporated into the linker moiety via two adjacent ring atoms and the ring atom of X¹, which is adjacent to the carbon atom of the amide bond, is also a carbon atom;
X² is a chemical bond or selected from C(R³R^{3a}), N(R³), O, C(R³R^{3a})-C(R⁴R^{4a}), C(R³R^{3a})-N(R⁴), N(R³)-C(R⁴R^{4a}), C(R³R^{3a})-O, or O-C(R³R^{3a}),
wherein in case X¹ is a cyclic fragment, X² is a chemical bond, C(R³R^{3a}), N(R³) or O;
optionally, in case X¹ is a cyclic fragment and X² is C(R³R^{3a}), the order of the X¹ fragment and the X² fragment within the linker moiety of formula (Xa) may be changed and the cyclic fragment is incorporated into the linker moiety via two adjacent ring atoms;
R¹, R³ and R⁴ are independently selected from the group consisting of H, C₁₋₄ alkyl and -N(R⁵R^{5a});
R^{1a}, R², R^{3a}, R^{4a} and R^{5a} are independently selected from the group consisting of H, and C₁₋₄ alkyl;
R⁵ is C(O)R⁶;
R⁶ is C₁₋₄ alkyl;
optionally, one of the pairs R^{1a}/R^{4a}, R^{3a}/R^{4a} or R^{1a}/R^{3a} form a chemical bond;
and wherein the structure given in (Xa) is substituted with p protein carrier moieties PC, either directly or through a spacer moiety SP (m=0 or 1), and wherein the structure given in (Xa) is optionally further substituted, provided that the hydrogen marked with the asterisk in formula (Xa) is not replaced by a substituent, and wherein p, PC, SP, and m have the meaning as defined in formula (1).

In yet another preferred embodiment, a reversible prodrug linker moiety L suitable for biologically active moieties comprising an aromatic amine group is described in WO 2011/012722. Therefore, a preferred sub-structure L in formula (2) for the water-soluble protein carrier-linked prodrug has the structure as given in formula (Xb): 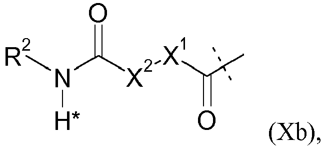
wherein the dashed line indicates the attachment to an aromatic amino group of the biologically active moiety D by forming an amide bond; and wherein X¹, X², and R² of formula (Xb) have the following meaning
X¹ is C(R¹R^{1a}) or a cyclic fragment selected from C₃₋₇ cycloalkyl, 4 to 7 membered heterocyclyl, phenyl, naphthyl, indenyl, indanyl, tetralinyl, and 9 to 11 membered heterobicyclyl,
wherein in case X¹ is a cyclic fragment, said cyclic fragment is incorporated into the linker moiety via two adjacent ring atoms and the ring atom of X¹, which is adjacent to the carbon atom of the amide bond, is also a carbon atom;
X² is a chemical bond or selected from C(R³R^{3a}), N(R³), O, C(R³R^{3a})-C(R⁴R^{4a}), C(R³R^{3a})-N(R⁴), N(R³)-C(R⁴R^{4a}), C(R³R^{3a})-O, or O-C(R³R^{3a}),
wherein in case X¹ is a cyclic fragment, X² is a chemical bond, C(R³R^{3a}), N(R³) or O;
optionally, in case X¹ is a cyclic fragment and X² is C(R³R^{3a}), the order of the X¹ fragment and the X² fragment within the linker moiety of formula (Xb) may be changed and the cyclic fragment is incorporated into the linker moiety via two adjacent ring atoms;
R¹, R³ and R⁴ are independently selected from the group consisting of H, C₁₋₄ alkyl and -N(R⁵R^{5a});
R^{1a}, R², R^{3a}, R^{4a} and R^{5a} are independently selected from the group consisting of H, and C₁₋₄ alkyl;
R⁵ is C(O)R⁶;
R⁶ is C₁₋₄ alkyl;
optionally, one of the pairs R^{1a}/R^{4a}, R^{3a}/R^{4a} or R^{1a}/R^{3a} form a chemical bond;
and wherein in the structure given in (Xb) a hydrogen is replaced by a bond for the attachment to the protein carrier PC (m=0) or to the spacer moiety SP (m=1), and wherein the structure given in (Xb) is optionally further substituted, provided that the hydrogen marked with the asterisk in formula (Xb) is not replaced by a substituent, and wherein PC, SP, and m have the meaning as defined in formula (2).

More preferably, the sub-structures of formulas (Xa) and (Xb) are selected from the group comprising formulas (i) through (xxix):

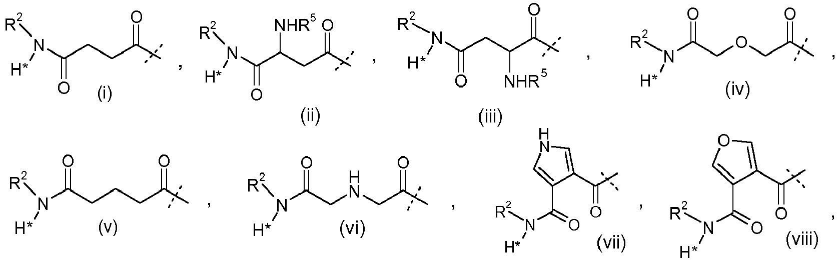

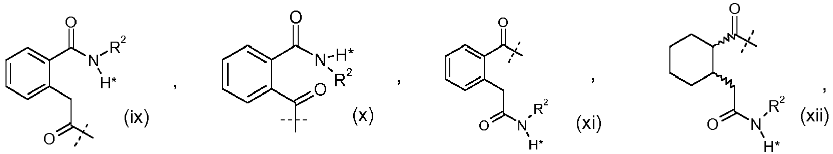

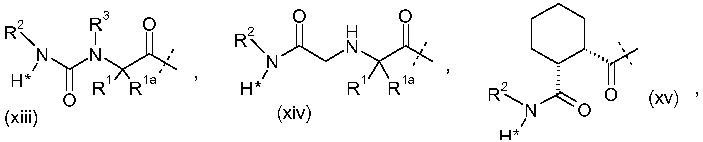

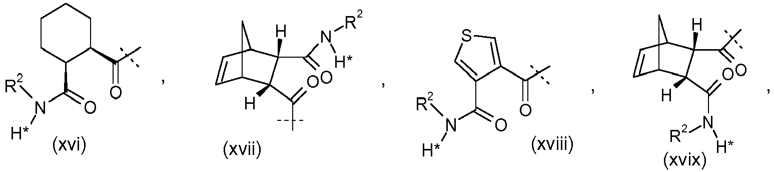

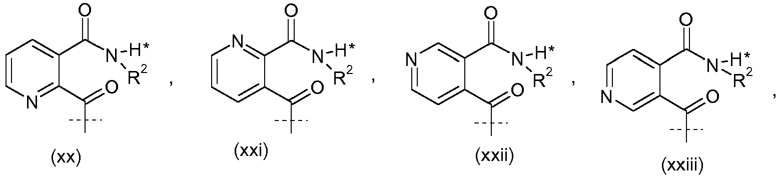

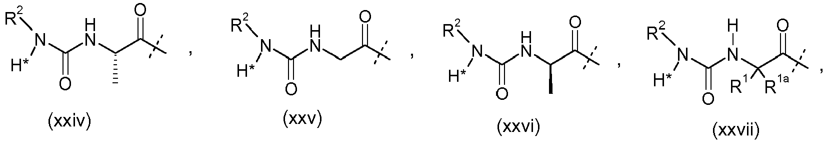

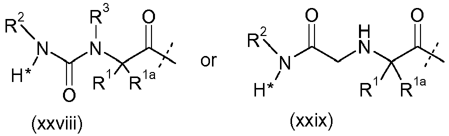

The amino substituent of the aromatic fragment of the biologically active moiety D forms together with the carbonyl-fragment (-C(O)-) on the right hand side of the sub-structure (as depicted in formula (Xa) and (Xb)) an amide bond within the conjugate comprising D and L. By consequence, the two parts (D and L of formula (Xa) and (Xb) are connected (chemically bound) by an amide fragment of the general structure Y¹-C(O)-N(R)-Y². Y¹ indicates the remaining parts of L as shown in (Xa) and (Xb) and Y² indicates the aromatic fragment of D. R is a substituent such as C₁₋₄ alkyl or preferably hydrogen. For example, said amide bond is indicated within formula (Xa) and (Xb) by the dashed line added diagonally on this bond.

As indicated above, the X¹-fragment of sub-structures of formula (Xa) and (Xb) may also be a cyclic fragment such as C₃₋₇ cycloalkyl, phenyl or indanyl. In case X¹ is such a cyclic fragment, the respective cyclic fragment is incorporated into the sub-structure of formula (Xa) and (Xb) via two adjacent ring atoms (of said cyclic fragment). For example, if X¹ is phenyl, the phenyl fragment of the sub-structure is bound to the X² fragment of the sub-structure via a first (phenyl) ring atom being in α-position (adjacent) to a second (phenyl) ring atom, which itself is bound to the carbon atom of the carbonyl-fragment on the right hand side of the sub-structure according to formula (Xa) and (Xb), i.e. the carbonyl fragment which together with the aromatic amino group of D forms an amide bond.

Preferably, the sub-structures of formula (Xa) and (Xb) are defined as follows:
X¹ is C(R¹R^{1a}), cyclohexyl, phenyl, pyridinyl, norbonenyl, furanyl, pyrrolyl or thienyl,
wherein in case X¹ is a cyclic fragment, said cyclic fragment is incorporated into the reversible prodrug linker moiety of formula (X) via two adjacent ring atoms;
X² is a chemical bond or selected from C(R³R^{3a}), N(R³), O, C(R³R^{3a})-O and C(R³R^{3a})-C(R⁴R^{4a});
R¹, R³ and R⁴ are independently selected from H, C₁₋₄ alkyl and -N(R⁵R^{5a});
R^{1a}, R^{3a}, R^{4a} and R^{5a} are independently selected from H and C₁₋₄ alkyl;
R² is C₁₋₄ alkyl;
R⁵ is C(O)R⁶;
R⁶ is C₁₋₄ alkyl;

More preferably, the sub-structure of formula (Xa) and (Xb) is selected from 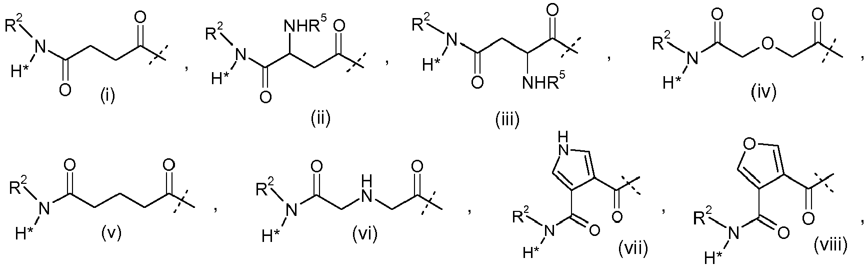 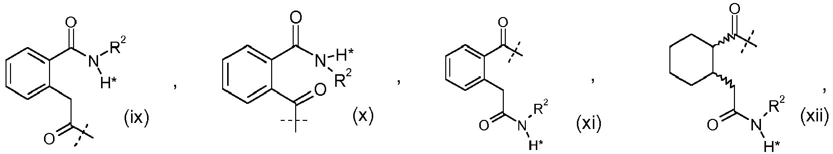 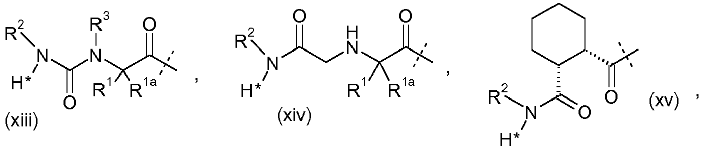 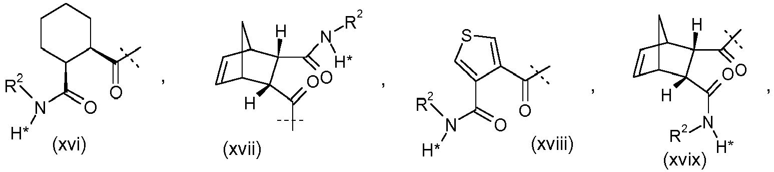 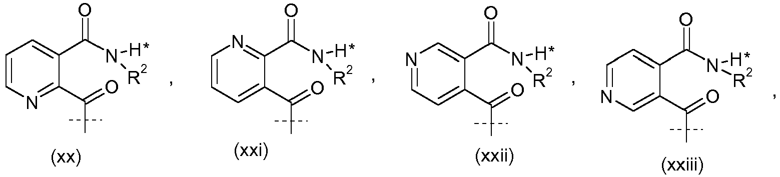 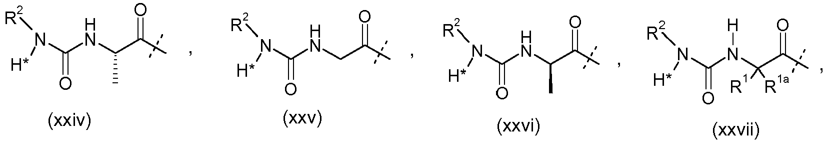 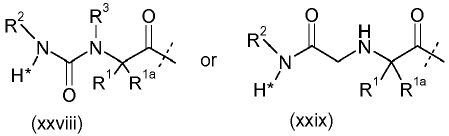 wherein
R⁵ is C(O)R⁶;
R¹, R^{1a}, R², R³ and R⁶ are independently from each other C₁₋₄ alkyl.

Yet another preferred reversible prodrug linker moiety for hydroxyl-comprising drugs is described in WO-A 2011/012721. Therefore, the general formula (XIa) is a preferred embodiment for the water-soluble carrier-linked prodrug according to formula (1), in which m is 1: 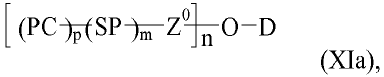 wherein D, SP, m, PC, p, n and Z⁰ in formula (XIa) have the following meaning:
D is a hydroxyl-comprising biologically active moiety D comprising O,
SP, m, PC, p and n are used as defined in formula (1),
Z⁰ is X⁰-C(O), X⁰-O-C(O), X⁰-S(O)₂, X⁰-C(S), X⁰-O-S(O)₂, X⁰-S(O)₂N(R¹), X⁰-CH(OR¹), X⁰-C(OR¹)(OR²), X⁰-C(O)N(R¹), X⁰-P(=O)(OH)O, X⁰-P(=O)(OR¹)O, X⁰-P(=O)(SH)O, X⁰-P(=O)(SR¹)O, X⁰-P(=O)(OR¹), X⁰-P(=S)(OH)O, X⁰-P(=S)(OR¹)O, X⁰-P(=S)(OH)N(R¹), X⁰-P(=S)(OR¹)N(R²), X⁰-P(=O)(OH)N(R¹) or X⁰-P(=O)(OR¹)N(R²),
R¹, R² are independently selected from the group consisting of C₁₋₆ alkyl; or R¹ and R² jointly form a C₁₋₆ alkylene bridging group,
X⁰ is (X^{0A})ₘ₁-(X^{0B})ₘ₂,
m1, m2 are independently 0 or 1,
X^{0A} is T⁰,
X^{0B} is a branched or unbranched C₁₋₁₀ alkylene group which is unsubstituted or substituted with one or more R³, which is/are the same or different,
R³ is halogen, CN, C(O)R⁴, C(O)OR⁴, OR⁴, C(O)R⁴, C(O)N(R⁴R^{4a}), S(O)₂N(R⁴R^{4a}), S(O)N(R⁴R^{4a}), S(O)₂R⁴, S(O)R⁴, N(R⁴)S(O)₂N(R^{4a}R^{4b}), SR⁴, N(R⁴R^{4a}), NO₂, OC(O)R⁴, N(R⁴)C(O)R^{4a}, N(R⁴)SO₂R^{4a}, N(R⁴)S(O)R^{4a}, N(R⁴)C(O)N(R^{4a}R^{4b}), N(R⁴)C(O)OR^{4a}, OC(O)N(R⁴R^{4a}), or T⁰,
R⁴, R^{4a}, R^{4b} are independently selected from the group consisting of H, T⁰, C₁₋₄ alkyl, C₂₋₄ alkenyl, and C₂₋₄ alkynyl, wherein C₁₋₄ alkyl, C₂₋₄ alkenyl, and C₂₋₄ alkynyl are optionally substituted with one or more R⁵, which is/are the same of different,
R⁵ is halogen, CN, C(O)R⁶, C(O)OR⁶, OR⁶, C(O)R⁶, C(O)N(R⁶R^{6a}), S(O)₂N(R⁶R^{6a}), S(O)N(R⁶R^{6a}), S(O)₂R⁶, S(O)R⁶, N(R⁶)S(O)₂N(R^{6a}R^{6b}), SR⁶, N(R⁶R^{6a}), NO₂, OC(O)R⁶, N(R⁶)C(O)R^{6a}, N(R⁶)SO₂R^{6a}, N(R⁶)S(O)R^{6a}, N(R⁶)C(O)N(R^{6a}R^{6b}), N(R⁶)C(O)OR^{6a}, or OC(O)N(R⁶R^{6a}),
R⁶, R^{6a}, R^{6b} are independently selected from the group consisting of H, C₁₋₆ alkyl, C₂₋₆ alkenyl, and C₂₋₆ alkynyl, wherein C₁₋₆ alkyl, C₂₋₆ alkenyl, and C₂₋₆ alkynyl are optionally substituted with one or more halogen, which is/are the same of different,
T⁰ is phenyl, naphthyl, azulenyl, indenyl, indanyl, C₃₋₇ cycloalkyl, 3- to 7-membered heterocyclyl, or 8- to 11-membered heterobicyclyl, wherein T⁰, is optionally substituted with one or more R⁷, which is/are the same or different,
R⁷ is halogen, CN, COOR⁸, OR⁸, C(O)R⁸, C(O)N(R⁸R^{8a}), S(O)₂N(R⁸R^{8a}), S(O)N(R⁸R^{8a}), S(O)₂R⁸, S(O)R⁸, N(R⁸)S(O)₂N(R^{8a}R^{8b}), SR⁸, N(R⁸R^{8a}), NO₂, OC(O)R⁸, N(R⁸)C(O)R^{8a}, N(R⁸)S(O)₂R^{8a}, N(R⁸)S(O)R^{8a}, N(R⁸)C(O)OR^{8a}, N(R⁸)C(O)N(R^{8a}R^{8b}), OC(O)N(R⁸R^{8a}), oxo (=O), where the ring is at least partially saturated, C₁₋₆ alkyl, C₂₋₆ alkenyl, or C₂₋₆ alkynyl, wherein C₁₋₆ alkyl, C₂₋₆ alkenyl, and C₂₋₆ alkynyl are optionally substituted with one or more R⁹, which is/are the same or different,
R⁸, R^{8a}, R^{8b} are independently selected from the group consisting of H, C₁₋₆ alkyl, C₂₋₆ alkenyl, and C₂₋₆ alkynyl, wherein C₁₋₆ alkyl, C₂₋₆ alkenyl, and C₂₋₆ alkynyl are optionally substituted with one or more R¹⁰, which is/are the same of different,
R⁹, R¹⁰ are independently selected from the group consisting of halogen, CN, C(O)R¹¹, C(O)OR¹¹, OR¹¹, C(O)R¹¹, C(O)N(R¹¹R^{11a}), S(O)₂N(R¹¹R^{11a}), S(O)N(R¹¹R^{11a}), S(O)₂R¹¹, S(O)R¹¹, N(R¹¹)S(O)₂N(R^{11a}R^{11b}), SR¹¹, N(R¹¹R^{11a}), NO₂, OC(O)R¹¹, N(R¹¹)C(O)R^{11a}, N(R¹¹)SO₂R^{11a}, N(R¹¹)S(O)R^{11a}, N(R¹¹)C(O)N(R^{11a}R^{11b}), N(R¹¹)C(O)OR^{11a}, and OC(O)N(R¹¹R^{11a}),
R¹¹, R^{11a}, R^{11b} are independently selected from the group consisting of H, C₁₋₆ alkyl, C₂₋₆ alkenyl, and C₂₋₆ alkynyl, wherein C₁₋₆ alkyl, C₂₋₆ alkenyl, and C₂₋₆ alkynyl are optionally substituted with one or more halogen, which is/are the same of different, and
wherein -(SP)ₘ- of formula (XIa) is covalently attached to X⁰.

Preferably, Z⁰ is X⁰-C(O), X⁰-C(O)O, or X⁰-S(O)₂. More preferably, Z⁰ is X⁰-C(O) or X⁰-C(O)O. Even more preferably, Z⁰ is X⁰-C(O).

Preferably, X⁰ is unsubstituted.

Preferably, m1 is 0 and m2 is 1.

Preferably, X⁰ is C(R¹R²)CH₂, wherein R¹ and R² are independently selected from the group consisting of H and C₁₋₄ alkyl, provided that at least one of R¹, R² is other than H, or (CH₂)ₙ, wherein n is 3, 4, 5, 6, 7 or 8.

Preferably, the moiety -(SP)ₘ- of formula (XIa) is covalently attached to X⁰ via an amide group.

Yet another preferred reversible prodrug linker moiety for hydroxyl-comprising drugs is described in WO-A 2011/012721. Therefore, the general formula (Xlb) is a preferred embodiment for a sub-structure 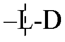 for the water-soluble carrier-linked prodrug according to formula (2): 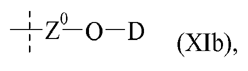
wherein the dashed line indicates attachment to SP (if m=1) or PC (if m=0) of formula (2), and
D and Z⁰ in formula (Xlb) have the following meaning:
   D is a hydroxyl-comprising biologically active moiety D comprising O,
   Z⁰ is X⁰-C(O), X⁰-O-C(O), X⁰-S(O)₂, X⁰-C(S), X⁰-O-S(O)₂, X⁰-S(O)₂N(R¹), X⁰-CH(OR¹), X⁰-C(OR¹)(OR²), X⁰-C(O)N(R¹), X⁰-P(=O)(OH)O, X⁰-P(=O)(OR¹)O, X⁰-P(=O)(SH)O, X⁰-P(=O)(SR¹)O, X⁰-P(=O)(OR¹), X⁰-P(=S)(OH)O, X⁰-P(=S)(OR¹)O, X⁰-P(=S)(OH)N(R¹), X⁰-P(=S)(OR¹)N(R²), X⁰-P(=O)(OH)N(R¹) or X⁰-P(=O)(OR¹)N(R²),
   R¹, R² are independently selected from the group consisting of C₁₋₆ alkyl; or R¹ and R² jointly form a C₁₋₆ alkylene bridging group,
   X⁰ is (X^{0A})ₘ₁-(X^{0B})ₘ₂,
   m1, m2 are independently 0 or 1,
   X^{0A} is T⁰,
   X^{0B} is a branched or unbranched C₁₋₁₀ alkylene group which is unsubstituted or substituted with one or more R³, which is/are the same or different,
   R³ is halogen, CN, C(O)R⁴, C(O)OR⁴, OR⁴, C(O)R⁴, C(O)N(R⁴R^{4a}), S(O)₂N(R⁴R^{4a}), S(O)N(R⁴R^{4a}), S(O)₂R⁴, S(O)R⁴, N(R⁴)S(O)₂N(R^{4a}R^{4b}), SR⁴, N(R⁴R^{4a}), NO₂, OC(O)R⁴, N(R⁴)C(O)R^{4a}, N(R⁴)SO₂R^{4a}, N(R⁴)S(O)R^{4a}, N(R⁴)C(O)N(R^{4a}R^{4b}), N(R⁴)C(O)OR^{4a}, OC(O)N(R⁴R^{4a}), or T⁰,
   R⁴, R^{4a}, R^{4b} are independently selected from the group consisting of H, T⁰, C₁₋₄ alkyl, C₂₋₄ alkenyl, and C₂₋₄ alkynyl, wherein C₁₋₄ alkyl, C₂₋₄ alkenyl, and C₂₋₄ alkynyl are optionally substituted with one or more R⁵, which is/are the same of different,
   R⁵ is halogen, CN, C(O)R⁶, C(O)OR⁶, OR⁶, C(O)R⁶, C(O)N(R⁶R^{6a}), S(O)₂N(R⁶R^{6a}), S(O)N(R⁶R^{6a}), S(O)₂R⁶, S(O)R⁶, N(R⁶)S(O)₂N(R^{6a}R^{6b}), SR⁶, N(R⁶R^{6a}), NO₂, OC(O)R⁶, N(R⁶)C(O)R^{6a}, N(R⁶)SO₂R^{6a}, N(R⁶)S(O)R^{6a}, N(R⁶)C(O)N(R^{6a}R^{6b}), N(R⁶)C(O)OR^{6a}, or OC(O)N(R⁶R^{6a}),
   R⁶, R^{6a}, R^{6b} are independently selected from the group consisting of H, C₁₋₆ alkyl, C₂₋₆ alkenyl, and C₂₋₆ alkynyl, wherein C₁₋₆ alkyl, C₂₋₆ alkenyl, and C₂₋₆ alkynyl are optionally substituted with one or more halogen, which is/are the same of different,
   T⁰ is phenyl, naphthyl, azulenyl, indenyl, indanyl, C₃₋₇ cycloalkyl, 3- to 7-membered heterocyclyl, or 8- to 11-membered heterobicyclyl, wherein T⁰, is optionally substituted with one or more R⁷, which is/are the same or different,
   R⁷ is halogen, CN, COOR⁸, OR⁸, C(O)R⁸, C(O)N(R⁸R^{8a}), S(O)₂N(R⁸R^{8a}), S(O)N(R⁸R^{8a}), S(O)₂R⁸, S(O)R⁸, N(R⁸)S(O)₂N(R^{8a}R^{8b}), SR⁸, N(R⁸R^{8a}), NO₂, OC(O)R⁸, N(R⁸)C(O)R^{8a}, N(R⁸)S(O)₂R^{8a}, N(R⁸)S(O)R^{8a}, N(R⁸)C(O)OR^{8a}, N(R⁸)C(O)N(R^{8a}R^{8b}), OC(O)N(R⁸R^{8a}), oxo (=O), where the ring is at least partially saturated, C₁₋₆ alkyl, C₂₋₆ alkenyl, or C₂₋₆ alkynyl, wherein C₁₋₆ alkyl, C₂₋₆ alkenyl, and C₂₋₆ alkynyl are optionally substituted with one or more R⁹, which is/are the same or different,
   R⁸, R^{8a}, R^{8b} are independently selected from the group consisting of H, C₁₋₆ alkyl, C₂₋₆ alkenyl, and C₂₋₆ alkynyl, wherein C₁₋₆ alkyl, C₂₋₆ alkenyl, and C₂₋₆ alkynyl are optionally substituted with one or more R¹⁰, which is/are the same of different,
   R⁹, R¹⁰ are independently selected from the group consisting of halogen, CN, C(O)R¹¹, C(O)OR¹¹, OR¹¹, C(O)R¹¹, C(O)N(R¹¹R^{11a}), S(O)₂N(R¹¹R^{11a}), S(O)N(R¹¹R^{11a}), S(O)₂R¹¹, S(O)R¹¹, N(R¹¹)S(O)₂N(R^{11a}R^{11b}), SR¹¹, N(R¹¹R^{11a}), NO₂, OC(O)R¹¹, N(R¹¹)C(O)R^{11a}, N(R¹¹)SO₂R^{11a}, N(R¹¹)S(O)R^{11a}, N(R¹¹)C(O)N(R^{11a}R^{11b}), N(R¹¹)C(O)OR^{11a}, and OC(O)N(R¹¹R^{11a}),
   R¹¹, R^{11a}, R^{11b} are independently selected from the group consisting of H, C₁₋₆ alkyl, C₂₋₆ alkenyl, and C₂₋₆ alkynyl, wherein C₁₋₆ alkyl, C₂₋₆ alkenyl, and C₂₋₆ alkynyl are optionally substituted with one or more halogen, which is/are the same of different, and
   wherein Z⁰ of formula (Xlb) is covalently attached to the rest of the water-soluble carrier-linked prodrug of formula (2).

Preferably, Z⁰ is X⁰-C(O), X⁰-C(O)O, or X⁰-S(O)₂. More preferably, Z⁰ is X⁰-C(O) or X⁰-C(O)O. Even more preferably, Z⁰ is X⁰-C(O).
Preferably, X⁰ is unsubstituted.

Preferably, m1 is 0 and m2 is 1.

Preferably, X⁰ is C(R¹R²)CH₂, wherein R¹ and R² are independently selected from the group consisting of H and C₁₋₄ alkyl, provided that at least one of R¹, R² is other than H, or (CH₂)ₙ, wherein n is 3, 4, 5, 6, 7 or 8.

If in formula (2) m = 1, then SP of formula (2) is connected to v sub-structures of formula (Xlb). If in formula (2) m = 0, then PC of formula (2) is connected to t sub-structures of formula (Xlb).

In another preferred embodiment, a sub-structure (PC)ₚ-(SP)ₘ-L- in formula (1) for the water-soluble protein carrier-linked prodrug has the structure as given in formula (XIIa): 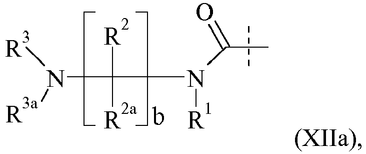 wherein the dashed line indicates attachment to the aromatic hydroxyl group of the hydroxyl-containing biologically active moiety D by forming a carbamate group; and wherein R¹, R², R^{2a}, R³, R^{3a} and b of formula (XIIa) are defined as follows:
R¹ is selected from the group consisting of C₁₋₄ alkyl; heteroalkyl; C₃₋₇ cycloalkyl; and 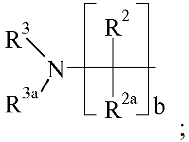
R², R^{2a}, R³, R^{3a} are independently selected from hydrogen, substituted or non-substituted linear, branched or cyclic C₁₋₄ alkyl or heteroalkyl;
b is independently 2, 3 or 4;
and wherein the structure given in (XIIa) is substituted with p protein carrier moieties PC, either directly or through a spacer moiety SP (m=0 or 1), and wherein the structure given in (XIIa) is optionally further substituted, and wherein p, PC, SP, and m have the meaning as defined in formula (1).

In another preferred embodiment, a sub-structure L in formula (2) for the water-soluble protein carrier-linked prodrug has the structure as given in formula (XIIb): 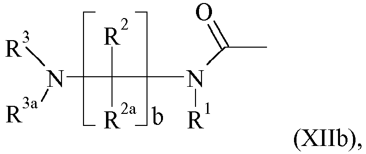 wherein the dashed line indicates attachment to the aromatic hydroxyl group of the hydroxyl-containing biologically active moiety D by forming a carbamate group; and wherein R¹, R², R^{2a}, R³, R^{3a} and b of formula (XIIb) are defined as follows:
R¹ is selected from the group consisting of C₁₋₄ alkyl; heteroalkyl; C₃₋₇ cycloalkyl; and 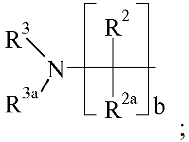
R², R^{2a}, R³, R^{3a} are independently selected from hydrogen, substituted or non-substituted linear, branched or cyclic C₁₋₄ alkyl or heteroalkyl;
b is independently 2, 3 or 4;
and wherein in the structure given in (XIIb) a hydrogen is replaced by a bond for the attachment to the protein carrier PC (m=0) or to the spacer moiety SP (m=1), and wherein the structure given in (XIIb) is optionally further substituted, and wherein PC, SP, and m have the meaning as defined in formula (2).

In yet another preferred embodiment, a sub-structure (PC)ₚ-(SP)ₘ-L- in formula (1) for the water-soluble protein carrier-linked prodrug has the structure as given in formula (XIIIa): 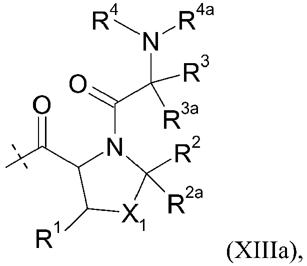 wherein the dashed line indicates attachment to an aliphatic amino group of the biologically active moiety D by forming an amide bond; and wherein X₁, R¹, R², R^{2a}, R³, R^{3a}, R⁴ and R^{4a} of formula (XIIIa) have the following meaning:
X₁ is selected from O, S or CH-R^{1a},
R¹ and R^{1a} are independently selected from H, OH, CH₃,
R², R^{2a}, R⁴ and R^{4a} are independently selected from H and C₁₋₄ alkyl,
R³, R^{3a} are independently selected from H, C₁₋₄ alkyl, and R⁵,
R⁵ is selected from 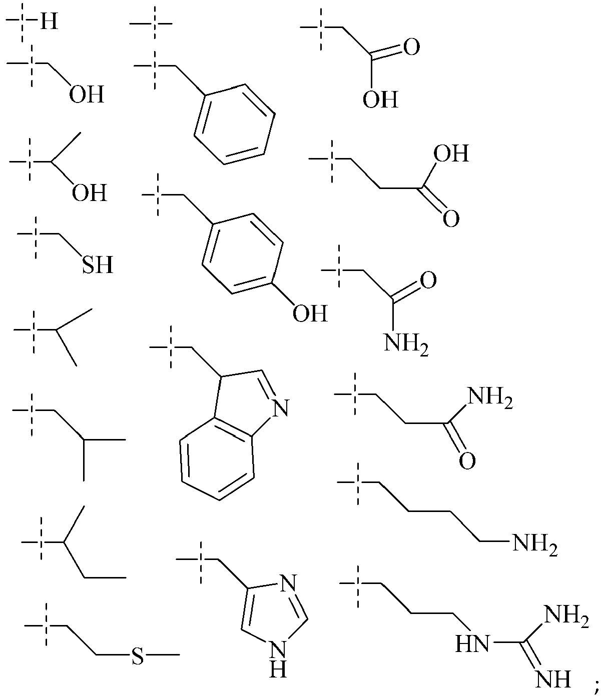 wherein the structure given in (XIIIa) is substituted with p protein carrier moieties PC, either directly or through a spacer moiety SP (m=0 or 1), and wherein the structure given in (XIIIa) is optionally further substituted, and wherein p, PC, SP, and m have the meaning as defined in formula (1).

In another preferred embodiment, a sub-structure L in formula (2) for the water-soluble protein carrier-linked prodrug has the structure as given in formula (XIIIb): 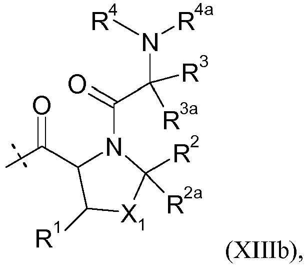 wherein the dashed line indicates attachment to an aliphatic amino group of the biologically active moiety D by forming an amide bond; and wherein X₁, R¹, R², R^{2a}, R³, R^{3a}, R⁴ and R^{4a} of formula (XIIIb) have the following meaning:
X₁ is selected from O, S or CH-R^{1a},
R¹ and R^{1a} are independently selected from H, OH, CH₃,
R², R^{2a}, R⁴ and R^{4a} are independently selected from H and C₁₋₄ alkyl,
R³, R^{3a} are independently selected from H, C₁₋₄ alkyl, and R⁵,
R⁵ is selected from 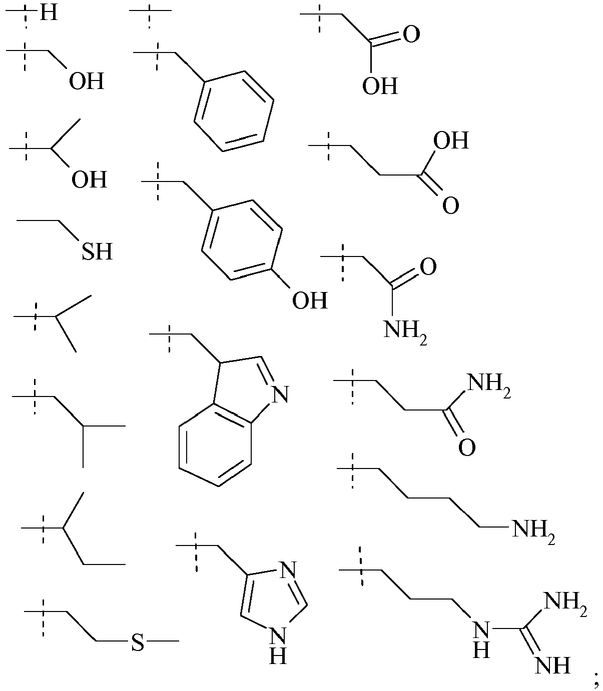 wherein dashed lines indicate attachment to the rest of the molecule,
   and wherein in the structure given in (XIIIb) a hydrogen is replaced by a bond for the attachment to the protein carrier PC (m=0) or to the spacer moiety SP (m=1), and wherein the structure given in (XIIIb) is optionally further substituted, and wherein PC, SP, and m have the meaning as defined in formula (2).

Preferably, in formula (XIIIa) and (XIIIb) one of the pair R³/R^{3a} is H and the other one is selected from R⁵.

Preferably, in formula (XIIIa) and (XIIIb) one of R⁴/R^{4a} is H.

Optionally, in formula (XIIIa) and (XIIIb) one or more of the pairs R³/R^{3a}, R⁴/R^{4a}, R³/R⁴ may independently form one or more cyclic fragments selected from C₃₋₇ cycloalkyl, 4- to 7-membered heterocyclyl, and 9- to 11-membered heterobicyclyl.

Optionally, in formula (XIIIa) and (XIIIb) R³, R^{3a}, R⁴ and R^{4a} are further substituted; suitable substituents are alkyl (such as C₁₋₆ alkyl), alkenyl (such as C₂₋₆ alkenyl), alkynyl (such as C₂₋₆ alkynyl), aryl (such as phenyl), heteroalkyl, heteroalkenyl, heteroalkynyl, heteroaryl (such as aromatic 4- to 7-membered heterocycle) or halogen moieties.

Suitable substituents in formula (XIIIa) and (XIIIb) are alkyl (such as C₁₋₆ alkyl), alkenyl (such as C₂₋₆ alkenyl), alkynyl (such as C₂₋₆ alkynyl), aryl (such as phenyl), heteroalkyl, heteroalkenyl, heteroalkynyl, heteroaryl (such as aromatic 4- to 7-membered heterocycle) or halogen moieties.

In yet another preferred embodiment, a sub-structure (PC)ₚ-(SP)ₘ-L- in formula (1) for the water-soluble protein carrier-linked prodrug has the structure as given in formula (XIVa): 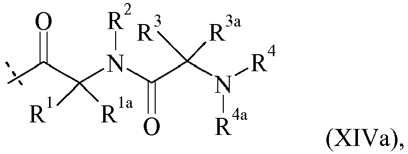 wherein the dashed line indicates the attachment to an aromatic amino group of a biologically active moiety D by forming an amide bond; and wherein R¹, R^{1a}, R², R^{2a}, R³, R^{3a}, R⁴ and R^{4a} of formula (XIVa) are defined as follows:
R¹, R^{1a}, R², R³, R^{3a}, R⁴ and R^{4a} are independently selected from H and C₁₋₄ alkyl;
and wherein the structure given in (XIVa) is substituted with p protein carrier moieties PC, either directly or through a spacer moiety SP (m=0 or 1), and wherein the structure given in (XIVa) is optionally further substituted, and wherein p, PC, SP, and m have the meaning as defined in formula (1).

In another preferred embodiment, a sub-structure L in formula (2) for the water-soluble protein carrier-linked prodrug has the structure as given in formula (XIVb): 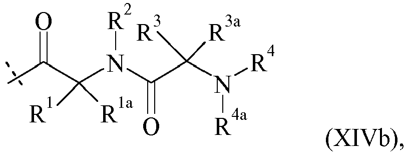 wherein the dashed line indicates the attachment to an aromatic amino group of a biologically active moiety D by forming an amide bond; and wherein R¹, R^{1a}, R², R^{2a}, R³, R^{3a}, R⁴ and R^{4a} of formula (XIVb) are defined as follows:
R¹, R^{1a}, R², R³, R^{3a}, R⁴ and R^{4a} are independently selected from H and C₁₋₄ alkyl;
and wherein in the structure given in (XIVb) a hydrogen is replaced by a bond for the attachment to the protein carrier PC (m=0) or to the spacer moiety SP (m=1), and wherein the structure given in (XIVb) is optionally further substituted, and wherein PC, SP, and m have the meaning as defined in formula (2).

Optionally, in formula (XIVa) and (XIVb) any two of R¹, R^{1a}, R², R³, R^{3a}, R⁴ and R^{4a} may independently form one or more cyclic fragments selected from C₃₋₇ cycloalkyl, 4- to 7-membered heterocyclyl, phenyl, naphthyl, indenyl, indanyl, tetralinyl, and 9- to 11-membered heterobicyclyl.

Optionally, in formula (XIVa) and (XIVb) R¹, R^{1a}, R², R³, R^{3a}, R⁴ and R^{4a} are further substituted; suitable substituents are alkyl, such as C₁₋₆ alkyl; alkene, such as such as C₂₋₆ alkene; alkine, such as such as C₂₋₆ alkine; aryl, such as phenyl; heteroalkyl; heteroalkene; heteroalkine; heteroaryl such as aromatic 4- to 7-membered heterocycle; or halogen moieties.

Suitable substituents in formula (XIVa) and (XIVb) are alkyl (such as C₁₋₆ alkyl), alkenyl (such as C₂₋₆ alkenyl), alkynyl (such as C₂₋₆ alkynyl), aryl (such as phenyl), heteroalkyl, heteroalkenyl, heteroalkynyl, heteroaryl (such as aromatic 4- to 7-membered heterocycle) or halogen moieties.

Preferably, in formula (XIVa) and (XIVb) one of R⁴ or R^{4a} is H.

In yet another preferred embodiment, a reversible prodrug linker moiety L suitable for biologically active moieties is described in US patent No 7585837. Therefore, a preferred sub-structure (PC)ₚ-(SP)ₘ-L- in formula (1) for the water-soluble protein carrier-linked prodrug has the structure as given in formula (XVa): 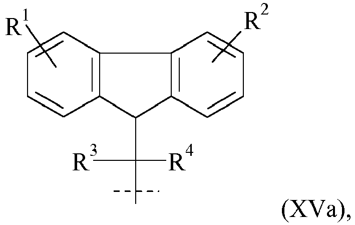 wherein the dashed line indicates the attachment to a functional group of the biologically active moiety D, wherein such functional group is selected from amino, carboxyl, phosphate, hydroxyl and mercapto; and wherein R¹, R², R³ and R⁴ of formula (XVa) are defined as follows:
R¹ and R² are independently selected from the group consisting of hydrogen, alkyl, alkoxy, alkoxyalkyl, aryl, alkaryl, aralkyl, halogen, nitro, -SO₃H, -SO₂NHR⁵, amino, ammonium, carboxyl, PO₃H₂, and OPO₃H₂;
R³, R⁴, and R⁵ are independently selected from the group consisting of hydrogen, alkyl, and aryl;
and wherein the structure given in (XVa) is substituted with p protein carrier moieties PC, either directly or through a spacer moiety SP (m=0 or 1), and wherein the structure given in (XVa) is optionally further substituted, and wherein p, PC, SP, and m have the meaning as defined in formula (1).

In yet another preferred embodiment, a reversible prodrug linker moiety L suitable for biologically active moieties is described in US patent No 7585837. Therefore, a preferred sub-structure L in formula (2) for the water-soluble protein carrier-linked prodrug has the structure as given in formula (XVb): 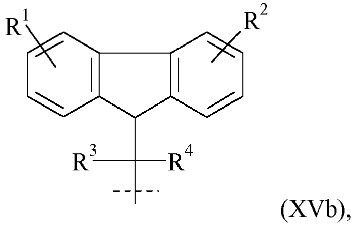 wherein the dashed line indicates the attachment to a functional group of the biologically active moiety D, wherein such functional group is selected from amino, carboxyl, phosphate, hydroxyl and mercapto; and wherein R¹, R², R³ and R⁴ of formula (XVb) are defined as follows:
R¹ and R² are independently selected from the group consisting of hydrogen, alkyl, alkoxy, alkoxyalkyl, aryl, alkaryl, aralkyl, halogen, nitro, -SO₃H, -SO₂NHR⁵, amino, ammonium, carboxyl, PO₃H₂, and OPO₃H₂;
R³, R⁴, and R⁵ are independently selected from the group consisting of hydrogen, alkyl, and aryl;
and wherein in the structure given in (XVb) a hydrogen is replaced by a bond for the attachment to the protein carrier PC (m=0) or to the spacer moiety SP (m=1), and wherein the structure given in (XVb) is optionally further substituted, and wherein PC, SP, and m have the meaning as defined in formula (2).

Suitable substituents for formulas (XVa) and (XVb) are alkyl (such as C₁₋₆ alkyl), alkenyl (such as C₂₋₆ alkenyl), alkynyl (such as C₂₋₆ alkynyl), aryl (such as phenyl), heteroalkyl, heteroalkenyl, heteroalkynyl, heteroaryl (such as aromatic 4 to 7 membered heterocycle) or halogen moieties.

Yet another preferred reversible prodrug linker moiety L is described in the international application WO-A 2002/089789. Therefore, the general formula (XVIa) is a preferred embodiment for the water-soluble carrier-linked prodrug according to formula (1), in which m is 1: 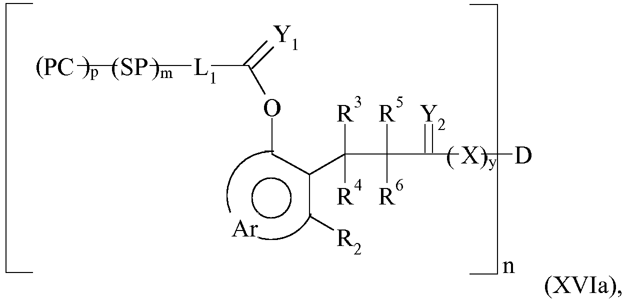 wherein D, PC, p, SP, m, n, L₁, X, y, Ar, Y₁, Y₂, R², R³, R⁴, R⁵, R⁶ of formula (XVIa) are defined as follows:
D is a biologically active moiety D of formula (1),
SP, m, PC, p, and n are used as defined in formula (1),
L₁ is a bifunctional linking group,
Y₁ and Y₂ are independently O, S or NR⁷;
R¹⁻⁷ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy, and C₁₋₆ heteroalkoxy;
Ar is a moiety which when included in formula (XVIa) forms a multisubstituted aromatic hydrocarbon or a multi-substituted heterocyclic group;
X is a chemical bond or a moiety that is actively transported into a target cell, a hydrophobic moiety, or a combination thereof,
y is 0 or 1.

Yet another preferred reversible prodrug linker moiety L is described in the international application WO-A 2002/089789. Therefore, the general formula (XVIb) is a preferred embodiment for a sub-structure 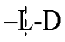 for the water-soluble carrier-linked prodrug according to formula (2): 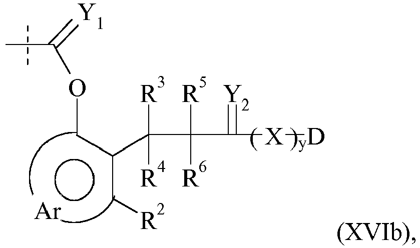 wherein
the dashed line indicates attachment to SP (if m=1) or PC (if m=0) of formula (2), and
D, L₁, X, y, Ar, Y₁, Y₂, R², R³, R⁴, R⁵, R⁶ of formula (XVIb) are defined as follows:
   D is a biologically active moiety D of formula (2),
   L₁ is a bifunctional linking group,
   Y₁ and Y₂ are independently O, S or NR⁷,
   R¹⁻⁷ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy, and C₁₋₆ heteroalkoxy,
   Ar is a moiety which when included in formula (XVIa) forms a multisubstituted aromatic hydrocarbon or a multi-substituted heterocyclic group,
   X is a chemical bond or a moiety that is actively transported into a target cell, a hydrophobic moiety, or a combination thereof,
   y is 0 or 1.

If in formula (2) m = 1, then SP of formula (2) is connected to v sub-structures of formula (XVIb). If in formula (2) m = 0, then PC of formula (2) is connected to t sub-structures of formula (XVIb).

A preferred reversible prodrug linker moiety for amine-comprising drugs is described in WO-A 2001/47562. Therefore, general formula (XVIIa) is a preferred embodiment for the water-soluble carrier-linked prodrug according to formula (1), in which m is 1: 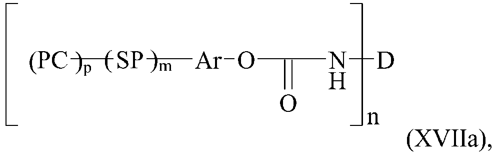 wherein SP, m, PC, p, n, Ar and D of formula (XVIIa) have the following meaning:
D is an amine-comprising biologically active moiety D of formula (I),
SP, m, PC, p, and n are used as defined in formula (1),
Ar is an aromatic group.

A preferred reversible prodrug linker moiety for amine-comprising drugs is described in WO-A 2001/47562. Therefore, general formula (XVIIb) is a preferred embodiment for a sub-structure 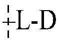 for the water-soluble carrier-linked prodrug according to formula (2): 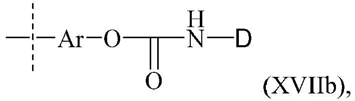 wherein
the dashed line indicates attachment to SP (if m=1) or PC (if m=0) of formula (2), and
Ar and D of formula (XVIIb) have the following meaning:
   D is an amine-comprising biologically active moiety D of formula (2) comprising NH,
   Ar is an aromatic group.

If in formula (2) m = 1, then SP of formula (2) is connected to v sub-structures of formula (XVIIb). If in formula (2) m = 0, then PC of formula (2) is connected to t sub-structures of formula (XVIIb).

A preferred reversible prodrug linker moiety for heteroaromatic amine-comprising drugs is described in US patent 7393953 B2. Therefore, general formula (XVIIIa) is a preferred embodiment for the water-soluble carrier-linked prodrug according to formula (1), in which m is 1: 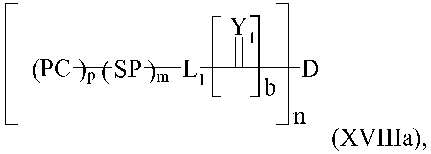 wherein SP, m, PC, p, n, D, L₁, Y₁, and b of formula (XVIIIa) have the following meaning:
D is a heteroaromatic amine-comprising biologically active moiety D of formula (1), SP, m, PC, p, and n are used as defined in formula (1),
Y₁ is O, S, or NR₂,
b is 0 or 1,
L₁ is a bifunctional linker, such as, for example, -NH(CH₂CH₂O)c(CH₂)NR₃-, -NH(CH₂CH₂O)_{c}C(O)-, -NH(CR₄R₅)_{c}OC(O)-, -C(O)(CR₄R₅)_{c}NHC(O)(CR₈R₇)_{d}NR₃, -C(O)O(CH₂)_{c}O-, -C(O)(CR₄R₅)_{c}NR₃-, -C(O)NH(CH₂CH₂O)_{c}(CH₂)_{c}NR₃-, -C(O)O-(CH₂CH₂O)_{c}NR₃-, -C(O)NH(CR₄R₅)_{c}O-, -C(O)O(CR₄R₅)_{c}O, -C(O)NH(CH₂CH₂O)_{c}-, 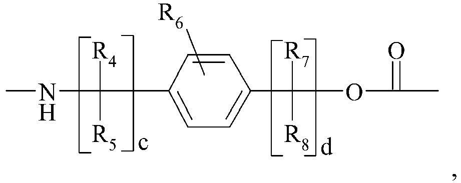 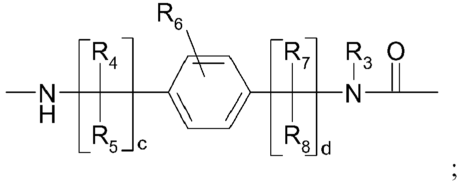
R₂, R₃, R₄, R₅, R₇ and R₈ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
R⁶ is selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy, NO₂, haloalkyl and halogen; and
c and d are selected independently from each other and each is a positive integer.

A preferred reversible prodrug linker moiety for heteroaromatic amine-comprising drugs is described in US patent 7393953 B2. Therefore, general formula (XVIIIb) is a preferred embodiment for a sub-structure 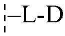 for the water-soluble carrier-linked prodrug according to formula (2): 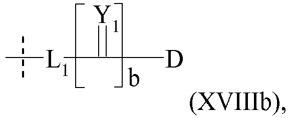 wherein
the dashed line indicates attachment to SP (if m=1) or PC (if m=0) of formula (2), and
Ar, D L₁, Y₁, and b of formula (XVIIIb) have the following meaning:
   D is a heteroaromatic amine-comprising biologically active moiety D of formula (2),
   Y₁ is O, S, or NR₂,
   b is 0 or 1,
   L₁ is a bifunctional linker, such as, for example,-NH(CH₂CH₂O)_{c}(CH₂)_{c}NR₃-, -NH(CH₂CH₂O)_{C}C(O)-, -NH(CR₄R₅)_{c}OC(O)-, -C(O)(CR₄R₅)_{c}NHC(O)(CR₈R₇)_{d}NR₃, -C(O)O(CH₂)_{c}O-, -C(O)(CR₄R₅)_{c}NR₃-, -C(O)NH(CH₂CH₂O)_{c}(CH₂)_{c}NR₃-, -C(O)O-(CH₂CH₂O)_{c}NR₃-, -C(O)NH(CR₄R₅)_{c}O-, -C(O)O(CR₄R₅)_{c}O, -C(O)NH(CH₂CH₂O)_{c}-, 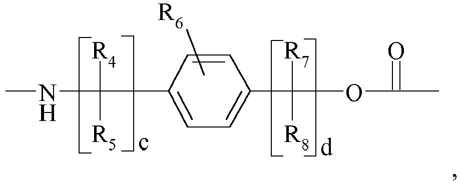 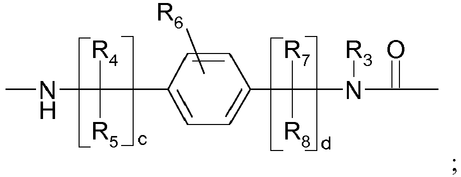
   R₂, R₃, R₄, R₅, R₇ and R₈ are independently selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy;
   R₆ is selected from the group consisting of hydrogen, C₁₋₆ alkyls, C₃₋₁₂ branched alkyls, C₃₋₈ cycloalkyls, C₁₋₆ substituted alkyls, C₃₋₈ substituted cycloalkyls, aryls, substituted aryls, aralkyls, C₁₋₆ heteroalkyls, substituted C₁₋₆ heteroalkyls, C₁₋₆ alkoxy, phenoxy and C₁₋₆ heteroalkoxy, NO₂, haloalkyl and halogen; and
   c and d are selected independently from each other and each is a positive integer.

If in formula (2) m = 1, then SP of formula (2) is connected to v sub-structures of formula (XVIIIb). If in formula (2) m = 0, then PC of formula (2) is connected to t sub-structures of formula (XVIIIb).

General formula (XIXa) is another preferred embodiment for the water-soluble carrier-linked prodrug according to formula (1), in which m is 1: 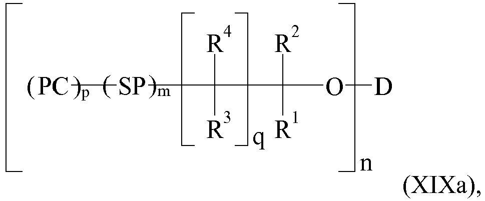 wherein
PC, p, SP, m, q, D, R¹, R², R³, R⁴ and n of formula (XIXa) have the following meaning:
D is a carboxyl-comprising biologically active moiety comprising O,
SP, m, PC, p, and n are used as defined in formula (1),
R¹ is selected from the group of unsubstituted alkyl; substituted alkyl; unsubstituted phenyl; substituted phenyl; unsubstituted naphthyl; substituted naphthyl; unsubstituted indenyl; substituted indenyl; unsubstituted indanyl; substituted indanyl; unsubstituted tetralinyl; substituted tetralinyl; unsubstituted C₃₋₁₀ cycloalkyl; substituted C₃₋₁₀ cycloalkyl; unsubstituted 4- to 7-membered heterocyclyl; substituted 4- to 7-membered heterocyclyl; unsubstituted 9- to 11-membered heterobicyclyl; and substituted 9- to 11-membered heterobicyclyl;
R² is selected from H, unsubstituted alkyl, and substituted alkyl;
R³ and R⁴ are independently selected from the group consisting of H, unsubstituted alkyl, and substituted alkyl;
q is 0 or 1,
optionally, R¹ and R³ are joined together with the atoms to which they are attached to form a ring A;
A is selected from the group consisting of C₃₋₁₀ cycloalkyl; 4- to 7-membered aliphatic heterocyclyl; and 9- to 11-membered aliphatic heterobicyclyl, wherein A is unsubstituted or substituted.

Preferably, R¹ of formula (XIXa) is C₁₋₆ alkyl or substituted C₁₋₆ alkyl, more preferably C₁₋₄ alkyl or substituted C₁₋₄ alkyl.

More preferably, R¹ of formula (XIXa) is selected from methyl, ethyl, n-propyl, isopropyl, n-butyl, isobutyl, sec-butyl, t-butyl, and benzyl.

Preferably, R² of formula (XIXa) is H.

Preferably, R³ of formula (XIXa) is H, C₁₋₆ alkyl or substituted C₁₋₆ alkyl, more preferably C₁₋₄ alkyl or substituted C₁₋₄ alkyl. More preferably, R³ is selected from methyl, ethyl, n-propyl, isopropyl, n-butyl, isobutyl, sec-butyl, t-butyl, and benzyl.

More preferably, R³ of formula (XIXa) is H.

Preferably, R⁴ of formula (XIXa) is s H, C₁₋₆ alkyl or substituted C₁₋₆ alkyl, more preferably C₁₋₄ alkyl or substituted C₁₋₄ alkyl. More preferably, R⁴ is selected from methyl, ethyl, n-propyl, isopropyl, n-butyl, isobutyl, sec-butyl, t-butyl, and benzyl.

More preferably, R⁴ of formula (XIXa) is H.

In another preferred embodiment, R¹ and R³ of formula (XIXa) are joined together with the atoms to which they are attached to form a ring A, wherein A is selected from the group consisting of cyclopropane, cyclobutane, cyclopentane, cyclohexane, and cycloheptane.

General formula (XIXb) is another preferred embodiment for a sub-strucuture 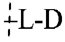 for the water-soluble carrier-linked prodrug according to formula (2): 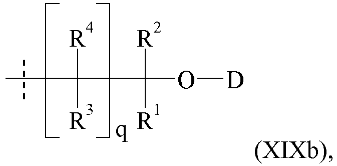 wherein
the dashed line indicates attachment to SP (if m=1) or PC (if m=0) of formula (2), and
D, q, R¹, R², R³, and R⁴ of formula (XIXb) have the following meaning:
   D is a carboxyl-comprising biologically active moiety comprising O,
   R¹ is selected from the group of alkyl, substituted alkyl, aryl, substituted aryl, cycloalkyl and cycloheteroalkyl,
   R² is selected from H, alkyl, and substituted alkyl,
   R³ and R⁴ are independently selected from the group consisting of H, alkyl, and substituted alkyl,
   q is 0 or 1,
   optionally, R¹ and R³ are joined together with the atoms to which they are attached to form a ring A,
   A is selected from the group consisting of C₃₋₁₀ cycloalkyl, 4- to 7-membered aliphatic heterocyclyl, and 9- to 11-membered aliphatic heterobicyclyl.

Preferably, R¹ of formula (XIXb) is C₁₋₆ alkyl or substituted C₁₋₆ alkyl, more preferably C₁₋₄ alkyl or substituted C₁₋₄ alkyl.

More preferably, R¹ of formula (XIXb) is selected from methyl, ethyl, n-propyl, isopropyl, n-butyl, isobutyl, sec-butyl, t-butyl, and benzyl.

Preferably, R² of formula (XIXb) is H.

Preferably, R³ of formula (XIXb) is H, C₁₋₆ alkyl or substituted C₁₋₆ alkyl, more preferably C₁₋₄ alkyl or substituted C₁₋₄ alkyl. More preferably, R³ is selected from methyl, ethyl, n-propyl, isopropyl, n-butyl, isobutyl, sec-butyl, t-butyl, and benzyl.

More preferably, R³ of formula (XIXb) is H.

Preferably, R⁴ of formula (XIXb) is s H, C₁₋₆ alkyl or substituted C₁₋₆ alkyl, more preferably C₁₋₄ alkyl or substituted C₁₋₄ alkyl. More preferably, R⁴ is selected from methyl, ethyl, n-propyl, isopropyl, n-butyl, isobutyl, sec-butyl, t-butyl, and benzyl.

More preferably, R⁴ of formula (XIXb) is H.

If in formula (2) m = 1, then SP of formula (2) is connected to v sub-structures of formula (XIXb). If in formula (2) m = 0, then PC of formula (2) is connected to t sub-structures of formula (XIXb).

General formula (XXa) is another preferred embodiment for the water-soluble carrier-linked prodrug according to formula (1), in which m is 1: 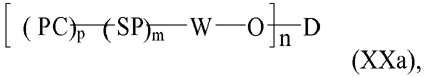 wherein
PC, p, SP, m, n, D, and W of formula (XX) have the following meaning:
D is a carboxyl-comprising biologically active moiety comprising O,
SP, m, PC, p, and n are used as defined in formula (1),
W is selected from linear C₁₋₁₅ alkyl.

General formula (XXb) is another preferred embodiment for sub-structures 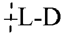 for the water-soluble carrier-linked prodrug according to formula (2): 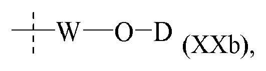 wherein
the dashed line indicates attachment to SP (if m=1) or PC (if m=0) of formula (2), and
D and W of formula (XXb) have the following meaning:
   D is a carboxyl-comprising biologically active moiety comprising O,
   W is selected from linear C₁₋₁₅ alkyl.

If in formula (2) m = 1, then SP of formula (2) is connected to v sub-structures of formula (XXb). If in formula (2) m = 0, then PC of formula (2) is connected to t sub-structures of formula (XXb).

A water-soluble protein carrier-linked prodrug comprises one or more moiety D which may be selected from the group of peptides, polypeptides, proteins, oligonucleotides, or small molecule biologically active moieties. A biologically active moiety D may comprise one or more functional groups, such as, for example, amine, hydroxyl, carboxyl, phosphate, or mercapto, through which a moiety D is connected to a moiety L.

Suitable aromatic amine-containing drugs are, for example, (-)-Carbovir, (±)-Hymenin, (±)-Norcisapride, (±)-Picumeterol, (R)-Aminoglutethimide, (R)-Clenbuterol, (S)-Aminoglutethimide, (S)-Clenbuterol, [6-p-aminophenylalanine]-angiotensin II, 10'-Demethoxystreptonigrin, 17-Aminogeldanamycin, 1-Aminoacridine, 1-Deazaadenine, 1-NA-PP 1, 1-NM-PP 1, 2,7-Diaminoacridine, 2,7-Dimethylproflavine, 2-Amino-6(5H)-phenanthridinone, 2-Aminoacridine, 2-amino-Carbanilide, 2-Aminohistamine, 2-Aminoperimidine, 2'-AMP, 2-Chloroadenosine, 2'-Deoxyxylotubercidin, 2-Sulfanilamidoimidazole, 3,4-Diaminocoumarin, 3'-Amino-4'-methoxyflavone, 3-Aminoacridine, 3-Aminopicolinic acid, 3-Deazaguanine, 4'-Aminoflavone, 4-Aminopyridine, 5'-ADP, 5-Aminoacridine, 5-amino-DL-Tryptophan, 5-Aminonicotinamide, 5'-AMP, 5'-ATP, 5-Chlorodeoxycytidine, 5'-CMP, 5-Dimethylamiloride, 5'-GDP, 5'-GMP, 5'-GTP, 5-Iodotubercidin, 5-Methylcytosine, 6-Aminoflavone, 6-Aminophenanthridine, 6-Aminothymine, 6-Benzylthioguanine, 6-Chlorotacrine, 6-Iodoamiloride, 7,8- Dihydroneopterin, 7-Aminonimetazepam, 7-Methoxytacrine, 7-Methyltacrine, 9-Deazaguanine, 9-Phenethyladenine, Abacavir, Acadesine, Acediasulfone, Acefurtiamine, Acetyl coenzyme A, Aciclovir, Actimid, Actinomycin, Acyclovir, Adefovir, Adenallene, Adenine, Adenophostin A, Adenosine, Adenosine monophosphate, Adenosine triphosphate, Adenosylhomocysteine, Aditeren, Afloqualone, Alamifovir, Albofungin, Alfuzosin, Allithiamine, Alpiropride, Amanozine, Ambasilide, Ambucaine, Amdoxovir, Ameltolide, Amethopterin, Amfenac, Amflutizole, Amicycline, Amidapsone, Amifampridine, Amiloride, Aminacrine, Aminoacridine, Aminoantipyrine, Aminobenzoate, Aminogenistein, Aminoglutethimide, Aminohippurate, Aminoisatin, Aminometradine, Aminonimetazepam, Aminophenylalanine, Aminopotentidine, Aminopterin, Aminopurvalanol A, Aminoquinuride, Aminosalicylic Acid, Amiphenazole, Amiphenosine, Amisometradine, Amisulpride, Amiterol, Amlexanox, Ammelin, Amonafide, Amoxecaine, Amphenidone, Amphethinile, Amphotalide, Amprenavir, Ampurine, Amrinone, AMT, Amthamine, Amtizole, Angustmycin A, Anileridine, Apadenoson, Apraclonidine, Apricitabine, Arafluorocytosine, Aramine, Arazide, Aristeromycin, Arprinocid, Ascamycin, Ascensil, Aspiculamycin, Atolide, Azabon, Azacitidine, Azaline B, Azamulin, Azanidazole, Azepexole, Aztreonam, Baquiloprim, Basedol, Batanopride, b-D-Adenosine, Bemitradine, Benfotiamine, Bentiamine, Benzamil, Benzocaine, Betoxycaine, Binodenoson, Biopterin, Bisbentiamine, Blasticidin, Bleomycin, Bleomycin A1, Bleomycin A2, Bleomycin A5, Bleomycin A6, Bleomycin DMA2, Brodimoprim, Bromfenac, Bromobuterol, Bromopride, Bropirimine, Buciclovir, Bunazosin, Butyrylthiamine disulfide, Cadeguomycin, cAMP, Candicidin, Capadenoson, Carbanilide, Carbodine, Carbovir, Carbutamide, Carumonam, CDP-dipalmitin, Cefcapenepivoxil, Cefclidin, Cefdaloxime, Cefdinir, Cefditoren,Cefempidone, Cefepime, Cefetamet, Cefetecol, Cefixime, Cefluprenam, Cefmatilen, Cefmenoxime, Cefodizime, Cefoselis, Cefotaxime, Cefotiam, Cefozopran, Cefpodoxime, Cefquinome, Cefrom, Ceftazidime, Cefteram, Ceftibuten, Ceftiofur, Ceftiolene, Ceftioxide, Ceftizoxime, Ceftobiprole, Ceftriaxone, Cefuzonam, Centazolone, Cetotiamine, cGMP, Chloroprocaine, Cidofovir, Cifostodine, Cipamfylline, Cisapride, Cladribine, Clafanone, Claforan, Clebopride, Clenbuterol, Clenproperol, Clofarabine, Clorsulon, Coelenteramine, Coenzyme A, Colchicamid, Coumarin 10, Coviracil, Crotonoside, Cyclobut A, Cyclobut G, Cycloclenbuterol, Cycotiamine, Cytallene, Cytarabine, Cytarazid, Cytidine, Cytidine diphosphate, Cytidoline, CytosineD-(+)-Neopterin, Dactinomycin, D-Amethopterin, dAMP, Damvar, Daniquidone, Dapsone, Daptomycin, Daraprim, Darunavir, DATHF, Dazopride, dCMP, dCTP, Debromohymenialdisine, Decitabine, Declopramide, Deisopropylhydroxyatrazine, Delafloxacin, Delfantrine, Denavir, Deoxyadenosine, Deoxy-ATP, Deoxycytidine, Deoxyguanosine, Dephosphocoenzyme A, Dequalinium, Desbutylbumetanide, Desciclovir, Desoxyminoxidil, dGMP, dGTP, Diacethiamine, Diaminoacridine, Diaveridine, Dichlorobenzamil, Dichloromethotrexate, Dichlorophenarsine, Dideoxycytidine, Dihydrobiopterin, Dihydrofolic acid, Dimethialium, Dimethocaine, Dimethyl methotrexate, Dinalin, DL-5,6,7,8-Tetrahydrofolic acid, DL-Methotrexate, Dobupride, Dovitinib, Doxazosin, Draflazine, Edatrexate, Elpetrigine, Elvucitabine, Emtricitabine, Entecavir, Enviradene, Epcitabine, Epiroprim, Eritadenine, Etanterol, Ethacridine, Ethaden, Ethylisopropylamiloride, Etoprine, Etoxazene, Etravirine, Etriciguat, FAD, Famciclovir, Fazarabine, Fenamol, Fepratset, Fiacitabine, Flucytosine, Fludara, Fludarabine, Fluocytosine, Folic acid, Formycin A, Fosamprenavir, Furalazine, Fursultiamine, Furyltriazine, Ganciclovir, Gancyclovir, Gastracid, Gemcitabine, Giracodazole, Gloximonam, Glybuthiazol, GSK 3B Inhibitor XII, GSK3BInhibitorXII, Guanine, Guanine arabinoside, Guanosine, Hexyl PABA, Hydroxymethylclenbuterol, Hydroxyprocaine, Hydroxytriamterene sulfate, Ibacitabine, Iclaprim, Imanixil, Imiquimod, Indanocine, Iobenzamic acid, Iocetamic acid, Iomeglamic acid, Iomeglamicacid, Ipidacrine, Iramine, Irsogladine, Isatoribine, Isobutamben, Isoritmon, Isosepiapterin, Ketoclenbuterol, Ketotrexate, Kopexil, Lamivudine, Lamotrigin, Lamotrigine, Lamtidine, Lappaconine, Lavendamycin, L-Cytidine, Lenalidomide, Leucinocaine, Leucovorin, L-g-Methylene-10-deazaaminopterin, Linifanib, Lintopride, Lisadimate, Lobucavir, Lodenosine, Lomeguatrib, Lometrexol, Loxoribine, L-S-Adenosylmethionine, Mabuterol, Medeyol, Melarsenoxyd, Melarsoprol B, Mesalazine, Metabutethamine, Metabutoxycaine, Metahexamide, Metazosin, Methioprim, Methotrexate, Methylanthranilate, Metioprim, Metoclopramide, Metoprine, Minoxidil, Mirabegron, Mitomycin, Mivobulin, Mocetinostat, Monocain, Mosapride, Mutamycin, N-(p-Aminophenethyl)spiroperidol, N6-[2-(4-aminophenyl)ethyl]adenosine Role, NAD+, NADH, NADH2, NADP+, NADPH2, Naepaine, Naminterol, Naretin, Nebidrazine, NECA, Nelarabine, Nelzarabine, Neolamin, Neotropine, Nepafenac, Nerisopam, Neurofort, Nifurprazine, Nimustine, Nitrine, N-Methyltetrahydrofolic acid, Nolatrexed, Nomifensine, Norcisapride, N-Propionylprocainamide, N-Sulfanilylnorfloxacin, o-Aminophenylalanine, Octotiamine, Olamufloxacin, Ormetoprim, Orthocaine, Oximonam, Oxybuprocaine, p-Aminoantipyrine, p-Aminobenzoate, p-Amino-D-phenylalanine, Pancopride, Parsalmide, Pasdrazide, Pathocidine, Pelitrexol, Pemetrexed, Penciclovir, Peplomycin, Peralopride, Phenamil, Phenazone, Phenazopyridine, Phenyl p-aminobenzoate, Phenyl-PAS-Tebamin, Phleomycin D1, Pibutidine, Picumeterol, Pirazmonam, Piridocaine, Piritrexim, Porfiromycin, Pralatrexate, Pramipexole, Prazobind, Prazosin, Preladenant, Procainamide, Procaine, Proflavine, Proparacaine, Propoxycaine, Prosultiamine, Prucalopride, Pseudoisocytidine, Psicofuranine, Pteridoxamine, Pteroyltriglutamic acid, Pyramine, Pyrimethamine, Questiomycin, Quinelorane, Racivir, Regadenoson, Renoquid, Renzapride, Resiquimod, Resorcein, Retigabine, Reverset, Riluzole, Rociclovir, Rufocromomycin, S-Adenosylmethionine, Sangivamycin, Sapropterin, S-Doxazosin, Sepiapterine, Silversulfadiazine, Sinefungin, Sipatrigine, Sparfloxacin, Sparsomycin, Stearyl-CoA, Stearylsulfamide, Streptonigrin, Succisulfone, Sufamonomethoxine, Sulamserod, Sulfabromomethazine, Sulfacetamide, Sulfachlorpyridazine, Sulfachrysoidine, Sulfaclomide, Sulfaclorazole, Sulfaclozine, Sulfacytine, Sulfadiasulfone, Sulfadiazine, Sulfadicramide, Sulfadimethoxine, Sulfadimidine, Sulfadoxine, Sulfaethoxypyridazine, Sulfaguanidine, Sulfaguanole, Sulfalene, Sulfamerazine, Sulfamethazine, Sulfamethizole, Sulfamethoxazole, Sulfamethoxydiazine, Sulfamethoxypyridazine, Sulfametomidine, Sulfametopyrazine, Sulfametrole, Sulfanilamide, Sulfanilamidoimidazole, Sulfanilylglycine, Sulfaperin, Sulfaphenazole, Sulfaproxyline, Sulfapyrazole, Sulfapyridine, Sulfasomizole, Sulfasymazine, Sulfathiadiazole, Sulfatroxazole, Sulfatrozole, Sulfisomidine, Sulfisoxazole, Tacedinaline, Tacrine, Talampanel, Talipexole, Talisomycin A, Tenofovir, Tenofovir disoproxil, Terazosin, Tetrahydrobiopterinm, Tetrahydrofolic acid, Tetroxoprim, Tezacitabine, Thiamine, Thiazosulfone, Thioguanine, Tiamiprine, Tigemonam, Timirdine, Tinoridine, Tiodazosin, Tirapazamine, Tiviciclovir, Tocladesine, Trancopal, Triacanthine, Triamterene, Triapine, Triciribine, Trimazosin, Trimethoprim, Trimetrexate, Tritoqualine, Troxacitabine, Tubercidin 5'-diphosphate, Tuvatidine, Tyrphostin AG 1112, Valacyclovir, Valganciclovir, Valopicitabine, Valtorcitabine, Velnacrine, Vengicide, Veradoline, Vidarabine, Viroxime, Vitaberin, Zalcitabine, Zhengguangmycin B2, Zinviroxime, Zorbamycin, Zoxazolamine, (±)-Saxitoxin, 2-Aminoperimidine, 6-Formylpterin, 8-13-Neurotensin, 8-Thioguanosine, 9-Deazaguanosine, 9-Desarginine-bradykinin, a4-10-Corticotropin, Afamelanotide, Agmatine, Alarelin, Ambazone, Amiloride, Aminopterine, Ampyrimine, Angiotensin, Angiotensin I, Angiotensin II, Antibiotic 0-129, Antipain, Arginine, Argiprestocin, Astressin, Atriopeptin III, Aviptadil, Benzylisothiourea, Betacyamine, Bisindolylmaleimide IX, Bivalirudin, Blasticidin S, Bleomycin B2, Bombesin 14, Buformin, Camostat, Cariporide, Carperitide, Cecropin P 1, Cetrorelix, Cilengitide, Creapure, Cyanoginosin LR, Cyanoviridin RR, Dalargine, Damvar, Deazaaminopterin, Defensin HNP 1, Deslorelin, Desmopressin, Dezaguanine, Dichloromethotrexate, Dihydrostreptomycin, Dimaprit, Dimethylamiloride, Diminazene, DL-Methotrexate, D-Methotrexate, Ebrotidine, Edatrexate, Eel Thyrocalcitonin, Elastatinal, Elcatonin, Enterostatin, Enviomycin, Eptifibatide, Ethylisopropylamiloride, Etilamide, Etoprine, Famotidine, Flupirtine, Furterene, Galanin, Galegin, Ghrelin, Glucagon, Gonadoliberin A, Guanethidine, Guanfacine, Guanoxan, Guanylthiourea, Gusperimus, Hexamidine, Histatin 5, Histrelin, Homoarginine, Icatibant, Imetit, Insulinotropin, Isocaramidine, Kallidin 10, Kemptide, Ketotrexate, Kiotorphin, Lactoferricin, Lamifiban, L-Bradykinin, Leucoverin, Leucovorin A, Leupeptin, Leuprolide, Lometrexol, Lutrelin, m-Chlorophenylbiguanide, Melagatran, Melanotan II, Melanotropin, Melittin, Metformin, Methotrexate dimethyl ester, Methotrexate monohydrate, Methoxtrexate, Methylisothiourea, Metoprine, Miacalcin, MIBG, Minoxidil, Mitoguazone, Mivobulin, Mivobulin isethionate, Moroxydine, Nafarelin, Neotine, Nesiritide, Netropsin, Neurotensin, N-Methyltetrahydrofolate, Nociceptin, Nolatrexed, Novastan, Panamidin, Pathocidine, Pebac, Peldesine, Pelitrexol, Pemetrexed, Pentamidine, Peramivir, Phenformine, Phenylbiguanide, Pig galanin, Pimagedine, Piritrexim, Pitressin, Porcine angiotensinogen, Porcine gastrin-releasing hormone, Porcine neuropeptide Y, Porcine PHI, Pralatrexate, Protein Humanin, Proteinase inhibitor E 64, Pyrimethamin, Quinespar, Rat atriopeptin, Rat atriopeptin, Resiquimod, Ribamidine, Rimorphin, Saralasin, Saxitoxin, Sermorelin, S-Ethylisothiourea, Spantide, Stallimycin, Stilbamidine, Streptomycin A, Substance P free acid, Sulfaguanidine, Synthetic LH-releasing hormone, Tallimustine, Teprotide, Tetracosactide, Tetrahydrobiopterin, Tetrahydrofolic acid, Thrombin receptor-activating peptide-14, Thymopentin, Tioguanin, Tiotidine, Tirapazamine, Triamteren, Trimetrexate, Tryptorelin, Tuberactinomycin B, Tuftsin, Urepearl, Viomycidin, Viprovex, Vitamin M, Xenopsin, Zanamivir, Zeocin, Ziconotide, Zoladex.

Suitable drugs with an amine group may be selected from the group consisting of Aphidicolin Glycinate, Cetrorelix Acetate, Picumeterol Fumarate, (-)-Draflazine, (-)-Indocarbazostatin B, (+)-(23,24)-Dihydrodiscodermolide, (+)-(R)-Pramipexole, (R)-(+)-Amlodipine, (R)-(+)-Terazosin, (R)-Ganciclovir Cyclic Phosphonate, (R)-Sufinosine, (R)-Zacopride, (S)-(-)-Norketamine, (S)-Oxiracetam, (S)-Sufinosine, (S)-Zacopride Hydrochloride, [90Y]-DOTAGA-Substance P, [ARG(Me)9] MS-10, [D-TYRI,ARG(Me)9] MS-10, [D-TYR1,AzaGLY7,ARG(Me)9] MS-10, [D-TYR1] MS-10, [Psi(CH2NH)TPG4]Vancomycin Aglycon, [TRP19] MS-10, 111IN-Pentetreotide, 13-Deoxyadriamycin Hydrochloride, 17-Aminogeldanamycin, 19-O-Methylgeldanamycin, 1-Methyl-D-Tryptophan, 21-Aminoepothilone B, 2-Aminoaristeromycin, 2-Aminoneplanocin A, 3-Chloroprocainamide, 3-Deazaadenosine, 3-Matida, 4-Aminosalicylic Acid, 4-Chlorophenylthio-DADME-Immucillin-A, 5,4'-Diepiarbekacin, 5'-Homoneplanocin A, 5-Aminosalicylic Acid, 8(R)-Fluoroidarubicin Hydrochloride, 99MTC-C(RGDFK*)2Hynic, 9-Aminocamptothecin, A-42867 Pseudoaglycone, Abacavir Succinate, Abacavir Sulfate, Abanoquil Mesilate, Abarelix, Acadesine, Acriflavine, Acyclovir, Acyclovir Elaidate, Acyclovir Oleate, Acyline, Adefovir, Adefovir Dipivoxil, Ademetionine Tosylate Sulfate, Adenallene, Adenophostin A, Adenophostin B, Adenosine, Aerothricin 1, Aerothricin 16, Aerothricin 41, Aerothricin 45, Aerothricin 5, Aerothricin 50, Aerothricin 55, Afloqualone, Ageliferin Diacetate, Ageliferin Dihydrochloride, Aladapcin, Alamifovir, Alatrofloxacin Mesilate, Alendronic Acid Sodium Salt, Alestramustine, Alfuzosin Hydrochloride, Aliskiren Fumarate, Alogliptin Benzoate, Alpha-Methylnorepinephrine, Alpha-Methyltryptophan, Altemecidin, Alvespimycin Hydrochloride, Amantadine Hydrochloride, Ambasilide, Ambazone, Ambroxol Nitrate, Amdoxovir, Ameltolide, Amelubant, Amezinium Methylsulfate, Amfenac Sodium, Amidox, Amifostine Hydrate, Amikacin, Amiloride Hydrochloride, Aminocandin, Aminoglutethimide, Aminoguanidine, Aminolevulinic Acid Hexyl Ester, Aminolevulinic Acid Methyl Ester, Amisulpride, Amlodipine, Amlodipine Besylate, Amoxanox, Amoxicillin Pulsys, Amphotericin B, Ampicillin Sodium, Amprenavir, Ampydin, Amrinone, Amrubicin Hydrochloride, Amselamine Hydrobromide, Amthamine, Anakinra, Anamorelin Hydrochloride, Anatibant Mesilate, Angiopeptin Acetate, Anisperimus, Antagonist-G, Antide, Antide-1, Antide-2, Antide-3, Antileukinate, Apadenoson, Apixaban, Aplonidine Hydrochloride, Apoptozole 1, Apoptozole 2, Apoptozole 3, Apricitabine, Arbekacin, Arbekacin sulfate, Arborcandin A, Arborcandin B, Arborcandin C, Arborcandin D, Arborcandin E, Arborcandin F, Argatroban Monohydrate, Argimesna, Arginine Butyrate, Argiotoxin-636, Armodafinil, Arotinolol Hydrochloride, Arterolane Maleate, Aspoxicillin, Atenolol, Atosiban, Atreleuton, Avorelin, Azacytidine, Azalanstat, Azaromycin SC, Azelnidipine, Azetirelin, Azodicarbonamide, Azoxybacilin, Aztreonam, Aztreonam L-Lysine, Azumamide A, Baclofen, Bactobolin, Balapiravir Hydrochloride, Balhimycin, Barusiban, Batracylin, Belactin A, Belactosin A, Belactosin C, Benanomicin B, Benexate Cyclodextrin, Benzocaine, Besifloxacin Hydrochloride, Beta-Amyloid (12-20), Binodenoson, Bleomycin A2 Sulfate, Boceprevir, Bogorol A, Boholmycin, Brasilicardin A, Bremelanotide, Brivanib Alaninate, Brivaracetam, Brodimoprim, Bromfenac Sodium, Bromhexine Hydrochloride, Brostallicin Hydrochloride, Bunazosin Hydrochloride, Buserelin Acetate, Butabindide, Butamidine, Buteranol, Cabin 1, Calcium-Like Peptide 1, Calcium-Like Peptide 2, Cambrescidin 800, Cambrescidin 816, Cambrescidin 830, Cambrescidin 844, Camostat, Canfosamide Hydrochloride, Capadenoson, Capeserod Hydrochloride, Capravirine, Caprazamycin A, Caprazamycin B, Caprazamycin C, Caprazamycin E, Caprazamycin F, Capromorelin, Carafiban Maleate, Carbachol, Carbamazepine, Carbetocin, Carbovir, Carboxyamidotriazole, Cariporide Hydrochloride, Carisbamate, Carpipramine, Carumonam Sodium, Caspofungin Acetate, Cefaclor, Cefcanel Daloxate Hydrochloride, Cefcapene Pivoxil Hydrochloride, Cefdaloxime, Cefdaloxime Pentexil Tosilate, Cefdinir, Cefditoren Pivoxil, Cefepime, Cefetamet Pivoxil, Cefetecol, Cefixime, Cefluprenam, Cefmatilen Hydrochloride Hydrate, Cefmenoxime Hydrochloride, Cefminox Sodium, Cefodizime, Cefodizime Sodium, Cefoselis Sulfate, Cefotaxime Sodium, Cefotetan Disodium, Cefotiam Hexetil, Cefotiam Hexetil Hydrochloride, Cefotiam Hydrochloride, Cefoxitin, Cefozopran, Cefozopran Hydrochloride, Cefpirome, Cefpodoxime Proxetil, Cefprozil, Cefprozil Monohydrate, Cefquinome, Ceftaroline, Ceftazidime, Cefteram Pivoxil, Ceftibuten, Ceftobiprole, Ceftobiprole Medorcaril, Ceftrazonal Bopentil, Ceftrazonal Sodium, Ceftriaxone Sodium, Ceftrizoxime Alapivoxil, Cefuroxime, Cefuroxime Axetil, Cefuroxime Pivoxetil, Centanamycin, Cephalexin Monohydrate, Ceranapril, Ceruletide Diethylamine, Cetefloxacin, Chlorofusin, Chloroorienticin A, Chloroorienticin B, Chlorotetain, Cibrostatin 1, Cidofovir, Cilastatin Sodium, Cilengitide, Cimaterol, Cinitapride Hydrogen Tartrate, Cipamfylline, Circinamide, Cisapride Hydrate, Cispentacin, Citicoline, Citrullimycine A, Cladribine, Clitocine, Clofarabine, Clopidogrel Sulfate, Compound 301029, Coumamidine Gamma1, Coumamidine Gamma2, Cromoglycate Lisetil Hydrochloride, Cycallene, Cyclic-Cidofovir, Cycloserine, Cyclotheonamide A, Cyclothialidine, Cygalovir, Cypemycin, Cysmethynil, Cystamidin A, Cystamine, Cystazosin, Cystocin, Cytarabine, Cytarabine Ocfosfate, Cytaramycin, Cytochlor, Cytomodulin, Dabigatran , Dabigatran Etexilate, Dacopafant, Dactimicin, Dactinomycin, Dactylocycline A, Dactylocycline B, DADME-Immucillin-G, Dalargin, Danegaptide Hydrochloride, Dapropterin Dihydrochloride, Dapsone, Darbufelone Mesilate, Darifenacin Hydrobromide, Darinaparsin, Darunavir, Daunorubicin, Davasaicin, Davunetide, Debrisoquine Sulfate, Decahydromoenomycin A, Decaplanin, Deferoxamine, Degarelix Acetate, Delafloxacin, Delta-Aminolevulinic Acid Hydrochloride, Deltibant, Denagliptin Hydrochloride, Denibulin Hydrochloride, Denufosol Tetrasodium, Deoxymethylspergualin, Deoxynegamycin, Deoxyvariolin B, Desacetylvinblastinehydrazide/Folate Conjugate, Des-F-Sitagliptin, Desglugastrin Tromethamine, Deslorelin, Desmopressin Acetate, Detiviciclovir Diacetate, Dexelvucitabine, Dexibuprofen Lysine, Dextroamphetamine Sulfate, Dezinamide, Dezocitidine, Diadenosine Tetraphosphate, Diaveridine, Dichlorobenzoprim, Dicloguamine Maleate, Didemnin X, Didemnin Y, Dideoxycytidine, Difurazone, Dilevalol, Dilevalol Hydrochloride, Disermolide, Disopyramide Phosphate, DI-VAL-L-DC, Docosyl Cidofovir, Dolastatin 14, Dolastatin C, Donitriptan Hydrochloride, Donitriptan Mesilate, Dovitinib Lactate, Doxazosin Mesylate, Doxorubicin Hydrochloride, Doxycycline Hyclate, D-Penicillamine, Draflazine, Droxidopa, DTPA-Adenosylcobalamin, Ebrotidine, Ecenofloxacin Hydrochloride, Efegatran Sulfate Hydrate, Eflornithine Hydrochloride, Eglumegad Hydrate, Eicosyl Cidofovir, Elacytarabine, Elastatinal B, Elastatinal C, Elpetrigine, Elvucitabine, Emtricitabine, Enalkiren, Enigmol, Eniporide Mesilate, Entecavir, Entinostat, Epinastine Hydrochloride, Epiroprim, Epirubicin Hydrochloride, Epithalon, Epofolate, Epostatin, Epsilon Aminocaproic Acid, Eremomycin, Eribulin Mesylate, Erucamide, Esafloxacine Hydrochloride, Eslicarbazepine Acetate, Etaquine, Ethanolamine, Ethylthio-DADME-Immucillin-A, Ethynylcytidine, Etravirine, Etriciguat, Exalamide, Examorelin, Exatecan Mesilate, Ezatiostat Hydrochloride, Famciclovir, Famotidine, Famotidine Bismuth Citrate, Favipiravir, Feglymycin, Felbamate, Fenleuton, Fidarestat, Fidexaban, Filaminast, Filarizone, Fingolimod Hydrochloride, Flucytosine, Fludarabine Phosphate, Fluorobenzyltriamterene, Fluorominoxidil, Fluoroneplanocin A, Flupiritine Maleate, Fluvirucin B2, Fluvoxamine Maleate, Folinic Acid, Fortimicin A, Fosamprenavir Calcium, Fosamprenavir Sodium, Fosfomycin Trometamol, Fradafiban, Freselestat, Frovatriptan, Fudosteine, Furamidine, G1 Peptide, Gabadur, Gabapentin, Gabexate Mesilate, Galarubicin Hydrochloride, Galmic, Galnon, Ganciclovir, Ganciclovir Elaidic Acid, Ganciclovir Monophosphate, Ganciclovir Sodium, Ganirelix, Ganirelix Acetate, Garomefrine Hydrochloride, Gemcitabine, Gemcitabine Elaidate, Gemifloxacin Mesilate, Gilatide, Girodazole, Glaspimod, Glucosamine Sulfate, Gludopa, Glutathione Monoethylester, Glutathione Monoisopropylester, Glycine-Proline-Melphalan, Glycopin, Glycothiohexide alpha, Golotimod, Goserelin, Growth Factor Antagonist-116, Growth Hormone Releasing Peptid 2, Guanabenz Acetate, Guanadrel Sulfate, Guanethidine Monosulfate, Guanfacine Hydrochloride, Gusperimus Hydrochloride, Halovir A, Halovir B, Halovir C, Halovir D, Halovir E, Hayumicin B, Hayumicin C1, Hayumicin C2, Hayumicin D, Helvecardin A, Helvecardin B, Hepavir B, Heptaminol AMP Amidate, Hexa-D-Arginine, Hexadecyl Cidofovir, Hexadecyloxypropyl-Cidofovir, Histamine Dihydrochloride, Histaprodifen, Histrelin, Histrelin Acetate, Human Angiotensin II, Hydrostatin A, Hydroxyakalone, Hydroxyurea, Hypeptin, Ibutamoren Mesilate, Icatibant Acetate, Iclaprim, Icofungipen, Idarubicin Hydrochloride, Ilatreotide, Ilonidap, Imetit, Imidafenacin, Imidazenil, Imiquimod, Immunosine, Impentamine, Incyclinide, Indanocine, Indantadol Hydrochloride, Indoxam, Inogatran, Intrifiban, Iobenguane[131I], Iodorubidazone (P), Iotriside, Isepamicin Sulfate, Isobatzelline A, Isobatzelline B, Isobatzelline C, Isobatzelline D, Isobutyramide, Isodoxorubicin, Isopropamide Iodide, Ispinesib Mesylate, Istaroxime, Janthinomycin A, Janthinomycin B, Janthinomycin C, Jaspine B, Kahalalide F, Kaitocephalin, Kanamycin, Karnamicin B1, Katanosin A, Katanosin B, Kistamicin A, L-4-Oxalysine, Labetalol Hydrochloride, Labradimil, Lagatide, Lamifiban, Lamivudine, Lamotrigine, Lanicemine 2(S)-Hydroxysuccinate, Lanicemine Hydrochloride, Lanomycin, Larazotide Acetate, Lazabemide Hydrochloride, L-Dopa Methyl Ester Hydrochloride, L-Dopamide, Lecirelin, Lenalidomide, Lenampicillin Hydrochloride, Leucettamine A, Leucovorin Calcium, Leuprolide Acetate, Leurubicin, Leustroducsin A, Leustroducsin B, Leustroducsin C, Leustroducsin H, Levetiracetam, Levodopa, Levodopa 3-O-Glucoside, Levodopa 4-O-Glucoside, Levoleucovorin Calcium, L-Histidinol, L-Homothiocitrulline, Liblomycin, Linagliptin, Linifanib, Lintopride, Lirexapride, Lirimilast, Lisinopril, L-Lysine-D-Amphetamine Dimesylate, Lobophorin A, Lobucavir, Lodenosine, Loloatin B, Lomeguatrib, Lometrexol, Lonafarnib, Loracarbef Hydrate, Loviride, Loxoribine, L-Simexonyl Homocysteine, L-Thiocitrulline, Lymphostin, Lysobactin, Mabuterol Hydrochloride, Makaluvamine A, Makaluvamine A, Makaluvamine B, Makaluvamine C, Managlinat Dialanetil, Matristatin A2, Melagatran, Melanotan II, Memantine Hydrochloride, Memno-Peptide A, Meprobamate, Meriolin-3, Mersacidin, Metaraminol, Metazosin, Metformin Hydrochloride, Methotrexate, Methyl Bestatin, Methyldopa, Methylthio-DADME-Immucillin-A, Metoclopramide Hydrochloride, Metyrosine, Mexiletine Hydrochloride, Micafungin Sodium, Midaxifylline, Mideplanin, Midoriamin, Milacainide Tartrate, Milacemide-[2H], Milnacipran Hydrochloride, Minamestane, Minocycline Hydrochloride, Minoxidil, Mirabegron, Mitomycin, Mivazerol, Mivobulin Isethionate, Mizoribine, Mocetinostat Dihydrobromide, Modafinil, Modafinil Sulfone, Moenomycin A Chloride Bismuth Salt, Mofegiline, Mofegiline Hydrochloride, Monamidocin, Monodansyl Cadaverine, Montirelin Tetrahydrate, Mosapride Citrate, Moxilubant, Moxilubant Maleate, Mozenavir Mesilate, M-Phenylene Ethynylene, Muraminomicin A, Muraminomicin B, Muraminomicin C, Muraminomicin D, Muraminomicin E1, Muraminomicin E2, Muraminomicin F, Muraminomicin G, Muraminomicin H, Muraminomicin I, Muraminomicin Z1, Muraminomicin Z2, Muraminomicin Z3, Muraminomicin Z4, Muramyl Dipeptide C, Mureidomycin A, Mureidomycin B, Mureidomycin C, Mureidomycin D, Mycestericin E, Myriocin, Nafamostat Mesylate, Nafarelin Acetate, Naglivan, Namitecan, Napsagatran, Nebostinel, Nebracetam Fumarate, Neldazosin, Nelzarabine, Nemonoxacin, Neomycin B-Hexaarginine Conjugate, Neomycin-Acridine, Nepafenac, Nepicastat Hydrochloride, Neramexane Hydrochloride, Neridronic Acid, Netamiftide Trifluoroacetate, Netilmicin Sulfate, Nocathiacin I, Nocathiacin II, Nocathiacin III, Nocathiacin IV, NO-Gabapentin, Nolatrexed Hydrochloride, NO-Mesalamine, Noraristeromycin, Nuvanil, O6-Benzylguanine, Ocimumoside A, Octacosamicin A, Octacosamicin B, Octreother, Octreotide Acetate, Oglufanide Disodium, Olamufloxacin, Olamufloxacin Mesilate, Olcegepant, Olradipine Hydrochloride, Omaciclovir, Ombrabulin, Ombrabulin Hydrochloride, Onnamide A, Opiorphin, Orbofiban Acetate, Orienticin A, Orienticin B, Orienticin C, Orienticin D, Oritavancin, Oseltamivir Carboxylate, Oseltamivir Phosphate, Otamixaban, Otenabant Hydrochloride, Ovothiol A, Oxazofurin, Oxcarbazepine, Oxiglutatione Sodium, Oxiracetam, Oxolide, Oxynor, Oxyphenarsine, Ozarelix, Pachymedusa Dacnicolor Tryptophyllin-1, Paecilaminol, Pafuramidine Maleate, PalauÀmine, Paldimycin B, Pamidronate Sodium, Pancopride, Papuamide A, Papuamide B, Papuamide C, Papuamide D, Parasin I, Paromomycin, Pasireotide, Paulomycin, Paulomycin A2, Paulomycin B, Paulomycin C, Paulomycin D, Paulomycin E, Paulomycin F, Pazufloxacin, Pazufloxacin Mesilate, PEG-Vancomycin, Pelagiomicin C, Peldesine, Pelitrexol, Pemetrexed Disodium, Penciclovir, Penicillin G Procaine, Pentamidine Gluconate, Pentamidine Isethionate, Pentamidine Lactate, Peplomycin, Peramivir, Perphanazine 4-Aminobutyrate, Phakellistatin 5, PHE-ARG-Beta-Naphthylamide, Phentermine, Phortress, Phospholine, Pibutidine Hydrochloride, Pimeloylanilide O-Aminoanilide, Piracetam, Pirarubicin, Pivampicillin, Pixantrone Maleate, Pluraflavin A, Pluraflavin B, Plusbacin A1, Plusbacin A2, Plusbacin A3, Plusbacin A4, Plusbacin B1, Plusbacin B2, Plusbacin B3, Plusbacin B4, PMEO-5-ME-DAPY, Pneumocandin A0, Pneumocandin B0, Pneumocandin B0 2-Phosphate, Pneumocandin D0, Polaprezinc, Polydiscamide A, Polymer Bound Human Leukocyte Elastase Inhibitor, Poststatin, PPI17-24, Pradimicin E, Pradimicin FA-2, Pralatrexate, Pramipexole Hydrochloride, Pranedipine Tartrate, Prazosin Hydrochloride, Prefolic A, Pregabalin, Preladenant, Primaquine Phosphate, Probestin, Procainamide Hydrochloride, Procaine Hydrochloride, Pro-Diazepam, Prostatin, Prucalopride, Prucalopride Hydrochloride, Prucalopride Succinate, Pseudomycin A', Pseudomycin B', Pyloricidin B, Pyradizomycin, Pyrazinamide, Pyrazinoylguanidine, Pyriferone, Pyrimethamine, Quinelorane Hydrochloride, R-(+)-Aminoindane, Ralfinamide, Ramoplanin A'1, Ramoplanin A'2, Ramoplanin A'3, Ramorelix, Ravidomycin N-oxide, Razaxaban Hydrochloride, Reblastatin, Regadenoson, Relcovaptan, Remacemide Hydrochloride, Resiquimod, Restricticin, Retaspimycin Hydrochloride, Retigabine Hydrochloride, Rhodopeptin C1, Rhodopeptin C2, Rhodopeptin C3, Rhodopeptin C4, Rhodostreptomycin A, Rhodostreptomycin B, Ribavirin, Ribavirin Eicosenate cis, Ribavirin Eicosenate trans, Ribavirin Elaidate, Ribavirin Oleate, Rilmazafone Hydrochloride Dihydrate, Riluzole, Rimacalib Hydrochloride, Rimeporide Hydrochloride, Riociguat, Ritipenem Acoxil, Robalzotan Hydrochloride, Robalzotan Tartrate Hydrate, Rociclovir, Romurtide, Rotigaptide, Roxifiban Acetate, Ruboxyl, Rufinamide, Rumycin 1, Rumycin 2, Sabarubicin Hydrochloride, Sabiporide Mesilate, Safinamide Mesilate, Safingol, Sagamacin, Sampatrilat, Sampirtine, Saprisartan, Saquinavir, Saquinavir Mesilate, Sardomizide Hydrochloride, Sardomozide, Saussureamine C, Saxagliptin, Secobatzelline A, Secobatzelline B, Seglitide, Selank, Seletracetam, Semapimod Hydrochloride, Senicapoc, Sepimostat Mesilate, Seproxetine, Seraspenide, Sevelamer Carbonate, Sevelamer Hydrochloride, Shepherdin, Sibrafiban, Silodosin, Silver Sulfadiazine, Sipatrigine, Sitafloxacin Hydrate, Sitagliptin Phosphate Monohydrate, S-Nitrosoglutathione, Sofigatran, Sonedenoson, Sotirimod, Sparfloxacin, Sperabillin A, Sperabillin B, Sperabillin C, Sperabillin D, Sphingofungin F, Spinorphin, Spisulosine, Squalamine Lactate, Streptomycin, Styloguanidine, Substance P(8-11), Sufinosine, Sulcephalosporin, Sulfostin, Sulphazocine, Sultamicilline Tosylate, Sunflower Trypsin Inhibitor-1, Surfen, Synadenol, Synguanol, Tabimorelin, Tacedinaline, Tacrine Hydrochloride, Tageflar, Talabostat, Talaglumetad Hydrochloride, Talampanel, Talipexole Dihydrochloride, Tallimustine Hydrochloride, Talopterin, Taltirelin, Tanespimycin, Tanogitran, Targinine, Technetium (99MTC) Depreotide, Teicoplanin-A2-1, Teicoplanin-A2-2, Teicoplanin-A2-3, Teicoplanin-A2-3, Teicoplanin-A2-5, Telavancin Hydrochloride, Telinavir, Temozolomide, Temurtide, Tenidap, Tenidap Sodium, Tenofovir, Tenofovir DF, Terazosin Hydrochloride, Tetracosyl Cidofovir, Tetracycline Hydrochloride, Tetrafibricin, Texenomycin A, Tezacitabine, TGP, Thioacet, Thiothio, Thrazarine, Thymoctonan, Thymopentin, Tiamdipine, Tigecycline, Tilarginine Hydrochloride, Timirdine Diethanesulfonate, Timodepressin, Tipifarnib, TNF-Alpha Protease Enzyme Inhibitor, Tobramycin, Tocainide Hydrochloride, Tokaramide A, Tomopenem, Topostatin, Torcitabine, Tosufloxacin, Tosufloxacin Tosilate, Tranexamic Acid, Trantinterol Hydrochloride, Tranylcypromine Sulfate, Trelanserin, Tresperimus Triflutate, Trichomycin A, Triciribine, Triciribine Phosphate, Trientine Hydrochloride, Trimazosin Hydrochloride, Trimetrexate Glucuronate, Trimexautide, Trimidox, Trovafloxacin, Trovafloxacin Hydrate, Trovafloxacin Hydrochloride Mesylate, Trovafloxacin Mesilate, Troxacitabine, Trybizine Hydrochloride, Tubastrine, Tuftsin, Tyroservatide, Tyrphostin 47, Ubenimex, Valacyclovir, Valganciclovir Hydrochloride, Valnemulin, Valomaciclovir Stearate, Valonomycin A, Valopicitabine, Valpromide, Valrocemide, Vamicamide, Vancomycin Hydrochloride, Vancoresmycin, Vapitadine Hydrochloride, Varespladib, Varespladib Methyl, Varespladib Mofetil, Velnacrine Maleate, Venorphin, Vigabatrin, Vilazodone Hydrochloride, Vindesine, Viramidine Hydrochloride, Viranamycin-B, Vitamin B3, W Peptide, Xemilofiban, Xylocydine, Zanamivir, Zileuton, Zoniporide Hydrochloride, Zorubicin Hydrochloride.

Suitable secondary amine-containing drugs may be selected from the group consisting of (-)-3-O-Acetylspectaline hydrochloride, (-)-3-O-tert-Boc-spectaline hydrochloride, (-)-Cicloprolol, (-)-Norchloro-[18F]fluoro-homoepibatidine, (-)-Salbutamol hydrochloride, (-)-Salmeterol, (+)-(S)-Hydroxychloroquine, (+)-Isamoltan, (+)-R-Pramipexole, (R)-(+)-Amlodipine, (R)-Clevidipine, (R)-NSP-307, (R)-Teludipine, (R)-Thionisoxetine, (S)-Clevidipine, (S)-N-Desmethyltrimebutine, (S)-Noremopamil, [99Tc]Demobesin 4, [Glu10,Nle17,Nle30]-Pancreatic polypeptide(2-36), [Nle17,N1e30]-Pancreatic polypeptide(2-36), [psi[CH2NH]Tpg4]Vancomycin aglycon, 15bbeta-Methoxyardeemin, 3-Bromomethcathinone, 4,5-Dianilinophthalimide, 4-Hydroxyatomoxetine, 5-Methylurapidil, 7-Oxostaurosporine, 99mTc-c(RGDfK*)2HYNIC, A-42867 pseudoaglycone, Abacavir succinate, Abacavir sulfate, Abarelix, Acarbose, Acebutolol hydrochloride, Aceclofenac, Acyline, Adaphostin, Adaprolol maleate, Adaprolol oxalate, Adecypenol, Adrogolide hydrochloride, Aglaiastatin C, Alchemix, Alinidine, Alkasar-18, Alminoprofen, Alniditan, alpha-Methylepinephrine, Alprafenone hydrochloride, Alprenolol hydrochloride, Alprenoxime hydrochloride, Altromycin A, Altromycin C, Alvespimycin hydrochloride, Ambroxol nitrate, Amfebutamone hydrochloride, Amibegron hydrochloride, Amifostine hydrate, Amineptine, Aminocandin, Aminochinol, Amitivir, Amlodipine, Amlodipine besylate, Amocarzine, Amodiaquine, Amosulalol hydrochloride, Amoxapine, Amsacrine, Anabasine hydrochloride, Anisperimus, Antide-1, Aranidipine, Araprofen, Arbutamine hydrochloride, Ardeemin, Arformoterol tartrate, Argatroban monohydrate, Argiopine, Arotinolol hydrochloride, Asperlicin E, Atenolol, Atevirdine mesylate, Azathioprine, Azelnidipine, Azepinostatin, Balamapimod, Balhimycin, Balofloxacin, Balofloxacin dihydrate, Bambuterol, Bamirastine hydrate, Banoxantrone, Baogongteng A, Barixibat, Barnidipine hydrochloride, Batoprazine, Batzelline A, Batzelline B, Batzelline C, Becampanel, Bederocin, Bedoradrine sulfate, Befunolol hydrochloride, Belactin B, Belotecan hydrochloride, Benazepril hydrochloride, Bendroflumethiazide, Benidipine hydrochloride, Berlafenone hydrochloride, Betaxolol hydrochloride, Bevantolol hydrochloride, Biemnidin, Bifemelane hydrochloride, Binospirone mesylate, Bioxalomycin alpha 1, Bis(7)-cognitin, Bisantrene hydrochloride, Bisnafide mesilate, Bisoprolol fumarate, Bitolterol mesylate, Bleomycin A2 sulfate, Boholmycin, Bopindolol, Bosutinib, Brinazarone, Brinzolamide, Bulaquine, Bumetanide, Buteranol, Butofilolol, Cadrofloxacin hydrochloride, Caldaret hydrate, Calindol Dihydrochloride, Capridine beta, Carmoterol hydrochloride, Carteolol hydrochloride, Carvedilol, Caspofungin acetate, Ceftaroline fosamil acetate, Ceftizoxime sodium, Ceftobiprole, Celiprolol hydrochloride, Cerebrocrast, Ceruletide diethylamine, Cevipabulin, Chinoin-169, Chloptosin, Chlordiazepoxide hydrochloride, Chloroorienticin A, Chloroorienticin B, Cilazapril, Cilnidipine, Ciluprevir, Cimaterol, Cinacalcet hydrochloride, Cinnamycin, Ciprofloxacin hydrochloride, Ciprofloxacin silver salt, Clevidipine butyrate, Clitocine, Clopenphendioxan, Cloranolol hydrochloride, Clozapine, Conantokin-R, Conophylline, Crisnatol mesilate, Cronidipine, Dabelotine mesilate, Dabigatran, Dabigatran etexilate, Dalbavancin, Dapivirine, Dapropterin dihydrochloride, Dasantafil, Debromoshermilamine, Decaplanin, Degarelix acetate, Delapril hydrochloride, Delavirdine mesilate, Delfaprazine hydrochloride, Delucemine hydrochloride, Demethylallosamidin, Demexiptiline hydrochloride, Denopamine, Deoxymethylspergualin, Deoxyspergualin Hydrochloride, Desacetylvinblastinehydrazide/folate conjugate, Desbutyl benflumetol, Desbutylhalofantrine hydrochloride, Desferri-salmycin A, Desferri-salmycin B, Desferri-salmycin C, Desferri-salmycin D, Desipramine hydrochloride, Desloratadine, Dexfenfluramine hydrochloride, Dexketoprofen meglumine, Dexmethylphenidate hydrochloride, Dexniguldipine hydrochloride, Dexsotalol, Diazepinomicin, Dichlorobenzoprim, Diclofenac potassium, Diclofenac sodium, Diclofenac zinc salt, Diethylnorspermine, Dihydrexidine, Dilevalol, Dilevalol hydrochloride, Dinapsoline, Dinoxyline, Dipivefrine hydrochloride, Discodermide, Discodermide acetate, Discorhabdin D, Discorhabdin P, Discorhabdin S, Discorhabdin T, Discorhabdin U, Dobutamine hydrochloride, Dobutamine phosphate, Dopexamine, Dopexamine hydrochloride, Doripenem, Dorzolamide hydrochloride, d-Pseudoephedrine hydrochloride, Droxinavir, Duloxetine hydrochloride, Duocarmycin A, Duocarmycin B1, Duocarmycin B2, Duocarmycin C1, Duocarmycin C2, Dynemicin A, Dynemicin C, Ebanicline, Ecteinascidin 1560, Ecteinascidin 722, Ecteinascidin 729, Ecteinascidin 736, Ecteinascidin 745, Ecteinascidin 770, Ecteinascidin 875, Efaroxan, Efegatran sulfate hydrate, Efepristin, Efonidipine hydrochloride ethanol, Elagolix sodium, Elansolid C1, Elarofiban, Elbanizine, Elgodipine hydrochloride, Elinafide mesilate, Elinogrel potassium, Elnadipine, Enalapril maleate, Enalapril nitrate, Enalaprilat, Enazadrem, Enkastin (D), Enkastin (D), Enkastin (D), Enkastin AD, Enkastin AE, Enkastin ID, Enkastin IE, Enkastin VD, Enkastin VE, Enoxacin, Epibatidine, Epostatin, Eremomycin, Ersentilide, Ersentilide hydrochloride, Ertapenem sodium, Esculeogenin A, Esculeoside A, Esmolol hydrochloride, Esperamicin A1, Etamsylate, Ethoxy-idazoxan, Eugenodilol, Ezlopitant, Falnidamol, Farglitazar, Fasobegron hydrochloride, Fasudil hydrochloride, Felodipine, Fenoldopam mesilate, Fenoterol hydrobromide, Fepradinol, Ferroquine, Ferulinolol, Finafloxacin hydrochloride, Flecainide acetate, Florbetaben, Florbetapir F 18, Flufenoxine, Flumezapine, Fluodipine, Fluoxetine hydrochloride, Fluparoxan, Flupirtine maleate, Foetidine 1, Foetidine 2, Folinic acid, Formoterol fumarate, Forodesine hydrochloride, Fosaprepitant dimeglumine, Fosopamine, Frovatriptan, Furnidipine, Furosemide, Gaboxadol, Gadobenic acid dimeglumine salt, Gadopentetate dimeglumine, Gadoterate meglumine, Galactomycin I, Galactomycin II, Garenoxacin mesilate, Gatifloxacin, Gefitinib, Glucolanomycin, Glutapyrone, Gosogliptin hydrochloride, Grepafloxacin hydrochloride, Gypsetin, Halofuginone hydrobromide, Helvecardin A, Helvecardin B, Herquline B, Hesperadin, Himastatin, Hispidospermidin, Homoepibatidine, Hydrochlorothiazide, Hydroflumethiazide, Hydroxychloroquine sulfate, Ibopamine, Idazoxan hydrochloride, Iganidipine hydrochloride, Imidapril, Imidapril hydrochloride, Imidazoacridinone, Imisopasem manganese, Immepip, Immepyr, Incadronate, Indacaterol, Indantadol hydrochloride, Indeloxazine hydrochloride, Indolmycin, Inogatran, Intoplicine, Iofetamine hydrochloride 1-123, Iptakalim hydrochloride, Isavuconazonium chloride hydrochloride, Isepamicin sulfate, Isofagomine tartrate, Isoquine, Ispronicline, Isradipine, Iturelix, Kaitocephalin, Ketamine hydrochloride, Kopsinine, Korupensamine A, Korupensamine B, Korupensamine C, Kosinostatin, Labedipinedilol A, Labedipinedilol B, Labetalol hydrochloride, Labradimil, Lacidipine, Ladasten, Ladostigil tartrate, Lagatide, Landiolol, Lapatinib ditosylate, Lenapenem hydrochloride, Lenapenem hydrochloride hydrate, Lerisetron, Leucovorin calcium, Levobetaxolol hydrochloride, Levobunolol hydrochloride, Levoleucovorin calcium, Levonebivolol, Liblomycin, Linaprazan, Lisinopril, Litoxetine, Lobenzarit sodium, Lodamin, Lofexidine hydrochloride, Lomefloxacin hydrochloride, Lorcaserin, Lotrafiban, Loviride, Lubazodone hydrochloride, Lumiracoxib, Mabuterol hydrochloride, Makaluvamine D, Makaluvamine E, Makaluvamine F, Makaluvone, Manidipine hydrochloride, Manifaxine hydrochloride, Manzamine B, Manzamine D, Maprotiline hydrochloride, Maropitant, Masnidipine hydrochloride, Mecamylamine hydrochloride, Meclofenamate sodium, Mefenamic acid, Mefloquine hydrochloride, Melagatran, Melogliptin, Meluadrine, Meluadrine tartrate, Memoquin, Mepindolol sulfate, Mepindolol transdermal patch, Meropenem, Methamphetamine hydrochloride, Methoctramine, Methyclothiazide, Methylhistaprodifen, Methylphenidate hydrochloride, Metipranolol, Metolazone, Metoprolol fumarate, Metoprolol succinate, Metoprolol tartrate, Mezacopride, Michellamine B, Microcin J25, Micronomicin sulfate, Midafotel, Milacemide-[2H], Minaprine hydrochloride, Mirabegron, Mitomycin, Mitoxantrone hydrochloride, Mivobulin isethionate, Modipafant, Moexipril hydrochloride, Moexiprilat, Montirelin tetrahydrate, Moranolin, Motesanib diphosphate, Moxifloxacin hydrochloride, Moxonidine hydrochloride hydrate, Muraminomicin I, Mureidomycin E, Mureidomycin F, Mureidomycins, N1,N8-Bisnorcymserine, Nadolol, Naproxen piperazine, Napsamycin A, Napsamycin B, Napsamycin C, Napsamycin D, Nardeterol, N-demethylated sildenafil, Nebivolol, Nemonapride, Neomycin-acridine, Neratinib, Netilmicin sulfate, Nicardipine hydrochloride, Nifedipine, Nifekalant hydrochloride, Niguldipine hydrochloride, Nilvadipine, Nimodipine, Nipradilol, Nisoldipine, Nitracrine dihydrochloride hydrate, Nitrendipine, Nitrofenac, Nitroso-nifedipine, Noberastine, Noberastine citrate, NO-ciprofloxacin, N-Octyl-beta-valienamine, Nolomirole hydrochloride, Norfloxacin, Norsegoline, Nortopixantrone hydrochloride, Nortriptyline hydrochloride, N-tert butyl isoquine, Oberadilol, Oberadilol monoethyl maleate, Odanacatib, Olanzapine, Olanzapine pamoate, Olradipine hydrochloride, Ontazolast, OPC-17083, Orbifloxacin, Orciprenaline sulphate, Orienticin A, Orienticin B, Orienticin C, Oritavancin, Osemozotan hydrochloride, Osutidine, Otenabant hydrochloride, Ovothiol B, Oxprenolol hydrochloride, Ozenoxacin, Pafenolol, Palau'amine, Palindore fumarate, Panobinostat, Parodilol hemifumarate, Parogrelil hydrochloride, Paroxetine, Paroxetine ascorbate, Paroxetine camsilate, Paroxetine hydrochloride, Paroxetine mesilate, Pazelliptine trihydrochloride, Pazelliptine trihydrochloride monohydrate, Pelitinib, Pelitrexol, Penbutolol sulfate, Pentostatin, Peplomycin, Perindopril, Perzinfotel, Phendioxan, Pibutidine hydrochloride, Picumeterol fumarate, Pindolol, Pirbuterol hydrochloride, Pittsburgh Compound B, Pixantrone maleate, Plerixafor hydrochloride, Polyglutamate camptothecin, Pozanicline hydrochloride, Pradimicin A, Pradimicin B, Pradimicin D, Pradimicin FA-1, Pradimicin FL, Pradimicin FS, Pradimicin L, Pradimicin S, Pradofloxacin, Pramipexole hydrochloride, Pranedipine tartrate, Pranidipine, Prefolic A, Premafloxacin, Premafloxacin hydrochloride, Premafloxacin magnesium, Primaquine phosphate, Prisotinol, Procaterol Hydrochloride Hemihydrate, Propafenone hydrochloride, Propranolol hydrochloride, Protriptyline hydrochloride, Proxodolol, Pumaprazole, Pyrindamycin A, Pyrindamycin B, Quinapril hydrochloride, Quinpramine, rac-Debromoflustramine E, Radezolid, Rafabegron, Ralfinamide, Ramipril, Rasagiline mesilate, Razupenem, Reboxetine mesilate, Repinotan, Repinotan hydrochloride, Reproterol hydrochloride, Retaspimycin hydrochloride, Retigabine hydrochloride, Rhodostreptomycin A, Rhodostreptomycin B, Rifabutin, Rilmenidine dihydrogen phosphate, Rimoterol hydrobromide, Risotilide, Rivanicline, Robenacoxib, Rolapitant hydrochloride, Safinamide mesilate, Sagandipine, Salbostatin, Salbutamol nitrate, Salbutamol sulfate, Salmaterol, Salmeterol xinafoate, Sarizotan hydrochloride, Saussureamine C, Sazetidine-A, Selodenoson, Sertraline, Sertraline hydrochloride, Setazindol, Sezolamide hydrochloride, Shishijimicin A, Shishijimicin B, Shishijimicin C, Sibanomicin, Sibenadet hydrochloride, Silodosin, Sitamaquine hydrochloride, Sivelestat sodium hydrate, Sofinicline, Solabegron hydrochloride, Solpecainol hydrochloride, Soraprazan, Sotalol hydrochloride, Sparfloxacin, Spermine dialdehyde, Spirapril, Spiroquinazoline, Squalamine lactate, Streptomycin, Stressin1-A, Sumanirole maleate, Suprofenac 1, Suprofenac 2, Suprofenac 3, Suronacrine maleate, Tafamidis meglumine, Tafenoquine succinate, Talarozole, Talibegron, Talibegron hydrochloride, Talniflumate, Talotrexin, Taltobulin, Taludipine hydrochloride, Tamsulosin hydrochloride, Tanespimycin, Tanogitran, Tauropyrone, Tazopsine, Tecalcet hydrochloride, Tecastemizole, Technetium (99mTc) apcitide, Technetium (99mTc) bicisate, Telatinib, Telavancin hydrochloride, Temacrazine mesilate, Temafloxacin hydrochloride, Temocapril hydrochloride, Terbutaline sulfate, Terodiline hydrochloride, Tertatolol hydrochloride, Tetracaine hydrochloride, Tetrahydrodercitin 1, Tetrindole, Tezampanel, Thiamet-G, Thiofedrine, Tiamdipine, Tiamenidine, Tianeptine sodium, Tiapafant, Tienoxolol hydrochloride, Tigecycline, Tilisolol hydrochloride, Timolol hemihydrate, Timolol maleate, Tinazoline hydrohloride, Tirofiban hydrochloride, Tizanidine hydrochloride, Toborinone, Tolfenamic acid, Tomatine, Tomoxetine hydrochloride, Topixantrone hydrochloride, Torasemide, Trabectedin, Trandolapril, Trandolaprilat, Trantinterol hydrochloride, Treprostinil diethanolamine, Tresperimus triflutate, Triacetyl dynemicin C, Trientine hydrochloride, Trifluproxim, Trimetazidine, Trimetrexate glucuronate, Trombodipine, Troxipide, Tulathromycin A, Tulathromycin B, Tulobuterol hydrochloride, Ufenamate, Ulifloxacin, Ulimorelin, Uncialamycin, Urapidil, Utibapril, Utibaprilat, Vabicaserin hydrochloride, Vancomycin hydrochloride, Vandetanib, Vanidipinedilol, Vaninolol, Vapitadine hydrochloride, Varenicline tartrate, Varlitinib, Vatalanib succinate, Vatanidipine, Vatanidipine hydrochloride, Vestipitant mesylate, Vicenistatin, Vildagliptin, Viloxazine hydrochloride, Vofopitant hydrochloride, Voglibose, Voreloxin, Xamoterol fumarate, Ximelagatran, Yttrium-90 edotreotide, Zabicipril hydrochloride, Zabiciprilat hydrochloride, Zabofloxacin hydrochloride, Zanapezil fumarate, Zelandopam hydrochloride, Zilpaterol, Zolmitriptan.

Suitable amine-containing drugs may also be selected from the group consisting of ACTH, adenosine deaminase, agalsidase, albumin, alfa-1 antitrypsin (AAT), alfa-1 proteinase inhibitor (API), alglucosidase, alteplase, anistreplase, ancrod serine protease, antibodies (monoclonal or polyclonal and fragments or fusions), antithrombin III, antitrypsins, aprotinin, asparaginases, biphalin, bone-morphogenic proteins, calcitonin (salmon), collagenase, DNase, endorphins, enfuvirtide, enkephalins, erythropoietins, factor VIIa, factor VIII, factor VIIIa, factor IX, fibrinolysin, fusion proteins, follicle-stimulating hormones, granulocyte colony stimulating factor (G-CSF), galactosidase, glucagon, glucagon-like peptides like GLP-1, glucocerebrosidase, granulocyte macrophage colony stimulating factor (GM-CSF), chorionic gonadotropin (hCG), hemoglobins, hepatitis B vaccines, hirudin, hyaluronidases, idurnonidase, immune globulins, influenza vaccines, interleukines (1 alfa, 1 beta, 2, 3, 4, 6, 10, 11, 12), IL-1 receptor antagonist (rhIL-1ra), insulins, interferons (alfa 2a, alfa 2b, alfa 2c, beta 1a, beta 1b, gamma 1a, gamma 1b), keratinocyte growth factor (KGF), lactase, leuprolide, levothyroxine, luteinizing hormone, lyme vaccine, natriuretic peptide, pancrelipase, papain, parathyroid hormone, PDGF, pepsin, phospholipase-activating protein (PLAP), platelet activating factor alcetylhydrolase (PAF-AH), prolactin, protein C, octreotide, secretin, sermorelin, superoxide dismutase (SOD), somatropins (growth hormone), somatostatin, streptokinase, sucrase, tetanus toxin fragment, tilactase, thrombins, thymosin, thyroid stimulating hormone, thyrothropin, transforming growth factors, tumor necrosis factor (TNF), TNF receptor-IgG Fc, tissue plasminogen activator (tPA), transferrin, TSH, urate oxidase, urokinase, Fab (fragment, antigen-binding), F(ab)2 fragments, Fc (fragment, crystallizable), pFc' fragment, Fv (fragment, variable), scFv (single-chain variable fragment), di-scFv/diabodies, bi-specific T-cell engager, CDRs (complementarity determining regions), single-domain antibodies (sdABs/Nanobodies), heavy chains (α, δ, ε, γ, µ) or heavy chain fragments, light chains (λ, κ) or light chain fragments, VH fragments (variable region of the heavy chain), VL fragments (variable region of the light chain), VHH fragments, VNAR fragments, shark-derived antibody fragments and affinity scaffold proteins, Kunitz domain-derived affinity scaffold proteins, centyrin-derived affinity scaffold proteins, ubiquitin-derived affinity scaffold proteins, lipocalin-derived affinity scaffold proteins, ankyrin-derived affinity scaffold proteins, Versabodies (disulfide-rich affinity scaffold proteins), fibronectin-derived affinity scaffold proteins, cameloid-derived antibody fragments and affinity scaffold proteins, llama-derived antibody fragments and affinity scaffold proteins, transferrin-derived affinity scaffold proteins, Squash-type protease inhibitors with cysteine-knot scaffold-derived affinity scaffold proteins.

Suitable drugs containing aromatic hydroxyl groups are, for example, (-)-cis-Resorcylide, (-)-Indocarbazostatin B, (-)-Salmeterol, (-)-Subersic acid, (+)-alpha-Viniferin, (+)-Etorphine, (+)-Indocarbazostatin, (+)-SCH-351448, (R)-Gossypol, (S)-(+)-Curcuphenol, (S)-Methylnaltrexone bromide, [8]-Gingerol, [Arg(Me)9] MS-10, [D-Tyr1,Arg(Me)9] MS-10, [D-Tyr1,AzaGly7,Arg(Me)9] MS-10, [D-Tyr1] MS-10, [psi[CH2NH]Tpg4]Vancomycin aglycon, [Trp19] MS-10, 13-Deoxyadriamycin hydrochloride, 14-Methoxymetopon, 14-Phenylpropoxymetopon, 18,19-Dehydrobuprenorphine hydrochloride, 2,12-Dimethyleurotinone, 2'-Hydroxymatteucinol, 2-Methoxyestradiol, 2-Methyleurotinone, 3,5-Dicaffeoylquinic acid, 3-Bromodiosmetine, 3-Bromodiosmine, 3-Chlorodiosmetine, 3-Chlorodiosmine, 4',7,8-Trihydroxyisoflavone, 4-Aminosalicylic acid, 4-Hydroxyatomoxetine, 4-Iodopropofol, 5-Iodofredericamycin A, 5Z-7-Oxozeaenol, 6-Carboxygenistein, 6-O-mPEG4-Nalbupine, 6-O-mPEG5-Nalbuphine, 7-Methylcapillarisin, 8(R)-Fluoroidarubicin hydrochloride, 8',9'-Dehydroascochlorin, 8-Carboxy-iso-iantheran A, 8-Paradol, 8-Prenylapigenin, 8-Prenylnaringenin, 9-Hydroxycrisamicin A, A-42867 pseudoaglycone, Abarelix, Acacetin, Aclarubicin, Acolbifene hydrochloride, Acotiamide hydrochloride hydrate, Acrovestone, Actinoplanone A, Actinoplanone B, Aculeacin Agamma, Adaphostin, Adarotene, Adxanthromycin A, Aerothricin 1, Aerothricin 16, Aerothricin 41, Aerothricin 45, Aerothricin 50, Aerothricin 55, Ajulemic acid, Alchemix, Aldifen, alpha-Mangostin, alpha-Methylepinephrine, alpha-Methylnorepinephrine, Alpha-Peltatin, Altromycin A, Altromycin B, Altromycin C, Altromycin D, Altromycins, Alvimopan hydrate, Alvocidib hydrochloride, Amamistatin A, Amamistatin B, Amarogentin, Amelubant, Amidox, Aminocandin, Amodiaquine, Amoxicillin trihydrate, Amrubicin Hydrochloride, Amurensin H, Anguillosporal, Anidulafungin, Ankinomycin, Annamycin, Annulin C, Antimycin A11, Antimycin A12, Antimycin A13, Antimycin A14, Antimycin A15, Antimycin A16, Apicularen A, Apicularen B, Apigenin, Apomine, Apomorphine hydrochloride, Arbidol, Arbutamine hydrochloride, Arformoterol tartrate, Artepillin C, Arzoxifene hydrochloride, Aspoxicillin, Atalaphillidine, Atalaphillinine, Atraric acid, Avorelin, Axitirome, Azaresveratrol, Azatoxin, Azepinostatin, Baicalein, Baicalin, Balhimycin, Balsalazide disodium, Banoxantrone, Bazedoxifene acetate, Bazedoxifene hydrochloride, Bedoradrine sulfate, Benadrostin, Benanomicin A, Benanomicin B, Benastatin A, Benastatin B, Benastatin C, Benastatin D, Benzbromarone, Berefrine, Berupipam maleate, beta-Mangostin, Biemnidin, Biochanin A, Bioxalomycin alpha 1, Bioxalomycin alpha2, Bismuth subsalicylate, Bisphenol, Bix, Bizelesin, Bogorol A, Brandisianin A, Brandisianin B, Brandisianin C, Brasilicardin A, Brevifolin carboxylic acid, Breynin A, Breynin B, Bromotopsentin, Buflomedil pyridoxalphosphate, Buprenorphine hydrochloride, Buserelin acetate, Butein, Buteranol, Butorphan, Butorphanol tartrate, Calebin A, Calocoumarin A, Caloporoside D, Caloporoside E, Caloporoside F, Calphostin A, Calphostin B, Calphostin C, Calphostin D, Calphostin I, Capillarisin, Capsazepine, Carbazomadurin A, Carbazomadurin B, Carbetocin, Carbidopa, Carmoterol hydrochloride, Caspofungin acetate, Cassigalol A, Cefetecol, Cefoperazone sodium, Cefpiramide sodium, Cefprozil, Cefprozil monohydrate, Cetrorelix Acetate, Chaetoatrosin A, Chafuroside, Chloroorienticin A, Chloroorienticin B, Chondramide A, Chondramide B, Chondramide C, Cinnatriacetin A, Cinnatriacetin B, cis-6-Shogaol, Citpressine I, Citreamicin-Alpha, Citreamicin-eta, Citrusinine-I, Clausenamine A, Combretastatin A-1, Combretastatin A-2, Combretastatin A-3, Combretastatin B-1, Combretastatin B-2, Combretastatin B-3, Combretastatin B-4, Combretastatin D-1, Combretastatin D-2, Complestatin, Coniferol Alcohol, Conophylline, Corynecandin, Cosalane, Crisamicin C, Crobenetine, Crobenetine hydrochloride, Curtisian A, Curtisian B, Curtisian D, Cyanidin Chloride Monohydrate, Cyclocommunol, Cycloproparadicicol, Cyclotheonamide A, Cyclothialidine, Cyrtominetin, Cytogenin, Cytosporone B, Cytotrienin I, Cytotrienin II, Dactylocycline A, Dactylocycline B, Dalargin, Dalbavancin, Damunacantal, Daphnodorin A, Daphnodorin B, Daphnodorin C ((-)-enantiomer), Darbufelone, Darbufelone mesilate, Daunorubicin, Daurichromenic acid, Davidigenin, Deacetyl moxisylyte hydrochloride, Decaplanin, Decyl gallate, Deferasirox, Dehydrozingerone, Delphinidin, Denopamine, Deoxymulundocandin, Dersalazine, Desacetylravidomycin N-oxide, Desglugastrin tromethamine, Deslorelin, Desmopressin acetate, Desvenlafaxine succinate, Dexanabinol, Dextrorphan, Dexylosylbenanomycin A, D-Fluviabactin, Diazaphilonic acid, Diazepinomicin, Dieckol, Diflunisal, Dihydrexidine, Dihydroavenanthramide D, Dihydrogranaticin B, Dihydrohonokiol B, Dihydroraloxifene, Dilevalol, Dilevalol hydrochloride, Dinapsoline, Dinoxyline, Dioncoquinone A, Dioncoquinone B, Dipotassium gossypolate, Dobutamine hydrochloride, Dobutamine Phosphate, Dopexamine, Dopexamine hydrochloride, Dosmalfate, Doxorubicin Hydrochloride, Doxorubicin, Morpholinyl, DoxoTam 12, Doxycycline hyclate, Dronabinol, Droxidopa, Duocarmycin B1, Duocarmycin B2, Duocarmycin C1, Duocarmycin C2, Dutomycin, Dynemicin A, Dynemicin C, Econazole Sulfosalicylate, Ecopipam, Ecteinascidin 1560, Ecteinascidin 722, Ecteinascidin 729, Ecteinascidin 736, Ecteinascidin 745, Ecteinascidin 757, Ecteinascidin 770, Ecteinascidin 875, Edotecarin, Edotreotide yttrium, Eflucimibe, Eflumast, Elansolid C1, Eldacimibe, Ellagic acid-4-gallate, Elliptinium acetate, Elsibucol, Eltrombopag olamine, Emodin, Enazadrem, Enofelast, Entacapone, ent-Estriol, Epidoxoform, Epigallocatechin-3-gallate, Epirubicin hydrochloride, Eplivanserin, Eplivanserin fumarate, Eplivanserin mesilate, Epocarbazolin A, Epocarbazolin B, Eprotirome, Eptazocine hydrobromide, Erabulenol A, Erabulenol B, Eremomycin, Estetrol, Estradiol, Estriol, Etalocib sodium, Etamsylate, Ethinylestradiol, Ethyl gallate, Etoposide, Eurotinone, Euxanthone, Evernimicin, Exifone, Ezetimibe, Fadolmidine hydrochloride, Feglymycin, Fenoldopam mesilate, Fenoterol hydrobromide, Fidaxomicin, Fidexaban, Fluostatin A, Fluostatin B, Foetidine 1, Foetidine 2, Folipastatin, Formobactin, Formoterol fumarate, Fosopamine, Frederine, Fulvestrant, Furaquinocin A, Furaquinocin B, Fusacandin A, Fusacandin B, Fusidienol, Galactomycin I, Galactomycin II, Galarubicin hydrochloride, Galocitabine, Gambogic acid, gamma-Mangostin, gamma-Tocotrienol, Ganirelix, Ganirelix acetate, Garvalone C, Garveatin E, Garveatin F, Genistein-7-phosphate, Gigantol, Gilvusmycin, Glucopiericidinol A1, Glucopiericidinol A2, Gludopa, Glycothiohexide alpha, Goserelin, Granaticin B, Griseusin C, Hatomarubigin A, Hatomarubigin B, Hatomarubigin C, Hatomarubigin D, Hayumicin A, Hayumicin B, Hayumicin C1, Hayumicin C2, Hayumicin D, Heliquinomycin, Helvecardin A, Helvecardin B, Hericenal A, Hericenal B, Hericenal C, Hidrosmin, Histrelin, Histrelin acetate, Hongoquercin A, Hongoquercin B, Honokiol diepoxide, Honokiol diepoxide, Human angiotensin II, Hydromorphone methiodide, Hymenistatin 1, Hypeptin, Hypericin, Hyperoside, Icariin, Idarubicin hydrochloride, Idronoxil, Ifenprodil, Imidazoacridinone, Incyclinide, Indacaterol, Indanocine, Integracin A, Integracin B, Integracin C, Integramycin, Integrastatin A, Integrastatin B, Intoplicine, Iodochlorhydroxyquin, Iododiflunisal, Iodorubidazone (p), Iolopride (1231), Ioxipride, Iralukast, Iralukast sodium, Irciniastatin A, Irciniastatin B, Isalmadol, Isobavachalcone, Isodoxorubicin, Iso-iantheran A, Isoliquiritigenin, Isomolpan Hydrochloride, Isoquine, Isovanihuperzine A, Jadomycin B, Jasplakinolide, Kadsuphilin C, Kaitocephalin, Kampanol A, Kampanol B, Kanglemycin A, Kapurimycin A1, Kapurimycin A3, Kapurimycin A3, Kehokorin D, Kehokorin E, Kigamicin A, Kigamicin B, Kigamicin C, Kigamicin D, Kigamicin E, Kigamicinone, Kistamicin A, Klainetin A, Klainetin B, Kodaistatin A, Kodaistatin B, Kodaistatin C, Kodaistatin D, Korupensamine A, Korupensamine B, Korupensamine C, Korupensamine D, Kosinostatin, Labetalol hydrochloride, Laccaridione A, Lactonamycin, Lactosylphenyl trolox, Ladirubicin, Lamellarin alpha 20-sulfate sodium salt, Lamifiban, Lanreotide acetate, Lasofoxifene, Lasofoxifene tartrate, Latamoxef sodium, L-Chicoric acid, L-Dopamide, Lecirelin, Ledazerol, Leuprolide acetate, Leurubicin, Levalbuterol hydrochloride, Levodopa, Levodopa 3-0-glucoside, Levodopa 4-O-glucoside, Levorphanol tartrate, L-Fluviabactin, Lipiarmycin B3, Lipiarmycin B4, Liquiritin apioside, Lithospermic acid B magnesium salt, Lobatamide C, Lobatamide F, Loloatin B, Luminacin D, Luteolin, Macrocarpin A, Macrocarpin B, Makaluvamine D, Makaluvamine E, Malonoben, Maltolyl p-coumarate, Mannopeptimycin beta, Manzamine F, Marinopyrrole A, Marmelin, Masoprocol, Mastprom, Matteuorienate A, Matteuorienate B, Matteuorienate C, Medicarpin, Melevodopa hydrochloride, Mellein, Meluadrine, Meluadrine tartrate, Memno-peptide A, Meptazinol hydrochloride, Mesalazine, Metaraminol, Methanobactin, Methyl gallate, Methyldopa, Methylnaltrexone bromide, Metirosine, Micacocidin A, Micacocidin B, Micafungin sodium, Michellamine B, Mideplanin, Mimopezil, Minocycline hydrochloride, Miproxifene, Mitoxantrone hydrochloride, Mivazerol, Modecainide, Mollugin, Monohydroxyethylrutoside, Morphine Glucuronide, Morphine hydrochloride, Morphine sulfate, Moxifetin hydrogen maleate, Mumbaistatin, Mureidomycin A, Mureidomycin B, Mureidomycin C, Mureidomycin D, Mureidomycin E, Mureidomycin F, Mureidomycins, Mycophenolate Mofetil, Mycophenolic acid sodium salt, Myrciacitrin I, Myrciacitrin II, Myrciaphenone B, Myriceric acid A, Mytolbilin, Mytolbilin acid, Mytolbilin acid methyl ester, Mytolbilinol, Naamidine A, Nabilone, N-Acetylcolchinol, Nafarelin acetate, Nalbuphine hydrochloride, Nalfurafine hydrochloride, N-Allylsecoboldine, Nalmefene, Naloxone hydrochloride, Naltrexone hydrochloride, Naltrindole, Napsamycin A, Napsamycin B, Napsamycin C, Napsamycin D, Nardeterol, N-Cyclopentyl-tazopsine, Nebicapone, Nelfinavir mesilate, Nemorubicin, Neparensinol A, Neparensinol B, Neparensinol C, Nerfilin I, Nicanartine, Nitecapone, Nocardione A, Nocathiacin I, Nocathiacin III, Nocathiacin IV, NO-Mesalamine, Nordamunacantal, Nostocyclopeptide M1, Nothramicin, N-tert butyl isoquine, Obelmycin H, Ochromycinone, Octyl gallate, Odapipam acetate, O-Demethylchlorothricin, O-Demethylmurrayafoline A, Oenothein B, Okicenone, Olanzapine pamoate, Olcegepant, Olsalazine sodium, Onjixanthone I, Onjixanthone II, Oolonghomobisflavan A, Oolonghomobisflavan C, Orciprenaline sulphate, Orienticin A, Orienticin B, Orienticin C, Orienticin D, Oritavancin, Orniplabin, Orthosomycin A, Orthosomycin B, Orthosomycin C, Orthosomycin D, Orthosomycin E, Orthosomycin F, Orthosomycin G, Orthosomycin H, Osutidine, Oximidine III, Oxymetazoline hydrochloride, Oxymorphazole dihydrochloride, Oxymorphone hydrochloride, Oxyphenarsine, Ozarelix, Paeciloquinine A, Paeciloquinine D, Paeciloquinone B, Paeciloquinone D, Pancratistatin-3,4-cyclic phosphate sodium salt, Pannorin, Papuamide A, Papuamide B, Papuamide C, Papuamide D, Paracetamol, Parvisporin B, PEG-vancomycin, Penicillide, Pentazocine hydrochloride, Pepticinnamin E, Phaffiaol, Phakellistatin 7, Phakellistatin 8, Phakellistatin 9, Phenochalasin A, Phentolamine mesilate, Phlorofucofuroeckol, Phomopsichalasin, Phthalascidin, Physostigmine salicylate, Piceatannol, Pidobenzone, Pinocembrin, Pipendoxifene, Pirarubicin, Pittsburgh Compound B, Platencin, Platensimycin, Pluraflavin A, Pluraflavin B, Pluraflavin E, Pneumocandin A0, Pneumocandin B0, Pneumocandin B0 2-phosphate, Pneumocandin D0, Polyestradiol phosphate, Polyketomycin, Popolohuanone E, Pradimicin A, Pradimicin B, Pradimicin D, Pradimicin E, Pradimicin FA-1, Pradimicin FA-2, Pradimicin FL, Pradimicin FS ((+)-enantiomer), Pradimicin L, Pradimicin Q, Pradimicin S, Pradimicin T1, Pradimicin T2, Prinaberel, Probucol, Procaterol Hydrochloride Hemihydrate, Propofol, Propyl gallate, Protocatechuic acid, Protocatechuic aldehyde, Pseudohypericin, Purpuromycin, Pyrindamycin A, Pyrindamycin B, Quercetin-3-O-methyl ether, Quinagolide hydrochloride, Quinobene, rac-Apogossypolone, Rac-Tolterodine, Raloxifene hydrochloride, Ramoplanin A'1, Ramoplanin A'2, Ramoplanin A'3, Ramorelix, Ravidomycin N-oxide, Rawsonol, Reblastatin, Reproterol hydrochloride, Resobene, Resorthiomycin, Retaspimycin hydrochloride, Rhodiocyanoside B, Rhododaurichromanic acid A, Rifabutin, Rifalazil, Rifamexil, Rifampicin, Rifapentine, Rifaximin, Rimoterol hydrobromide, Riodoxol, Rohitukine, Rotigaptide, Rotigotine, Roxindole Mesilate, Ruboxyl, Rufigallol, Rumycin 1, Rumycin 2, Russuphelin A, Sabarubicin hydrochloride, Saintopin, Saintopin E, Sakyomicin A, Sakyomicin E, Salazopyridazin, Salbutamol nitrate, Salbutamol sulfate, Salcaprozic acid sodium salt, Salicylazobenzoic acid, Salicylihalamide A, Salicylihalamide B, Saliphenylhalamide, Salmaterol, Salmeterol xinafoate, Saloxin, Salvianolic acid L, Sampatrilat, Sanglifehrin A, Sanglifehrin B, Sanglifehrin C, Sanglifehrin D, Saptomycin D, Sapurimycin, Saricandin, Secoisolariciresinol diglucoside, Seglitide, Semorphone hydrochloride, Shishijimicin A, Shishijimicin B, Shishijimicin C, Sibenadet hydrochloride, Silychristin, Sinomenine, Sivifene, Siwenmycin, Sootepenseone, Spinorphin, Spinosulfate A, Spinosulfate B, Spiroximicin, Stachybocin A, Stachybocin B, Stachybocin C, Stachybotrin C, Stachybotrydial, Staplabin, Sterenin A, Sterenin C, Sterenin D, Streptopyrrole, Succinobucol, Sulfasalazine, Sulphazocine, Susalimod, Symbioimine, Syriacusin A, Syriacusin B, Syriacusin C, Tageflar, Taiwanhomoflavone A, TAP-doxorubicin, Tapentadol hydrochloride, Taramanon A, Tazofelone, Tazopsine, Tebufelone, Technetium Tc 99m depreotide, Teicoplanin-A2-1, Teicoplanin-A2-2, Teicoplanin-A2-3, Teicoplanin-A2-3, Teicoplanin-A2-5, Telavancin hydrochloride, Temoporfin, Teniposide, Tenuifoliside A, Tenuifoliside B, Tenuifoliside C, Terbutaline sulfate, Terprenin, Tetracycline hydrochloride, Tetragalloylquinic acid, Tetrahydrocurcumin, Tetrahydroechinocandin B, Tetrahydroswertianolin, Thenorphine, Theophylline rutoside, Thiazinotrienomycin B, Thiazinotrienomycin F, Thiazinotrienomycin G, Thielavin G, Thielocin B3, Thymopentin, Tigecycline, Tipelukast, Tocotrienol, Tokaramide A, Tolcapone, Tolterodine Tartrate, Topotecan Acetate, Topotecane Hydrochloride, Topsentine B1, Trabectedin, trans-Resveratrol, Traxoprodil, Traxoprodil mesylate, Trimidox, Triphendiol, Troglitazone, Tubastrine, Tubulysin A, Tubulysin B, Tubulysin C, Tucaresol, Tyropeptin A10, Tyropeptin A6, Tyropeptin A9, Tyroservatide, Tyrphostin 47, Uncarinic acid A, Uncarinic acid B, Uncialamycin, Valrubicin, Vancomycin hydrochloride, Veinamitol, Venorphin, Verticillatine, Vexibinol, Vialinin B, Vinaxanthone, W Peptide, Wiedendiol A, Wiedendiol B, Woodorien, Xamoterol Fumarate, Xanthoangelol E, Xanthofulvin, Xanthomegnin, Xipamide, Yatakemycin, Zelandopam hydrochloride, Zorubicin hydrochloride.

Suitable drugs with a carboxyl group may be be selected from the list containing (-)-Subersic acid, (+)-Deoxoartelinic acid, (+)-Hemipalmitoylcarnitinium, (+)-Indobufen, (+)-SCH-351448, (E)-p-Coumaroylquinic acid, (Z)-Indenaprost, [111In-DTPA-Pro1,Tyr4]bombesin, [90Y]-DOTAGA-substance P, [psi[CH2NH]Tpg4]Vancomycin aglycon, 111In-Pentetreotide, 11-Keto-Beta-Boswellic Acid, 15-Methoxypinusolidic acid, 1-Methyl-D-tryptophan, 3,5-Dicaffeoylquinic acid, 3-MATIDA, 3-O-Acetyloleanolic acid, 4-Aminosalicylic acid, 6alpha-Fluoroursodeoxycholic acid, 6-Carboxygenistein, 7-Chlorokynurenic acid, 8-Carboxy-iso-iantheran A, 99mTc-c(RGDfK*)2HYNIC, A-42867 pseudoaglycone, Aceclofenac, Acemetacin, Aceneuramic acid sodium salt, Acetyl-11-Keto-Beta-Boswellic Acid, Acetyl-Beta-Boswellic Acid, Acetylcysteine, Achimillic Acids, Acipimox, Acitazanolast, Acrivastine, Actarit, Adapalene, Adarotene, Ademetionine tosylate sulfate, Adxanthromycin A, Ajulemic acid, Alacepril, Aladapcin, Aleglitazar, Alitretinoin, Alminoprofen, Alogliptin benzoate, alpha-Linolenic acid, alpha-Lipoic acid, alpha-Methyltryptophan, Alprostadil, Altemicidin, Alutacenoic acid B, Alvimopan hydrate, Amiglumide, Amineptine, Aminocaproic acid, Aminolevulinic acid hydrochloride, Amlexanox, Amoxicillin trihydrate, Amphotericin B, Amsilarotene, Anakinra, Antiflammin-1, Antiflammin-2, Antiflammin-3, Apalcillin sodium, Aplaviroc hydrochloride, Argatroban monohydrate, Argimesna, Artelinate, Artepillin C, Artesunate, Arundifungin, Ascosteroside, Asiatic acid, Aspirin, Aspoxicillin, Assamicin I, Assamicin II, Ataluren, Atorvastatin, Atorvastatin calcium, Atrasentan, Azaromycin SC, Azelaic Acid, Azepinostatin, Azilsartan, Azoxybacilin, Aztreonam, Aztreonam L-lysine, Azumamide E, Baclofen, Bafilomycin C1, Baicalin, Balhimycin, Balofloxacin, Balofloxacin dihydrate, Balsalazide disodium, Bamirastine hydrate, Belactosin A, Belactosin C, Benanomicin A, Benanomicin B, Benastatin A, Benastatin B, Benazepril hydrochloride, Benthocyanin A, Bepotastine besilate, Beraprost sodium, Besifloxacin hydrochloride, Beta-Boswellic Acid, beta-Hydroxy beta-methylbutyrate, Betamipron, Beta-Sialosylcholesterol Sodium Salt, Bevirimat, Bexarotene, Bezafibrate, Biapenem, Bilastine, Bimosiamose, Bindarit, Binfloxacin, Biphenyl-indanone A, Boc-Belactosin A, Borrelidin, Brasilicardin A, Brasilinolide A, Bremelanotide, Brevifolin carboxylic acid, Bucillamine, Bumetanide, Bungeolic acid, Buprenorphine hemiadipate, Buprenorphine-Val-carbamate, Butibufen, Butoctamide hemisuccinate, Butyzamide, Cabin 1, Cadrofloxacin hydrochloride, Calbistrin A, Calbistrin B, Calbistrin C, Calbistrin D, Calcium-like peptide 1, Calcium-like peptide 2, Caloporoside B, Caloporoside C, Caloporoside D, Caloporoside E, Caloporoside F, Calpinactam, Calteridol calcium, Camprofen, Candesartan, Candoxatril, Candoxatrilat, Canfosfamide hydrochloride, Canrenoate potassium, Caprazamycin A, Caprazamycin B, Caprazamycin C, Caprazamycin E, Caprazamycin F, Captopril, Carbidopa, Carmoxirole hydrochloride, Carprofen, Cefaclor, Cefalexin monohydrate, Cefbuperazone sodium, Cefcanel, Cefdaloxime, Cefdinir, Cefetecol, Cefixime, Cefmatilen hydrochloride hydrate, Cefmenoxime hydrochloride, Cefminox sodium, Cefodizime, Cefonicid sodium, Cefoperazone sodium, Cefoselis sulfate, Cefotiam hydrochloride, Cefoxitin, Cefpimizole sodium, Cefpiramide sodium, Cefprozil, Cefprozil monohydrate, Ceftaroline fosamil acetate, Ceftazidime, Ceftibuten, Ceftobiprole, Cefuroxime, Ceranapril, Cerivastatin sodium, Ceruletide diethylamine, Cetefloxacin, Cetirizine hydrochloride, Chenodeoxycholic acid, Chinoin-169, Chlorambucil, Chloroorienticin A, Chloroorienticin B, Choline fenofibrate, Choline thioctate, Chrolactomycin, Cilastatin sodium, Cilazapril, Cilengitide, Cilomilast, Ciluprevir, Cinaciguat, Cinalukast, Cinatrin A, Cinatrin B, Cinatrin C1, Cinatrin C2, Cinatrin C3, Cinnatriacetin A, Cinnatriacetin B, Ciprofibrate, Ciprofloxacin hydrochloride, Circinamide, Cispentacin, Citrullimycine A, Clavaric acid, Clavulanate potassium, Clinofibrate, Clopidogrel Sulfate, Colletoic acid, Complestatin, Conagenin, Cosalane, Creatine phosphate, Cyclocreatine, Cycloplatam, Cyclothialidine, Cytomodulin, Cytosporic acid, Dabigatran, Daglutril, Dalargin, Dalbavancin, Danegaptide hydrochloride, Danofloxacin, Darinaparsin, Darusentan, Daurichromenic acid, Davunetide, Decahydromoenomycin A, Decaplanin, Decatromicin A, Decatromicin B, Deferasirox, Delafloxacin, Delapril Hydrochloride, Deltibant, Deoxylaidlomycin, Deoxynegamycin, Dersalazine, Desacetylvinblastinehydrazide/folate conjugate, Desferri-danoxamine, Desferrinordanoxamine, Desglugastrin tromethamine, Desmin-370, Dexibuprofen, Dexibuprofen lysine, Dexketoprofen, Dexketoprofen choline, Dexketoprofen D,L-lysine, Dexketoprofen lysine, Dexketoprofen meglumine, Dexketoprofen trometamol, Dexloxiglumide, Dexpemedolac, dextro-Ciprofibrate, Dexylosylbenanomycin A, Diacerein, Diazaphilonic acid, Di-Calciphor, Difenoxin, Diflunisal, Dihydroavenanthramide D, Dihydrogranaticin B, Dihydroisosteviol, Dihydrolipoic acid, Disalazine, Disila-bexarotene, Disodium cromproxate, Disodium lettusate, Doqualast, Doripenem, Dormitroban, Dorrigocin A, Dorrigocin B, Droxidopa, DTPA-adenosylcobalamin, Duramycin, Dynemicin A, Ecabet Sodium, Ecenofloxacin hydrochloride, Econazole Sulfosalicylate, Edetic acid, Edotreotide yttrium, Efletirizine, Eflornithine hydrochloride, Eglumetad hydrate, Elansolid C1, Elarofiban, Elastatinal B, Elastatinal C, Elsibucol, Eltrombopag olamine, Elvitegravir, Emricasan, Enalapril maleate, Enalapril nitrate, Enalaprilat, Enfumafungin, Enkastin (D), Enkastin AD, Enkastin AE, Enkastin ID, Enkastin IE, Enkastin VD, Enkastin VE, Enoloxone, Enoxacin, Enrasentan, Enrofloxacin, Epalrestat, Epidioxymanadic acid A, Epidioxymanadic acid B, Epithalon, Epofolate, Epoprostenol sodium, Epostatin, Epristeride, Eprosartan mesilate, Eprotirome, Eptaloprost, Eptastatin sodium, Eptastigmine Tartrate, Eptifibatide, Erdosteine, Eremomycin, Ertapenem sodium, Ertiprotafib, Eryloside F, Esafloxacin Hydrochloride, Esonarimod, Etacrynic acid, Etalocib sodium, Etodolac, Etretin, Evatanepag, Evernimicin, Exisulind, Ezetimibe glucuronide, Fandofloxacin hydrochloride, Faranoxi, Farglitazar, Faropenem sodium, Fasobegron hydrochloride, Febuxostat, Feglymycin, Felbinac, Felbinac Lysine Salt, Fenbufen, Fexofenadine hydrochloride, Fidexaban, Finafloxacin hydrochloride, Fleroxacin, Flobufen, Flomoxef Sodium, Flunoprost, Flunoxaprofen, Flurbiprofen, Fluvastatin sodium, Folinic acid, Fondaparinux sodium, Fosfosal, Fradafiban, Frusemide, Fudosteine, Furprofen, G1 peptide, Gabadur, Gabapentin, Gabapentin enacarbil, Gabusectin, Gadobenic acid dimeglumine salt, Gadobutrol, Gadocoletic acid trisodium salt, Gadodenterate, Gadomelitol, Gadopentetate dimeglumine, Gadoterate meglumine, Gadoteridol, Gambogic acid, Gamendazole, Gamma-Linolenic Acid, Ganefromycin Alpha, Ganefromycin Beta, Ganglioside GM1, Ganoderic acid X, Garenoxacin mesilate, Gastrazole, Gatifloxacin, Gemfibrozil, Gemifloxacin mesilate, Gemopatrilat, Gilatide, Gimatecan, Giripladib, Glaspimod, Glucarolactam potassium, Gludopa, Glutathione Monoethyl Ester, Glutathione Monoisopropyl Ester, Glycine-proline-Melphalan, Glycopin, Glycyrrhizinic acid, Golotimod, Goodyeroside B, Goralatide, Grepafloxacin hydrochloride, GS-143, Haterumadioxin A, Haterumadioxin B, Helvecardin A, Helvecardin B, Heptelidic acid chlorohydrin, Hericenal A, Hericenal B, Hericenal C, Homoindanomycin, Hongoquercin A, Hongoquercin B, Human angiotensin II, Hyaluronate sodium, Hydrostatin A, Ibuprofen, Icatibant acetate, Icofungipen, Idrapril, Ifetroban, Ilepatril, Iloprost, Imidapril, Imidapril hydrochloride, Imiglitazar, Imipenem, Indanaprost (S), Indanomycin, Indeglitazar, Indobufen, Indole-3-propionic acid, Indometacin, Indomethacin trometamol, Indoxam, Indynaprost, Inogatran, Inosiplex, Iododiflunisal, Iodofiltic acid-[123I], Iodostearic Acid, Iralukast, Iralukast sodium, Isalsteine, Isobongkrekic acid, Isotretinoin, Itavastatin calcium, Itriglumide, Kaitocephalin, Kanglemycin A, Kapurimycin A1, Kapurimycin A3, Ketoprofen, Ketoprofen lysine, Ketorolac, Ketorolac tromethamine, Khafrefungin, Kijimicin, Kistamicin A, L-4-Oxalysine, Labradimil, Lamectacin, Lamifiban, Lanthiopeptin, Lapaquistat acetate, Larazotide acetate, Laropiprant, Latamoxef sodium, L-Chicoric acid, Lenapenem hydrochloride, Lenapenem hydrochloride hydrate, Levocabastine hydrochloride, Levocetirizine dihydrochloride, levo-Ciprofibrate, Levodopa, Levodopa 3-O-glucoside, Levodopa 4-O-glucoside, Levofloxacin, Levonadifloxacin arginine salt, L-Homothiocitrulline, Licofelone, Licorice-saponin C2, Lidorestat, Limaprost alfadex, Limazocic, Linoleic acid 18:2w6-cis,9-cis, Linotroban, Lintitript, Lipohexin, Lisinopril, Lithium succinate, Lithospermic acid B magnesium salt, Loloatin B, Lomefloxacin hydrochloride, Lometrexol, Longestin, Lonidamine, Loracarbef hydrate, Lorglumide, Lotrafiban, Loxiglumide, L-Simexonyl homocysteine, L-Thiocitrulline, Lubiprostone, Lumiracoxib, Lu-Tex bis(gluconate), Lysinated-betulonic acid, Lysine acetylsalicylate, Macrocarpin B, Madecassic acid, Maracenin A1, Maracenin A2, Maracenin B1, Maracenin B2, Maracenin C1, Maracenin C2, Maracenin D1, Maracenin D2, Marbofloxacin, Maslinic acid, Matristatin A1, Matristatin A2, Matteuorienate A, Matteuorienate B, Matteuorienate C, Mebrofenin, Meclinertant, Mefenamic acid, Melagatran, Memno-peptide A, Meptazinol-Val-carbamate, Meropenem, Mersacidin, Mesalazine, Metesind glucuronate, Methanobactin, Methotrexate, Methoxatin, Methyldopa, Methylenolactocin, Methylhomoindanomycin, Metiapril, Metirosine, Micacocidin A, Micacocidin B, Midafotel, Midoriamin, Milrinone Lactate, Minerval, Mipitroban, Mispyric acid, Mixanpril, Moenomycin A chloride bismuth salt, Moexipril hydrochloride, Moexiprilat, Mofezolac, Momordin Ic, Monamidocin, Monoethanolamine oleate, Montelukast sodium, Morphine Glucuronide, Moxifloxacin hydrochloride, Mumbaistatin, Mupirocin, Muraglitazar, Muraminomicin A, Muraminomicin B, Muraminomicin C, Muraminomicin D, Muraminomicin E1, Muraminomicin E2, Muraminomicin F, Muraminomicin G, Muraminomicin H, Muraminomicin I, Muraminomicin Z1, Muraminomicin Z2, Muraminomicin Z3, Muraminomicin Z4, Mureidomycin A, Mureidomycin B, Mureidomycin C, Mureidomycin D, Mureidomycin E, Mureidomycin F, Mureidomycins, Mycaperoxide A, Mycaperoxide B, Mycestericin E, Mycophenolic acid sodium salt, Myriceric acid A, Mytolbilin acid, Nadifloxacin, Nafagrel hydrochloride, Nafagrel hydrochloride hemihydrate, Nagstatin, Napirimus, Napsagatran, Napsamycin A, Napsamycin B, Napsamycin C, Napsamycin D, Nateglinide, Naveglitazar, Nebostinel, Nemonoxacin, Neu5Ac2en, Niacin, Niglizin, Nileprost beta-cyclodextrin clathrate, Nooglutil, Norfloxacin, Norfloxacin succinil, Obeticholic acid, Octacosamicin A, Octacosamicin B, O-Demethylchlorothricin, Ofloxacin, Olamufloxacin, Olamufloxacin mesilate, Olanzapine pamoate, Oleanolic acid, Olmesartan, Olopatadine Hydrochloride, Olsalazine sodium, Omapatrilat, Onnamide A, OPC-17083, Opiorphin, Orbifloxacin, Oreganic acid, Orienticin A, Orienticin B, Orienticin C, Orienticin D, Oritavancin, Orniplabin, Oseltamivir carboxylate, Ovothiol A, Ovothiol B, Ovothiol C, Oxaprozin, Oxeglitazar, Oxiglutatione sodium, Oxymorphone-Val-carbamate, Oxynor, Ozagrel hydrochloride, Ozenoxacin, Pactimibe, Padoporfin, Paeciloquinone B, Paeciloquinone D, Paldimycin B, Palovarotene, Panipenem, Parasin I, Parinaric acid, Paulomycin, Paulomycin A2, Paulomycin B, Paulomycin C, Paulomycin D, Paulomycin E, Paulomycin F, Pazufloxacin, Pazufloxacin mesilate, Pefloxacin, PEG-vancomycin, Pelagiomicin C, Peliglitazar, Pelitrexol, Pelretin, Penasterol, Penicillamine, Peramivir, Perindopril, PG-camptothecin, Phomallenic acid C, Phomoidride A, Phomoidride B, Phosphinic cyclocreatine, Phosphosalsalate, Physostigmine salicylate, Pibaxizine, Pidotimod, Piraxostat, Piretanide, Pirfenoxone, Pirprofen, Pivagabine, Pixantrone maleate, Plakotenin, Platencin, Platensimycin, Plevitrexed, Pluraflavin E, Plusbacin A1, Plusbacin A2, Plusbacin A3, Plusbacin A4, Plusbacin B1, Plusbacin B2, Plusbacin B3, Plusbacin B4, Polyalthidin, Pomisartan, Ponalrestat, Poststatin, PPI17-24, Pradimicin A, Pradimicin B, Pradimicin D, Pradimicin E, Pradimicin FA-1, Pradimicin FA-2, Pradimicin FL, Pradimicin FS ((+)-enantiomer), Pradimicin L, Pradimicin Q, Pradimicin S, Pradimicin T1, Pradimicin T2, Pradofloxacin, Pralatrexate, Pranoprofen, Prefolic A, Pregabalin, Premafloxacin, Premafloxacin hydrochloride, Prezatide copper acetate, Proamipide, Probenecid, Probestin, Procysteine, Proglumide, Propagermanium, Propofol hemisuccinate, Prostatin, Prostratin succinate, Protocatechuic acid, Protoporphyrin IX gallium(III) complex, Prulifloxacin, Prulifloxacin Hydrochloride, Prulifloxacin Mesylate, Pseudomycin A', Pseudomycin B', Pycnanthuquinone A, Pycnanthuquinone B, Pyloricidin B, Pyridazomycin, Pyrrolosporin A, Quiflapon Sodium, Quinapril hydrochloride, Quinlukast, Rafabegron, Ragaglitazar, Raltitrexed, Ramatroban, Ramipril, Raxofelast, Razupenem, Rebamipide bismuth citrate tetramethyledamine, Rebamipide bismuth L-tartrate tetramethyledamine, Repaglinide, Resobene, Reveromycin A, Rhododaurichromanic acid A, Ridogrel, Robenacoxib, Rocagloic acid, Rolafagrel, Romazarit, Romurtide, Rosaprostol sodium, Rosuvastatin calcium, Rosuvastatin sodium, Rufloxacin Gluconate, Rufloxacin hydrochloride, Rumycin 1, Rumycin 2, Salazopyridazin, Salcaprozic acid sodium salt, Salicylazobenzoic acid, S-Allylmercaptocaptopril, Salmisteine, Salvianolic acid L, Samixogrel, Sampatrilat, Sanfetrinem, Sanfetrinem sodium, Sapurimycin, Sarpogrelate hydrochloride, Saussureamine A, Saussureamine B, Saussureamine C, Saussureamine D, Saussureamine E, Scabronine G, Scopadulcic acid B, Securioside A, Securioside B, Selank, Semduramicin, Seocalcitol, Seratrodast, Serofendic acid, Sessiloside, Shepherdin, Sialosylcholesterol-Alpha Sodium Salt, Sitafloxacin hydrate, S-Nitrosocaptopril, S-Nitrosoglutathione, Sodelglitazar, Sodium cromoglycate, Sodium oxybate, Sofalcone, Solabegron hydrochloride, Sorbicillactone A, Sparfloxacin, Sphingofungin F, Spinorphin, Spirapril, Spiriprostil, Spiroglumide, Spiroximicin, Squalestatin I, Stachybocin A, Stachybocin B, Stachybocin C, Staplabin, Starrhizin, Sterenin D, Subtilopentadecanoic acid, Succinobucol, Sufotidine bismuth citrate, Sugammadex sodium, Sulfasalazine, Sulindac, Sulopenem, Sulukast, Sunflower trypsin inhibitor-1, Susalimod, Tafamidis meglumine, Tageflar, Talaglumetad hydrochloride, Talibegron, Talibegron hydrochloride, Talopterin, Taltobulin, Tamibarotene, Tanogitran, Tanomastat, TAP-doxorubicin, Tarenflurbil, Targinine, Tazarotenic Acid, Tebipenem, Teicoplanin-A2-1, Teicoplanin-A2-2, Teicoplanin-A2-3, Teicoplanin-A2-5, Telavancin hydrochloride, Telmesteine, Telmisartan, Temafloxacin hydrochloride, Temocapril hydrochloride, Temurtide, Tenosal, Terbogrel, Terestigmine tartrate, Terikalant fumarate, Tesaglitazar, Tetomilast, Tetradecylselenoacetic acid, Tetrafibricin, Tetragalloylquinic acid, Tetrahydroechinocandin B, Tetronothiodin, Tezampanel, Thermozymocidin, Thiazohalostatin, Thielavin G, Thielocin, Thielocin B3, Thiofoscarnet, Thioxamycin, Thrazarine, Thymic humoral factor gamma-2, Thymopentin, Tiagabine hydrochloride, Tibenelast, Ticolubant, Tilarginine hydrochloride, Tiliquinatine, Timodepressin, Tipelukast, Tiplasinin, Tirofiban hydrochloride, Tisartan, Tolfenamic acid, Tolmetin, Tolrestatin, Tomopenem, Tosufloxacin, Tosufloxacin Tosilate, Trandolapril, Trandolaprilat, Tranexamic acid, Tranilast, Treprostinil diethanolamine, Treprostinil sodium, Tretinoin, Triacetylshikimic acid, Trichomycin A, Triflusal, Trimexautide, Trimoprostil, Tripterin, Tropesin, Trovafloxacin, Trovafloxacin hydrate, Trovafloxacin hydrochloride mesylate, Trovafloxacin mesilate, Tubelactomicin A, Tuberactomycin D, Tuberactomycin E, Tubulysin A, Tubulysin B, Tubulysin C, Tucaresol, Tuftsin, Turbinaric acid, Tyroservatide, Ubenimex, Ulifloxacin, Uncarinic acid A, Uncarinic acid B, Unoprostone, Ursodeoxycholic acid, Ursolic acid phosphate, Utibapril, Utibaprilat, Vadimezan, Valonomycin A, Valproate Semisodium, Valproic acid, Valsartan, Vancomycin hydrochloride, Varespladib, Vebufloxacin, Vedaprofen, Veliflapon, Verlukast, Vinaxanthone, Viquidacin, Viranamycin-A, Viscosin, Vitilevuamide, Voreloxin, W Peptide, Xanthofulvin, Zabicipril Hydrochloride, Zabiciprilat Hydrochloride, Zabofloxacin hydrochloride, Zaltoprofen, Zanamivir, Zaragozic acid D3, Zenarestat, Zofenoprilat, Zofenoprilat arginine, Zolasartan, Zonampanel.

Suitable drugs with a phosphate group may be selected fromt the group consisting of Adenophostin A, Adenophostin B, Atrinositol, Buflomedil pyridoxalphosphate, Cytostatin, Fludarabine phosphate, Fosfluconazole, Fosfonochlorin, Fosfosal, Fosopamine, Fosquidone, Fostamatinib, Ganciclovir monophosphate, Genistein-7-phosphate, Hydroxyphoslactomycin B, Leustroducsin A, Leustroducsin B, Leustroducsin C, Leustroducsin H, Mangafodipir trisodium, Menadiol sodium diphosphate, Miproxifene phosphate, Monophosphoryl lipid A, Phospholine, Phosphosalsalate, Pneumocandin B0 2-phosphate, Tafluposide, Triciribine phosphate, Ursolic acid phosphate.
Suitable drugs with a thiol group may be selected fromt the group consisting of Acetylcysteine, Antileukinate, Argimesna, Bucillamine, Butixocort, Captopril, Dihydrolipoic acid, Gemopatrilat, Glutathione monoethyl ester, Glutathione monoisopropyl ester, Midoriamin, Omapatrilat, Ovothiol A, Ovothiol B, Ovothiol C, Penicillamine, Rebimastat, Shepherdin, Zofenoprilat, Zofenoprilat arginine.

Also disclosed is a biologically active moiety D connected to n protein carrier moiety/moieties PC through n moiety/moieties of (a) reversible prodrug linker(s), meaning that for each protein carrier PC there is one reversible prodrug linker L which connects the protein carrier PC to the biologically active moiety D, with p of formula (1) being 1. Optionally, one or more of the n prodrug linker moiety/moieties L may be connected to a protein carrier PC through a spacer moiety SP. If n is larger than 1, each PC, SP, L, and m of the n sub-structres (PC)ₚ - (SP)ₘ - L are selected independently of each other and may be the same or different. Preferably, when n is larger than 1, each PC, SP, L, and m of the n sub-structures (PC)ₚ - (SP)ₘ - L are the same.

Also disclosed is a water-soluble protein carrier-linked prodrug as shown in formula (3): 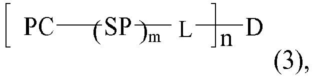 wherein
each PC independently is a protein carrier moiety comprising, preferably consisting, of an amino acid sequence
   (i) of at least 100 amino acid residues forming random coil conformation and
   (ii) comprising alanine, serine and proline residues,
each SP is independently a spacer moiety, preferably comprising q=0 branching points BP
each L is independently a reversible prodrug linker moiety,
D is a biologically active moiety,
m is 0 or 1,
n is an integer ranging of from 1 to 32, preferably from 1 to 16, more preferably from 1 to 10, even more preferably n is selected from 1, 2, 3, 4, 5, 6, 7, 8, 9, and 10,
or a pharmaceutically acceptable salt thereof.

Accordingly in a preferred embodiment in formula (1) p is 1.

More preferably, n of formula (3) is 1, so that one biologically active moiety D is connected to one protein carrier PC through one reversible prodrug linker L, and optionally the reversible prodrug linker L is connected to the protein carrier PC through a spacer moiety SP.

In another preferred embodiment, n and m of formula (3) are both 1.

In another preferred embodiment, one biologically active moiety D is connected to more than one protein carrier PC through n spacer moiety/moieties SP comprising each at least one, preferably p-1 branching points BP. Each of the more than one moieties PC may be the same or different and preferably all moieties PC are the same. Also disclosed is a water-soluble protein carrier-linked prodrug as shown in formula (4): 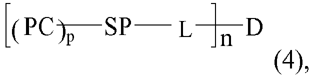 wherein
each PC is independently a protein carrier moiety comprising, preferably consisting of, an amino acid sequence
   (i) of at least 100 amino acid residues forming random coil conformation and
   (ii) comprising alanine, serine and proline residues,
each SP is independently a spacer moiety comprising q branching points BP,
each L is independently a reversible prodrug linker moiety,
D is a biologically active moiety,
each p is independently an integer of from 2 to 32, preferably from 2 to 16, more preferably from 2 to 10, even more preferably p is selected from 2, 3, 4, 5, 6, 7, 8, 9, and 10, most preferably p is 4,
n is selected from an integer ranging of from 1 to 32, preferably from 1 to 16, more preferably from 1 to 10, even more preferably n is selected from 1, 2, 3, 4, 5, 6, 7, 8, 9, and 10, preferably n is 1,
each q is independently an integer of from 1 to 32, preferably from 1 to 16, more preferably from 2 to 8, most preferably q is 3,
or a pharmaceutically acceptable salt thereof.

Accordingly in a preferred embodiment in formula (1) m is 1.

More preferably, n of formula (4) is 1, so that one biologically active moiety D is connected to one reversible prodrug linker L, and to each reversible prodrug linker L are connected 2 to 32 moieties of the protein carrier PC through a spacer moiety SP, wherein each spacer moiety SP comprises 1 to 31 branching point(s) BP.

Even more preferably, a biologically active moiety D is connected to p = 4 protein carrier moieties PC and n in formula (4) is 1.

In another preferred embodiment one protein carrier PC is connected to more than one biologically active moieties D. Also disclosed is as water-soluble protein carrier-linked as shown in formula (5): 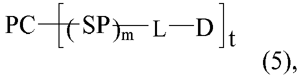 wherein
PC is a protein carrier moiety comprising, preferably consisting of, an amino acid sequence
   (i) of at least 100 amino acid residues forming random coil conformation and
   (ii) comprising alanine, serine and proline residues,
each SP is independently a spacer moiety preferably comprising q=0 branching points BP,
each L is independently a reversible prodrug linker moiety,
each D is independelty a biologically active moiety,
each m is indenpendently 0 or 1,
t is an integer ranging of from 2 to 200, preferably from 2 to 100, more preferably from 2 to 25, even more preferably t is 2, 3, 4, 5, 6, 7, 8, 9, or 10,
or a pharmaceutically acceptable salt thereof.

Accordingly in a preferred embodiment in formula (2) v is 1.

More preferably, m of formula (5) is 1.

In another preferred embodiment one protein carrier PC is connected to 2 sub-structures (SP)ₘ - (L - D)ᵥ (t in formula (2) is 2). Preferably, the 2 sub-structures (SP)ₘ - (L - D)ᵥ are connected to the protein carrier PC through the N- and C-terminus of the protein carrier PC, respectively, and each of the 2 sub-stuctures (SP)ₘ - (L - D)ᵥ may be the same or different and preferably are the same.

Also disclosed is a water-soluble protein carrier-linked prodrug as shown in formula (6): 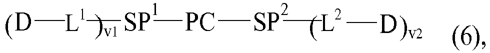 wherein
PC is a protein carrier moiety comprising, preferably consisting of, an amino acid sequence
   (i) of at least 100 amino acid residues forming random coil conformation and
   (ii) comprising alanine, serine and proline residues,
SP¹ and SP² are independently spacer moieties comprising q1 and q2 branching points BP, respectively,
L¹ and L² are independently reversible prodrug linker moieties,
D is a biologically active moiety,
v1 and v2 are independently selected from an integer ranging from 1 to 32, preferably from 1 to 16, more preferably from 1 to 10, even more preferably v1 and v2 are independently selected from 1, 2, 3, 4, 5, 6, 7, 8, 9, and 10,
q1 and q2 are independently selected from an integer ranging from 1 to 32, preferably from 1 to 16, more preferably from 2 to 8, most preferably q1 and q2 are both 3,
or a pharmaceutically acceptable salt thereof.

Accordingly in a preferred embodiment in formula (2) t is 2. Accordingly informula (6) v1 + v2 = v, Sp1, Sp2 are independently Sp and L1, L2 are independently L.
Preferably, SP¹ and SP² comprise, preferably consist of a an amino acid, peptide or polypeptide, even more preferably SP¹ and SP² comprise, preferably consist of, lysine, dilysine, trilysine, tetralysine, pentalysine, hexalysine, heptalysine, octalysine, nonalysine, decalysine, undecalysine, dodecalysine, tridecalysine, tetradecalysine, pentadecalysine, hexadecalysine, heptadecalysine, octadecalysine, nonadecalysine, di(glutamic acid), tri(glutamic acid), tetra(glutamic acid), penta(glutamic acid), hexa(glutamic acid), hepta(glutamic acid), octa(glutamic acid), nona(glutamic acid), deca(glutamic acid), undeca(glutamic acid), dodeca(glutamic acid), trideca(glutamic acid), tetradeca(glutamic acid), pentadeca(glutamic acid), hexadeca(glutamic acid), heptadeca(glutamic acid), octadeca(glutamic acid), nonadeca(glutamic acid), di(aspartic acid), tri(aspartic acid), tetra(aspartic acid), penta(aspartic acid), hexa(aspartic acid), hepta(aspartic acid), octa(aspartic acid), nona(aspartic acid), deca(aspartic acid), undeca(aspartic acid), dodeca(aspartic acid), trideca(aspartic acid), tetradeca(aspartic acid), pentadeca(aspartic acid), hexadeca(aspartic acid), heptadeca(aspartic acid), octadeca(aspartic acid), nonadeca(aspartic acid), all in bound form.

More preferably, SP¹ = SP², L¹ = L², q1=q2, and v1=v2.

Also disclosed is a water-soluble protein carrier-linked prodrug of formula (2), wherein the protein carrier PC is connected to 2 sub-structures -(SP)ₘ-(L-D)ᵥ through its N- and C-terminus, respectively, and in addition the protein carrier PC is also connected 1 or more sub-structure(s) -(SP)ₘ-(L-D)ᵥ through the amine groups of lysine residues of the protein carrier PC. Each sub-structure (SP)ₘ - (L - D)ᵥ may be the same or different and preferably are the same. Even more preferably, each spacer moiety SP independently comprises, preferably consists of, lysine, dilysine, trilysine, tetralysine, pentalysine, hexalysine, heptalysine, octalysine, nonalysine, decalysine, undecalysine, dodecalysine, tridecalysine, tetradecalysine, pentadecalysine, hexadecalysine, heptadecalysine, octadecalysine, nonadecalysine, di(glutamic acid), tri(glutamic acid), tetra(glutamic acid), penta(glutamic acid), hexa(glutamic acid), hepta(glutamic acid), octa(glutamic acid), nona(glutamic acid), deca(glutamic acid), undeca(glutamic acid), dodeca(glutamic acid), trideca(glutamic acid), tetradeca(glutamic acid), pentadeca(glutamic acid), hexadeca(glutamic acid), heptadeca(glutamic acid), octadeca(glutamic acid), nonadeca(glutamic acid), di(aspartic acid), tri(aspartic acid), tetra(aspartic acid), penta(aspartic acid), hexa(aspartic acid), hepta(aspartic acid), octa(aspartic acid), nona(aspartic acid), deca(aspartic acid), undeca(aspartic acid), dodeca(aspartic acid), trideca(aspartic acid), tetradeca(aspartic acid), pentadeca(aspartic acid), hexadeca(aspartic acid), heptadeca(aspartic acid), octadeca(aspartic acid), nonadeca(aspartic acid), all in bound form.

The present invention is a protein carrier reagent of formula (A) or (B):

PC-SP-LG (A),

(PC)ₚ-SP-LG (B),

wherein
PC, SP and p are used as described above, and
LG is a leaving group or an electrophile.

In formula (A) a protein carrier PC as described in the present invention is connected via the N- or C-terminus of PC or via one of the side chains of PC to a spacer moiety SP which does not comprise branching points BP and SP is connected to a leaving group or an electrophile.

Preferably, PC in formula (A) is connected to SP via the N-terminus of PC, i.e. via the N-terminal amine group (-NH₂).

Preferably, SP of formula (A) is selected from C₁₋₁₅ alkyl, C₂₋₁₀ alkenyl, and C₂₋₁₅ alkynyl which are optionally further interrupted with one or more of the following: -O-, -S-, -N(R)-, -C(O)-, -C(O)N(R)-, -N(R)C(O)N(R)- and -N(R)C(O)-, wherein R is selected from H or C₁₋₆ alkyl; and/or SP is optionally substituted. More preferably, SP of formula (A) is selected from C₁₋₆ alkyl, C₂₋₆ alkenyl and C₂₋₆ alkynyl.

The leaving group or electrophile LG of formula (A) is selected from formulas (a) to (f): 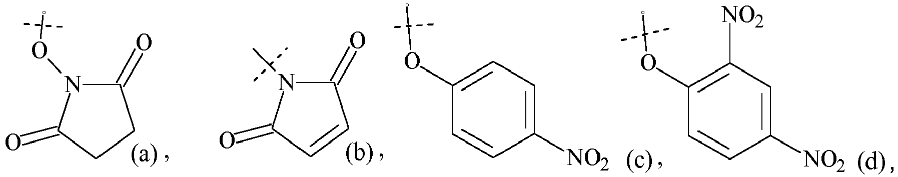 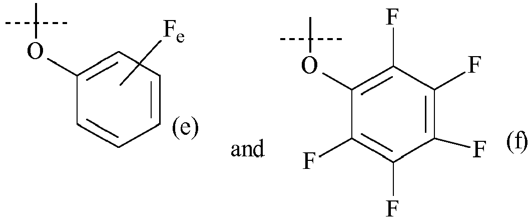 wherein
dashed lines indicate attachment to the rest of the molecule, and
e is 1, 2, 3 or 4.

More preferably, LG of formula (A) is selected from formula (a) or (b).

In formula (B) p moieties of the protein carrier PC as described in the present invention are connected to a spacer moiety SP which comprises q branching proints BP and which moiety SP is also connected to a leaving group or an electrophile LG.

In formula (B) a moiety PC is connected via its N- or C-terminus or via one of its side chains to SP. Preferably, each moiety PC of formula (B) is connected to SP via its N-terminus, i.e. via the N-terminal amine group (-NH₂).

Preferably, SP of formula (B) is selected from C₁₋₁₅ alkyl, C₂₋₁₀ alkenyl, and C₂₋₁₅ alkynyl which are optionally further interrupted with one or more of the following: -O-, -S-, -N(R)-, -C(O)-, -C(O)N(R)-, -N(R)C(O)N(R)- and -N(R)C(O)-, wherein R is selected from H or C₁₋₆ alkyl; and/or SP is optionally substituted and comprises q branching points.

Preferably, SP comprises 1, 2, 3, or 4 branching points BP, more preferably, SP comprises 1 or 2 branching points and most preferably, SP comprises 1 branching point.

The leaving group or electrophile LG of formula (B) is selected from formulas (a) to (f): 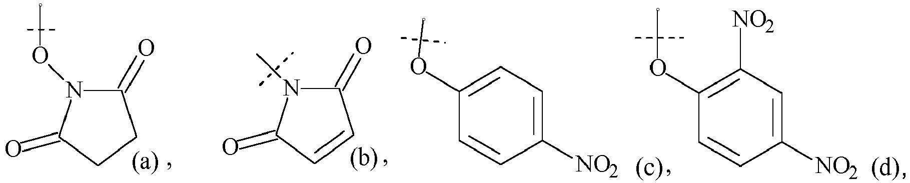 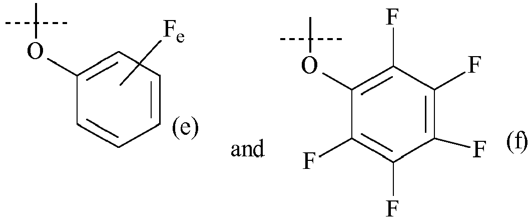 wherein
dashed lines indicate attachment to the rest of the molecule, and
e is 1, 2, 3 or 4.

More preferably, LG of formula (B) is selected from formula (a) or (b).
Most preferably, the protein carrier reagent is selected from one of the following structures: 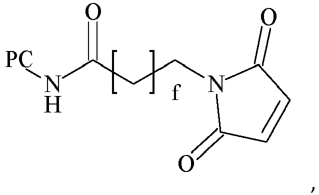 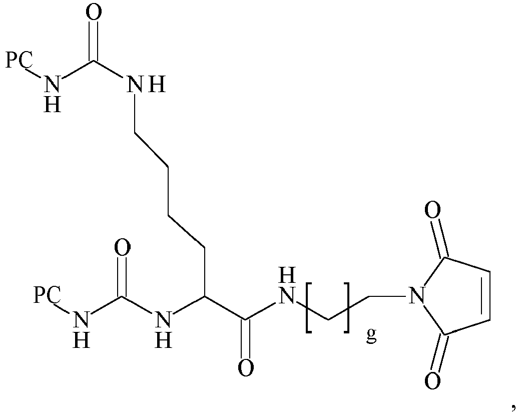 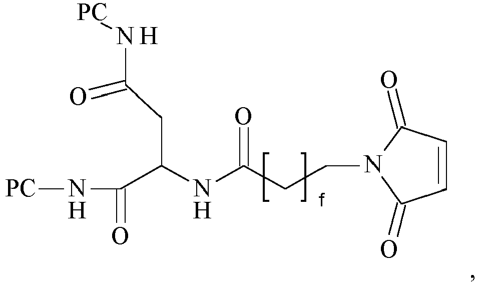 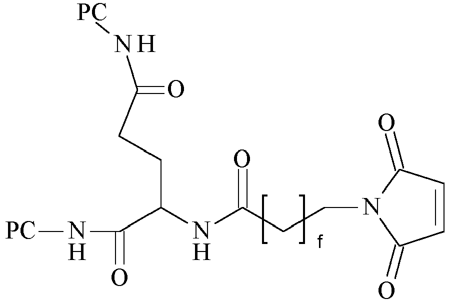 wherein
- PC: is attached to the rest of the molecule through its N-terminus which is shown as the amine (-NH-) adjacent to PC;
- g: is selected from 1, 2, 3, 4, 5, 6, 7, 8, 9, and 10, preferably, g is selected from 1, 2, 3, 4, 5, and 6, most preferably, g is 1, 2, or 3;
- f: is selected from 0, 1, 2, 3, 4, 5, 6, 7, and 8, preferably, f is selected from 0, 1, 2, and 3; 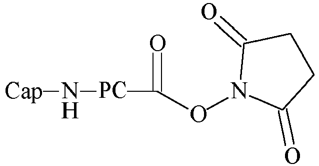 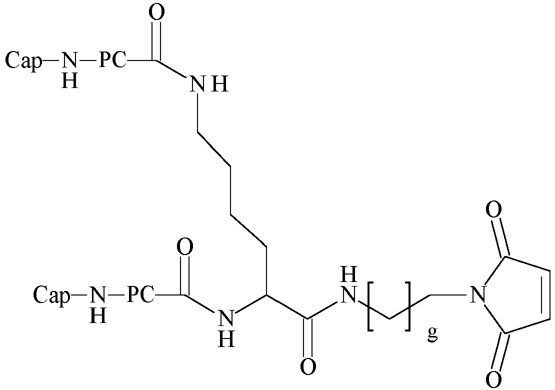
and 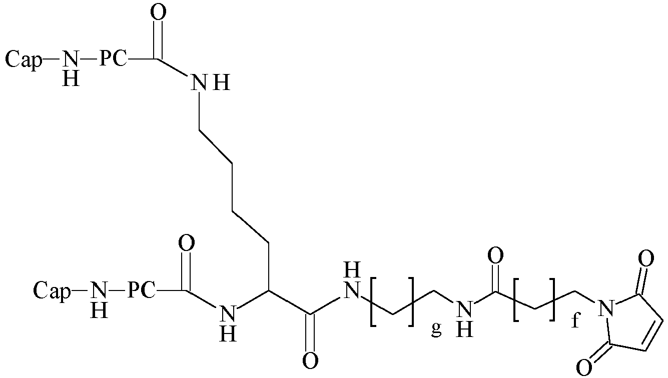 wherein
- PC: is attached to the rest of the molecule through its C-terminus which is shown as the -C(O)- adjacent to PC;
- g: is selected from 1, 2, 3, 4, 5, 6, 7, 8, 9, and 10, preferably, g is selected from 1, 2, 3, 4, 5 and 6, most preferably, g is 1, 2, or 3;
- f: is selected from 0, 1, 2, 3, 4, 5, 6, 7, and 8, preferably, f is selected from 0, 1, 2, and 3; and
- Cap: is a capping group blocking the N-terminal amino group which is shown as the -NH- adjacent to PC and is C₁₋₂₀ alkanoyl.

Preferably, Cap is selected from the group consisting of formyl, acetyl, linear or branched propanoyl, butanoyl, pentanoyl, hexanoyl, heptanoyl, octanoyl, nonanoyl, and decanoyl.

Most preferably, Cap is selected from acetyl and propanoyl.

Preferably, PC in the protein carrier reagents contains no lysine, aspartate, or glutamate residues.

Also disclosed is a pharmaceutical composition comprising the water-soluble protein carrier-linked prodrugs or a pharmaceutical salt thereof, optionally together with one or more excipients.

The pharmaceutical compositions are further described in the following paragraphs.

The pharmaceutical composition comprising the water-soluble protein carrier-linked prodrug may be provided as a liquid composition or as a dry composition. Suitable methods of drying are, for example, spray-drying and lyophilization (freeze-drying). A preferred method of drying is lyophilization.

Preferably, the water-soluble protein carrier-linked prodrug is sufficiently dosed in the composition to provide a therapeutically effective amount of the biologically active moiety for at least one day in one application in the case of therapeutically active moieties. More preferably, one application of the pharmaceutical composition comprising the water-soluble protein carrier-linked prodrug is sufficient for at least 12 hours, such as for one day, two days, such as three days, four days, five days, six days, or is sufficiently dosed for at least one week, such as for one week, two weeks, three weeks, four weeks, five weeks, six weeks, seven weeks, eight weeks, three months, four months, five months or six months.

In one embodiment, the pharmaceutical composition comprises more than one water-soluble protein carrier-linked prodrug. Said one or more water-soluble protein carrier-linked prodrugs may comprise different reversible prodrug linker moieties having different or the same half-lives, may comprise different biologically active moieties, and/or may comprise different water-soluble protein carrier moieties.

In another embodiment the pharmaceutical composition comprises also other biologically active moieties in their free form or as prodrugs other than those of the present invention.

The pharmaceutical composition of water-soluble protein carrier-linked prodrug optionally comprises one or more excipients.

Excipients may be categorized as buffering agents, isotonicity modifiers, preservatives, stabilizers, anti-adsorption agents, oxidation protection agents, viscosifiers/viscosity enhancing agents, or other auxiliary agents. In some cases, these ingredients may have dual or triple functions. The pharmaceutical compositions of water-soluble protein carrier-linked prodrugs contain one or more excipients, selected from the groups consisting of:
(i) Buffering agents: physiologically tolerated buffers to maintain pH in a desired range, such as sodium phosphate, bicarbonate, succinate, histidine, citrate and acetate, sulphate, nitrate, chloride, pyruvate. Antacids such as Mg(OH)₂ or ZnCO₃ may be also used. Buffering capacity may be adjusted to match the conditions most sensitive to pH stability
(ii) Isotonicity modifiers: to minimize pain that can result from cell damage due to osmotic pressure differences at the injection depot. Glycerin and sodium chloride are examples. Effective concentrations can be determined by osmometry using an assumed osmolality of 285-315 mOsmol/kg for serum
(iii) Preservatives and/or antimicrobials: multidose parenteral preparations require the addition of preservatives at a sufficient concentration to minimize risk of patients becoming infected upon injection and corresponding regulatory requirements have been established. Typical preservatives include m-cresol, phenol, methylparaben, ethylparaben, propylparaben, butylparaben, chlorobutanol, benzyl alcohol, phenylmercuric nitrate, thimerosol, sorbic acid, potassium sorbate, benzoic acid, chlorocresol, and benzalkonium chloride
(iv) Stabilizers: Stabilization is achieved by strengthening of the protein-stabilizing forces, by destabilization of the denatured state, or by direct binding of excipients to the protein. Stabilizers may be amino acids such as alanine, arginine, aspartic acid, glycine, histidine, lysine, proline, sugars such as glucose, sucrose, trehalose, polyols such as glycerol, mannitol, sorbitol, salts such as potassium phosphate, sodium sulphate, chelating agents such as EDTA, hexaphosphate, ligands such as divalent metal ions (zinc, calcium, etc.), other salts or organic molecules such as phenolic derivatives. In addition, oligomers or polymers such as cyclodextrins, dextran, dendrimers, PEG or PVP or protamine or HSA may be used
(v) Anti-adsorption agents: Mainly ionic or non-ionic surfactants or other proteins or soluble polymers are used to coat or adsorb competitively to the inner surface of the composition's or composition's container. Suitable surfactants are e.g., alkyl sulfates, such as ammonium lauryl sulfate and sodium lauryl sulfate; alkyl ether sulfates, such as sodium laureth sulfate and sodium myreth sulfate; sulfonates such as dioctyl sodium sulfosuccinates, perfluorooctanesulfonates, perfluorobutanesulfonates, alkyl benzene sulfonates; phosphates, such as alkyl aryl ether phosphates and alkyl ether phosphates; carboxylates, such as fatty acid salts (soaps) or sodium stearate, sodium lauroyl sarcosinate, perfluorononanoate, perfluorooctanoate; octenidine dihydrochloride; quaternary ammonium cations such as cetyl trimethylammonium bromide, cetyl trimethylammonium chloride, cetylpyridinium chloride, polyethoxylated tallow amine, benzalkonium chloride, benzethonium chloride, 5-bromo-5-nitor-1,3-dioxane, dimethyldioctadecylammonium chloride, dioctadecyldimethylammonium bromide; zwitterionics, such as 3-[(3-cholamidopropyl)dimethylammonio]-1-propanesulfonate, cocamidopropyl hydroxysultaine, amino acids, imino acids, cocamidopropyl betaine, lecithin; fatty alcohols, such as cetyl alcohol, stearyl alcohol, cetostearyl alcohol, oleyl alcohol; polyoxyethylene glycol alkyl ethers, such as octaethylene glycol monododecyl ether, pentaethylene glycol monododecyl ether; polyoxypropylene glycol alkyl ethers; glucoside alkyl ethers, such as decyl glucoside, lauryl glucoside, octyl glucoside; polyoxyethylene glycol octylphenol ethers such as Triton X-100; polyoxyethylene glycol alkylphenol ethers such as nonoxynol-9; glycerol alkyl esters such as glyceryl laurate; polyoxyethylene glycol sorbitan alkyl esters such as polysorbates; sorbitan alkyl esters; cocamide MEA and cocamide DEA; dodecyl dimethylamine oxide; block copolymers of polyethylene glycol and polypropylene glycol, such as poloxamers (Pluronic F-68), PEG dodecyl ether (Brij 35), polysorbate 20 and 80; other anti-absorption agents are dextran, polyethylene glycol, PEG-polyhistidine, BSA and HSA and gelatines. Chosen concentration and type of excipient depends on the effect to be avoided but typically a monolayer of surfactant is formed at the interface just above the CMC value
(vi) Lyo- and/or cryoprotectants: During freeze- or spray drying, excipients may counteract the destabilizing effects caused by hydrogen bond breaking and water removal. For this purpose sugars and polyols may be used but corresponding positive effects have also been observed for surfactants, amino acids, non-aqueous solvents, and other peptides. Trehalose is particulary efficient at reducing moisture-induced aggregation and also improves thermal stability potentially caused by exposure of protein hydrophobic groups to water. Mannitol and sucrose may also be used, either as sole lyo/cryoprotectant or in combination with each other where higher ratios of mannitol:sucrose are known to enhance physical stability of a lyophilized cake. Mannitol may also be combined with trehalose. Trehalose may also be combined with sorbitol or sorbitol used as the sole protectant. Starch or starch derivatives may also be used
(vii) Oxidation protection agents: antioxidants such as ascorbic acid, ectoine, methionine, glutathione, monothioglycerol, morin, polyethylenimine (PEI), propyl gallate, vitamin E, chelating agents such aus citric acid, EDTA, hexaphosphate, thioglycolic acid
(viii) Spreading or diffusing agent: modifies the permeability of connective tissue through the hydrolysis of components of the extracellular matrix in the intrastitial space such as hyaluronic acid, a polysaccharide found in the intercellular space of connective tissue. A spreading agent such as hyaluronidase temporarily decreases the viscosity of the extracellular matrix and promotes diffusion of injected drugs.
(ix) Other auxiliary agents: such as wetting agents, viscosity modifiers, antibiotics, hyaluronidase. Acids and bases such as hydrochloric acid and sodium hydroxide are auxiliary agents necessary for pH adjustment during manufacture.

In a general embodiment the pharmaceutical composition comprising the water-soluble protein carrier-linked prodrugs in either dry or liquid form may be provided as a single or multiple dose composition.

Also disclosed is a liquid or dry pharmaceutical composition comprising the water-soluble protein carrier-linked prodrug provided as a single dose, meaning that the container in which it is supplied contains one pharmaceutical dose.

Alternatively, the liquid or dry pharmaceutical composition comprising the water-soluble protein carrier-linked prodrug is a multiple dose composition, meaning that the container in which it is supplied contains more than one therapeutic dose, i.e., a multiple dose composition contains at least 2 doses. Such multiple dose composition of water-soluble protein carrier-linked prodrug can either be used for different patients in need thereof or can be used for one patient, wherein the remaining doses are stored after the application of the first dose until needed.

Also disclosed is that the pharmaceutical composition is in a container. Suitable containers for liquid or dry compositions are, for example, syringes, vials, vials with stopper and seal, ampouls, and cartridges. In particular, the liquid or dry composition comprising the water-soluble protein carrier-linked prodrug according to the present invention is provided in a syringe. If the pharmaceutical composition comprising the water-soluble protein carrier-linked prodrug is a dry pharmaceutical composition the container preferably is a dual-chamber syringe. In such embodiment, said dry pharmaceutical composition is provided in a first chamber of the dual-chamber syringe and reconstitution solution is provided in the second chamber of the dual-chamber syringe.
Prior to applying the dry composition of water-soluble protein carrier-linked prodrug to a patient in need thereof, the dry composition is reconstituted. Reconstitution can take place in the container in which the dry composition of water-soluble protein carrier-linked prodrug is provided, such as in a vial, syringe, dual-chamber syringe, ampoule, and cartridge. Reconstitution is done by adding a predefined amount of reconstitution solution to the dry composition. Reconstitution solutions are sterile liquids, such as water or buffer, which may contain further additives, such as preservatives and/or antimicrobials, such as, for example, benzylalcohol and cresol. Preferably, the reconstitution solution is sterile water. When a dry composition is reconstituted, it is referred to as a "reconstituted pharmaceutical composition" or "reconstituted composition".

Also disclosed is a water-soluble protein carrier-linked prodrug or a pharmaceutically acceptable salt thereof of or a pharmaceutical composition, for use as medicament for topical, enteral administration, parenteral administration, inhalation, injection, orinfusion, intraarticular, intradermal, subcutaneous, intramuscular, intravenous, intraosseous, and intraperitoneal, intrathecal, intracapsular, intraorbital, intracardiac, transtracheal, subcuticular, intraarticular, subcapsular, subarachnoid, intraspinal, intraventricular or intrasternal administration.

Also disclosed is a water-soluble protein carrier-linked prodrug or a pharmaceutically acceptable salt thereof or a pharmaceutical composition, wherein such water-soluble protein carrier-linked prodrug or pharmaceutically acceptable salt thereof or pharmaceutical composition is suitable to be administered to a patient via topical, enteral or parenteral administration and by methods of external application, inhalation, injection or infusion, including intraarticular, intradermal, subcutaneous, intramuscular, intravenous, intraosseous, and intraperitoneal, intrathecal, intracapsular, intraorbital, intracardiac, transtracheal, subcuticular, intraarticular, subcapsular, subarachnoid, intraspinal, intraventricular and intrasternal application.

Also disclosed is a method of preparing a reconstituted composition comprising a therapeutically effective amount of water-soluble protein carrier-linked prodrug, and optionally one or more pharmaceutically acceptable excipients, the method comprising the step of
- contacting the pharmaceutical composition comprising water-soluble protein carrier-linked prodrug with a reconstitution solution.

Also disclosed is a reconstituted pharmaceutical composition comprising a therapeutically effective amount of the water-soluble protein carrier-linked prodrug, and optionally one or more pharmaceutically acceptable excipients.

Also disclosed is the method of manufacturing a dry composition of water-soluble protein carrier-linked prodrug. In one embodiment, such dry composition is made by
(i) admixing the water-soluble protein carrier-linked prodrug optionally with one or more excipients,
(ii) transfering amounts equivalent to single or multiple doses into a suitable container,
(iii) drying the composition in said container, and
(iv) sealing the container.

Suitable containers are vials, syringes, dual-chamber syringes, ampoules, and cartridges.

Another aspect of the present invention is a kit of parts.

If the administration device is simply a hypodermic syringe then the kit may comprise the syringe, a needle and a container comprising the dry pharmaceutical composition of water-soluble protein carrier-linked prodrug for use with the syringe and a second container comprising the reconstitution solution.

If the pharmaceutical composition is a liquid composition then the kit may comprise the syringe, a needle and a container comprising the liquid composition of water-soluble protein carrier-linked prodrug for use with the syringe.

In more preferred embodiments, the injection device is other than a simple hypodermic syringe and so the separate container with reconstituted or liquid water-soluble protein carrier-linked prodrug is adapted to engage with the injection device such that in use the liquid composition in the container is in fluid connection with the outlet of the injection device. Examples of administration devices include hypodermic syringes and pen injector devices. Particularly preferred injection devices are the pen injectors in which case the container is a cartridge, preferably a disposable cartridge. Optionally, the kit of parts comprises a safety device for the needle which can be used to cap or cover the needle after use to prevent injury.

A preferred kit of parts comprises a needle and a container containing the composition according to the present invention and optionally further containing a reconstitution solution, the container being adapted for use with the needle. Preferably, the container is a dual-chamber syringe.
Also disclosed is a cartridge comprising a pharmaceutical composition of water-soluble protein carrier-linked prodrug as hereinbefore described for use with a pen injector device. The cartridge may contain a single dose or multiplicity of doses of the water-soluble protein carrier-linked prodrug.

Also disclosed is a water-soluble protein carrier-linked prodrug or pharmaceutically acceptable salt thereof, or a pharmaceutical composition for use as a medicament.

Also disclosed is a water-soluble protein carrier-linked prodrug or a pharmaceutically acceptable salt thereof, or a pharmaceutical composition for the preparation of a medicament and/or diagnostic.

It is understood, that the disease that can be treated and/or diagnosed a water-soluble protein carrier-linked prodrug or a pharmaceutically acceptable salt thereof, or a pharmaceutical composition depends on the active agent. A water-soluble protein carrier-linked prodrug with an active agent moiety which has anti-cancer activity, like Doxorubicin, is typically administered to a cancer patient. Analogously, a water-soluble protein carrier-linked prodrug with an active agent moiety which has anti-inflammatory activity, like aminosalicylic acid, is typically administered to a patient which suffers from an inflammatory disease, like rheumatoid arthritis, IBD or Morbus Crohn. Analogously, a water-soluble protein carrier-linked prodrug with an active agent moiety which has neurological activity is typically administered to a patient suffering from a neurological disease like Alzheimer's disease or Parkinson's disease. Analogously, a water-soluble protein carrier-linked prodrug with an active agent moiety which has anti-infective activity, like Gancyclovir, is typically administered to a patient suffering from a infectious disease like bacterial, viral, protozoal or fungal infection.

In case the water-soluble protein carrier-linked prodrugs contain one or more acidic or basic groups, the disclosure also comprises their corresponding pharmaceutically or toxicologically acceptable salts, in particular their pharmaceutically utilizable salts. Thus, the water-soluble protein carrier-linked prodrugs which contain acidic groups can be used, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Water-soluble protein carrier-linked prodrugs according to the invention which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, and adipic acid. If the water-soluble protein carrier-linked prodrugs according to the invention simultaneously contain acidic and basic groups in the molecule, the invention also includes, in addition to the salt forms mentioned, inner salts or betaines (zwitterions). The respective salts can be obtained by customary methods which are known to the person skilled in the art like, for example by contacting these with an organic or inorganic acid or base in a solvent or dispersant, or by anion exchange or cation exchange with other salts. The present invention also includes all salts of the prodrugs which, owing to low physiological compatibility, are not directly suitable for use in pharmaceuticals but which can be used, for example, as intermediates for chemical reactions or for the preparation of pharmaceutically acceptable salts.
Also disclosed is a method of treating, controlling, delaying or preventing in a mammalian patient, preferably in a human, in need of the treatment of one or more conditions comprising administering to said patient a diagnostically and/or therapeutically effective amount of a water-soluble protein carrier-linked prodrug or a pharmaceutically acceptable salt thereof or a pharmaceutical composition.

Yet another aspect are nucleic acid molecules comprising one or more sequence elements encoding the above mentioned polymer carrier moieties PC. The person skilled in the art clearly knows by applying standard molecular knowledge how to translate the protein sequence of the protein carrier PC into a nucleic acid sequence. It is understood, that the protein carrier moieties of the present invention may be synthesized e.g. by recombinant expression in e.g. bacteria, yeast, fungi, insect or mammalian cells using methods skilled in the art, or by chemical peptide synthesis.

Also disclosed is a polypeptide comprising the sequence as described for the polymer carrier PC.

A preferred process for the preparation of a prodrug is as follows:
In the following sections the term "random coil protein" refers to a protein with an amino acid sequence of at least 100 amino acid residues in random coil confirmation comprising alanine, serine and proline residues and the term "carrier" refers to a conjugate comprising the random coil protein and a spacer moiety.

A preferred starting material is a random coil protein which is obtained through recombinant methods known in the art. It is understood that the random coil protein may be modified by means of one or more spacer moieties. Such modifications can be introduced at the N-terminus, the C-terminus and at suitable internal amino residues having functional groups, such as the hydroxyl groups of serine residues. Preferably, such modifications are introduced either at the N-terminus, the C-terminus or both. For instance, the random coil protein may be coupled to an alkyl diamine such as ethylene diamine through its C-terminal carboxy group in order to provide for an amino functionality in proximity to the C-terminal protein region and in addition to the N-terminal amino group. It is also understood that the carboxy group may be suitably activated to facilitate condensation reactions, and that the N-terminal amino group, the C-terminal carboxy group or any other functional groups may be suitably protected by protecting groups known in the art to suppress their participation in a reaction intended to proceed at another functional group of the carrier. Such modified random coil protein is referred to as "random coil protein reagent" in the following sections. Similarly, the skilled artisan knows how to obtain linker reagents and knows how to conjugate such linker reagent to for example a drug, a spacer reagent or a random coil protein reagent.

A one-step process is provided wherein a water-soluble protein carrier-linked prodrug is prepared by reacting the random coil protein reagent with a reagent comprising the drug moiety conjugated to a reversible prodrug linker reagent or a reversible prodrug linker-spacer reagent.

A two-step process is provided wherein the water-soluble protein carrier-linked prodrug is prepared by reacting the random coil protein reagent with a reversible prodrug linker reagent or reversible prodrug linker-spacer reagent to obtain a carrier reagent, and subsequently reacting the carrier reagent with a drug moiety.

Analogously, by using suitable protecting groups the person skilled in the art knows how to combine random coil protein reagents, reversible prodrug linker reagents, spacer reagents and drug moieties to obtain prodrugs.

A few examples are given below:
Using the process methods described above, the random coil protein reagent may be attached to a biologically active moiety through one or more anchoring points. In case of one anchoring point, the carrier in the corresponding random coil protein prodrug monoconjugate may be linear or branched and contains at least two moieties of the protein carrier PC. A branched random coil protein carrier is a conjugate comprising two or more random coil protein chains, to form a molecule with one anchoring point for attachment to a drug linker reagent. This could be two 10 kDa random coil protein chains joined to form one branched 20 kDa random coil protein carrier. In the case where the molecule contains two or three branching points, the molecule is referred to 3 and 4 armed random coil protein carrier, respectively. Such branched random coil protein carrier may be obtained by reacting the random coil protein through either the N- or C-terminus with a branching moiety such as lysine. For instance, a 4 arm random coil protein carrier comprising four random coil protein chains may be obtained by reacting the random coil protein through either the N- or C-terminus with a branching moiety such as trilysine.

In case of more than one anchoring point, such as in a bisconjugate prodrug, the corresponding random coil protein in the carrier-linked prodrug may be branched or linear. Bisconjugates may contain one or two transient linkages, and the random coil protein carrier may be linear or branched or contain a mixture of one linear and one branched carrier. In case the bisconjugate contains one transient linkage and one linear and one branched carrier, the transient linkage may be on either carrier. In case a branched random coil protein carrier is used, there may be one or more branching units.

To such random coil protein, preferentially either through the C-terminal carboxy or the N-terminal amino group, or both, which may be modified for instance by spacer moieties, lysine residues are coupled sequentially to form a random coil protein hyperbranched polymer carrier (representing the protein carrier moiety and the spacer moiety/moieties wherein the spacer comprises one or more branching points). It is understood that the lysines can be partially or fully protected by protective groups during the coupling steps and that also the final hyperbranched polymer carrier may contain protective groups. A preferred building block is bis-boc lysine. For instance, the C-terminal carboxy group may be reacted in a first step with an alkyl diamine such as ethylene diamine, and subsequently, lysine moieties may be coupled to both N- and C-termini of the random coil protein to generate a bolaform block copolymer of the spacer hyperbranched oligolysine and the random coil protein.

Alternatively, instead of sequential additions of lysine residues, a hyperbranched poly-lysine moiety as spacer may be assembled first and subsequently coupled to a random coil protein through the N-terminal amino group and/or the C-terminal carboxy group whereas the carboxy group is first modified with a suitable spacer such as an alkyl diamine for instance such as ethylene diamine. Such polylysine hyperbranch may be obtained by batch condensation or by means of sequential assembly using protected lysine building blocks.

For example it may be desirable to generate a hyperbranched random coil protein block copolymer carrying 16 amino groups, consequently fifteen lysines would be attached to the N-terminus of a random coil protein.

In another embodiment, the hyperbranched spacer moiety comprising peptides may be coupled directly to the C-terminal carboxy group. In this case the dendritic moieties may be generated from glutamic or aspartic acid, and the resulting hyperbranched polymer carrier would carry a number of terminal carboxy groups.

Alternatively, instead of sequential additions of glutamic or aspartic acid residues, a hyperbranched poly-glutamate or poly-aspartate moiety may be assembled first and subsequently coupled to the random coil protein. Such polyglutamate or -aspartate may be obtained by batch condensation or by means of sequential assembly using corresponding protected amino acid building blocks.

It is also understood that all or a fraction of such hyperbranched random coil protein carrier's reactive functional groups may be present in a free form, as salts or conjugated to protecting or activating groups. Due to practical reasons, the hyperbranched random coil protein's number of branches per hyperbranch will be in a range, for example 4 to 7, more preferably 6 to 7, more preferably approximately seven.

### Materials and Methods

Side chain protected GRF(1-29)amide on Rink amide resin (synthesized by Fmoc-strategy) was obtained from Peptide Specialty Laboratories GmbH, Heidelberg, Germany. Prior to use N-terminal Fmoc protecting group was removed by treating resin 2 x 10 min with piperidine/DMF 1/4 (v/v). Recombinant human Growth Hormone (rhGH) was obtained from NIBSC, Hertfordshire, UK.
*Carrier Proteins are obtainable from MCLAB, San Francisco, U.S.A.*
All other chemicals were purchased from Sigma-ALDRICH Chemie GmbH, Taufkirchen, Germany.

### RP-HPLC purification:

RP-HPLC was done on a 100x20 or a 100x40 mm C18 ReproSil-Pur 300 ODS-3 5µ column (Dr. Maisch, Ammerbuch, Germany) connected to a Waters 600 HPLC System and Waters 2487 Absorbance detector. Linear gradients of solution A (0.1% TFA in H₂O) and solution B (0.1% TFA in acetonitrile or 0.1% TFA in 2/1 (v/v) methanol/isopropanol) were used. HPLC fractions containing product were lyophilized. Alternatively, if the HCl salt of the purified product was desired, TFA was replaced by 0.01 % HCl (v/v, 37 % HCl) in solution A and solution B.

### LC-MS Analytics:

Ultra performance liquid chromatography-electronspray ionization mass spectrometry (UPLC-ESI-MS) was performed on a Waters Acquity Ultra Performance LC instrument connected to a Thermo scientific LTQ Orbitrap Discovery instrument and spectra were, if necessary, interpreted by Thermo scientific software xcalibur. M/z signals corresponding to the most abundant isotope are given.

### Cation exchange chromatography

The purification of conjugates by cation exchange chromatography was performed using an ÄKTA Explorer system (GE Healthcare) equipped with a Macrocap SP column. The respective conjugate in 20 mM sodium acetate buffer pH 4 was applied to the column that was pre-equilibrated in 20 mM sodium acetate buffer pH 4 (buffer A). Conjugate was eluted using linear gradients of buffer B (20 mM sodium acetate, 1 M sodium chloride, pH 4.5). The eluent was monitored by detection at 215 and 280 nm.

### Size exclusion chromatography

Size exclusion chromatography analysis was performed on a ÄKTA Explorer (GE Healthcare) system equipped with a Superdex 200 or a Sepharose 6 column (10 x 300 mm). 20 mM sodium phosphate, 135 mM sodium chloride, pH 7.4 was used as mobile phase. A flow rate of 0.75 ml/min and detection wavelengths 215 nm and 280 nm were used.

### Example 1: Synthesis of bicine linker building block (not according to the invetion)

### Synthesis of (S-trityl)cysteamine la

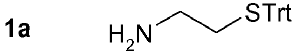

9.5 g tritylchloride was dissolved in 35 mL HFIP. 4.25 g cysteamine hydrochloride was added and mixture was stirred for 30 min at RT. Solvent was removed under reduced pressure and the residue was washed twice with 0.1 % HCl solution. Residue was purified by recristallization from toluene.
Yield: 11.8 g. MS: [M+Na]⁺ = 342.6 (MW calculated = 319.5 g/mol)

### Synthesis of serinyl-(S-trityl)cysteamine 1b

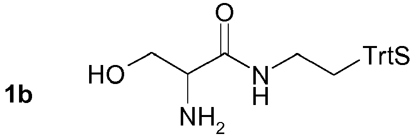

150 mg **1a,** 138 mg Fmoc-Ser-OH, and 219 mg PyBOP were dissolved in 1 mL of DMF (anhydrous, mol. sieve). 293 µl of DIEA was added and mixture was stirred for 1 h at RT. 150 µL piperidine and 50 µl DBU were added and mixture was stirred for 10 min at RT. Mixture was diluted with acetonitrile and water and acidified by addition of acetic acid. 1a was purified by RP-HPLC.
Yield: 140 mg (HCl salt). MS: [M+Na]⁺ = 429.3 (MW calculated = 406.6 g/mol)

### Example 2: GRF-linker conjugate (not according to the invetion)

### Synthesis of GRF-linker-thiol 2a

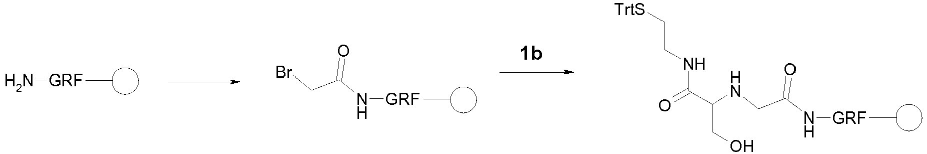

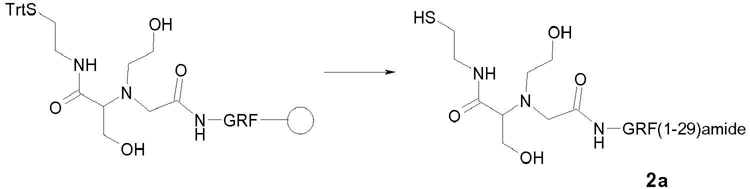

150 mg side-chain protected GRF(1-29) resin (0.1 mmol/g, 15 µmol) was suspended in a solution of 84 mg bromoacetic acid (600 µmol) and 94 µl (600 µmol) DIC in 1 ml DMF. The mixture was shaken for 30 min at RT. After washing the resin six times with DMF the resin was incubated for 2 h in a solution of 150 mg **1b** (HCl salt) and 150 µl DIEA in 1 mL DMF (anhydrous, mol. sieve). After washing resin six times with DMF the resin was incubated for 2 h in a solution of 100 mg glycolaldehyde dimer and 100 mg sodium cyanoborohydride in 1 mL DMF (anhydrous, mol. sieve) and 10 µL acetic acid for 2 h at RT. Resin was washed six times each with DMF and DCM. Cleavage of the peptide from resin and removal of protecting groups was achieved with 96/2/2 (v/v/v) TFA/triethylsilane/water for 60 min. Volatiles were removed under nitrogen flow. **2a** was purified by RP-HPLC and lyophilized.
Yield: 2 mg. MS: [M+3H]³⁺ = 1203.5 (MW calculated = 3606 g/mol)

### Synthesis of GRF-linker-maleimide 2b

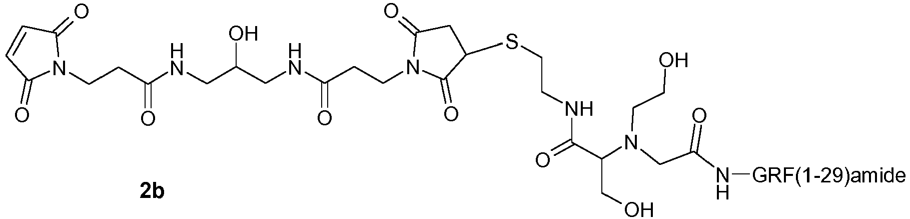

2 mg **2a** in 0.5 mL ACN/0.1 M phosphate buffer pH 6.0 1/1 (v/v) is reacted with 0.5 mg N,N'-Bis(3-maleimidopropionyl)-2-hydroxy-1,3-propanediamine at RT. Compound **2b** is purified RP-HPLC.

### Example 3: Synthesis of ethyl phenol linker building block (not according to the invetion)

### Synthesis of intermediate 3a

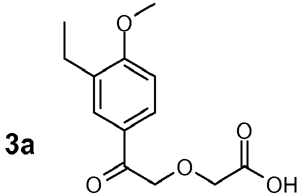

To 464 mg diglycolic acid anhydride in 2.0 mL 2-ethylanisole 1.06 g AlCl₃ was added portionwise. Reaction mixture was heated to 100 °C for 2 h, cooled to RT and quenched with 2M HCl/ice. **3a** was purified by RP-HPLC.
Yield: 350 mg. MS: [M+Na]⁺ = 275.6 (MW calculated = 252.1 g/mol)

### Synthesis of intermediate 3b

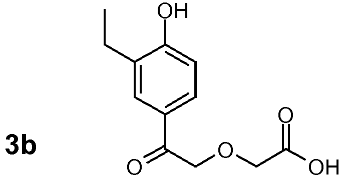

310 mg **3a** was suspended in 5 mL DCM (anhydrous, mol. sieve). 511 mg AlCl₃ was added portionwise. Reaction mixture was stirred for 5 h at 50°C in a pressure tube. Reaction cake was extracted ten times with 35 °C DCM. DCM was evaporated under reduced pressure. **3b** was used in the next step without further purification.
Yield: 250 mg. MS: [M+Na]⁺ = 261.6 (MW calculated = 238.1 g/mol)

### Synthesis of intermediate 3c

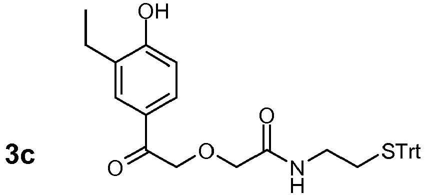

268 mg **3b,** 790 µL collidine and 268 mg EDC HCl were dissolved in 5 mL DMF (anhydrous mol. sieve). 568 mg S-Tritylcysteamine HCl was added and the mixture was reacted for 1h at RT. **3c** was purified by RP-HPLC.
Yield: 460 mg. MS: [M+Na]⁺ = 562.8 (MW calculated = 539.2 g/mol)

### Synthesis of intermediate 3d

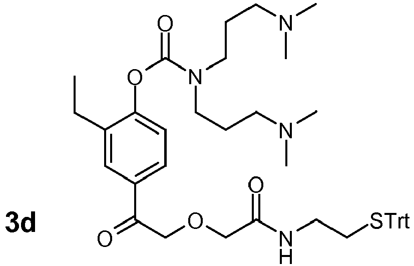

460 mg **3c** was dissolved in a mixture of 3 mL of THF (dry, mol. sieve), 3 mL of DCM (dry, mol. sieve) and 0.3 mL DIEA. 205 mg p-Nitrophenyl-chloroformate (45 mg, 0.222 mmol) and DIEA (113 µl, 0.665 mmol) were added and the mixture was stirred for 90 min at RT. 381 µl Bis-[3-(dimethylamino)-propyl]-amine was added and stirring was continued for 30 min. 300 µl of AcOH were added and solvents were removed under reduced pressure. **3d** was purified by RP-HPLC.
Yield: 400 mg (TFA salt). MS: [M+H]⁺ = 754.0 (MW calculated = 752.4 g/mol)

### Synthesis of intermediate 3e

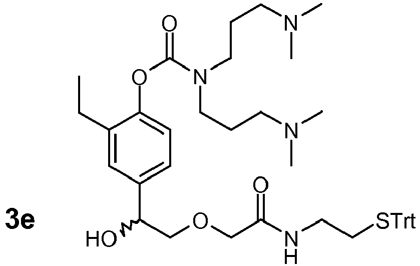

400 mg **3d** (TFA salt) was dissolved in a mixture of 9.5 ml methanol and 0.5 mL water. 78 mg NaBH₄ was added portionwise and the mixture was stirred for 30 min at RT. 0.5 ml of acetic acid were added and **3e** was purified by RP-HPLC.
Yield: 400 mg (TFA salt). MS: [M+H]⁺ = 756.0 (MW calculated = 754.4 g/mol)

### Synthesis of intermediate 3f

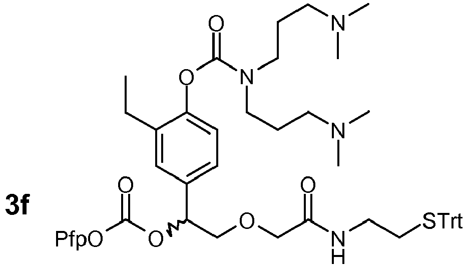

485 mg bis-(pentafluorophenyl)carbonate, 12 mg DMAP and 400 mg (TFA salt) **3e** were dissolved in 6 mL acetonitrile (dry, mol. sieve). 435 µL DIEA was added and reaction mixture was stirred for 15 min at RT, cooled to 0°C, and acidified with acetic acid (0.5 mL). Product **3f** was purified by RP-HPLC.
Yield: 430 mg (TFA salt). MS: [M+Na]⁺ = 988.1 (MW calculated = 964.4 g/mol)

### Synthesis of intermediate 3g

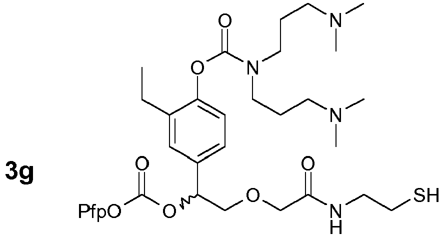

43 mg **3f** was dissolved 1 mL HFIP. 10 µL TES was added and mixture was stirred for 10 min at RT. Ice cold acetonitrile/water=9/1 (v/v) was added and **3g** was purified by RP-HPLC.
Yield: 27 mg (TFA salt). MS: [M+H]⁺ = 724.2 (MW calculated = 722.8 g/mol)

### Example 4: Random coil protein carrier activated linker reagent

### Synthesis of (3-maleimido)propionyl random coil protein carrier 4a

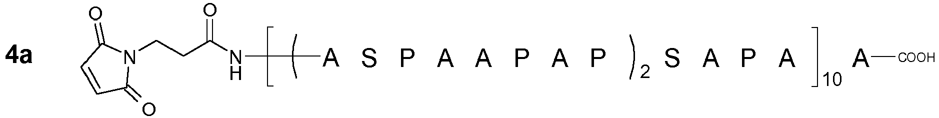

A solution of 5 mg of random coil protein [(ASPAAPAP)₂SAPA]₁₀A in 1 mL PBS containing 0.01 % Tween 20/DMSO (9/1, v/v) is mixed with a solution of 1 mg 3-(maleimido)propionic acid N-hydroxysuccinimide ester reagent in 50 µl DMSO and shaken ad RT for 1 h. Excess reagent is removed by repeated ultrafiltration using a centrifugal filter device with a MWCO of 5 kD and PBS containing 0.01 % Tween 20 as washing buffer.

### Synthesis of random coil protein carrier activated linker reagent 4b

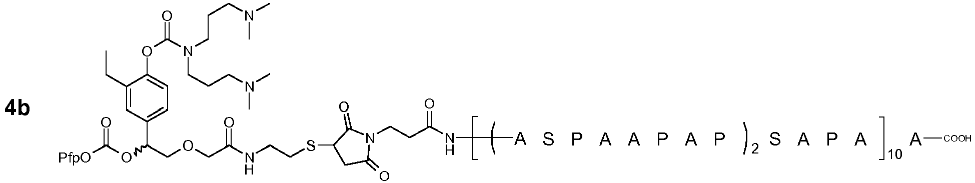

A solution of 2.5 mg of **4a** in 0.5 mL PBS containing 0.01 % Tween 20/DMSO (1/1, v/v) is mixed with a solution of 0.5 mg **3g** 50 µl acetonitrile/water (9/1, v/v). pH is adjusted to approx. pH 7.5 by titration wit 0.5 M phosphate buffer pH 7.5. Mixture is shaken for 15 min at RT. 0.2 µL of mercaptoethanol is added and mixture shaken for further 10 min. 2 mL 50 mM acetate pH 4.5 containing 0.01 % Tween 20 is added and precipitate is removed by centrifugation. Excess reagent is removed by repeated ultrafiltration of supernatant, using a centrifugal filter device with a MWCO of 5 kD and 10 mM acetate pH 4.5 containing 0.01 % Tween 20 as washing buffer.

### Example 5: Synthesis of random coil protein linker rhGH prodrug (not according to the invetion)

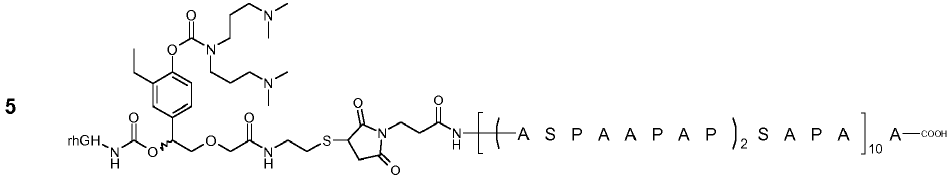

A solution of 2.5 mg **4b** in 0.5 mL 10 mM acetate pH 4.5 containing 0.01 % Tween 20 is mixed with a solution of 2.5 mg rhGH in 0.5 mL 50 mM borate pH 9. Mixture is shaken for 2 h at RT. The pH of the mixture is adjusted to pH 4.5 with acetic acid and diluted with water in order to obtain a conductivity of 0.4 mS. After incubation for 16 h at 5 °C, **5** is purified by cation exchange chromatography.

### Example 6: Synthesis of N-terminal random coil protein linker GRF prodrug (not according to the invetion)

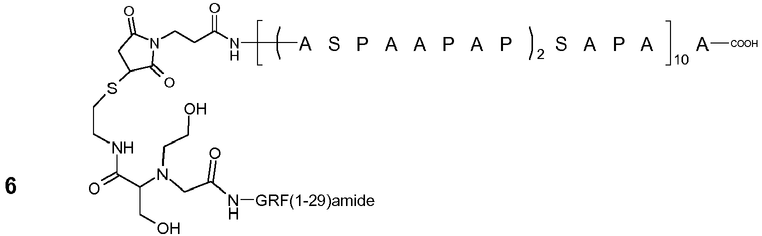

1.5 mg **2a** is dissolved in a solution of 2.5 mg of **4a** in 0.5 mL PBS. pH is adjusted to approx. pH 7.5 by titration with 0.5 M phosphate buffer pH 7.5. Mixture is shaken for 15 min at RT. 0.2 µL of mercaptoethanol is added and mixture shaken for further 10 min. The pH of the mixture is adjusted to pH 4.5 with acetic acid and diluted with water in order to obtain a conductivity of 0.4 mS. **6** is purified by cation exchange chromatography.

### Example 7: Synthesis of C-terminal random coil protein linker GRF prodrug (not according to the invetion)

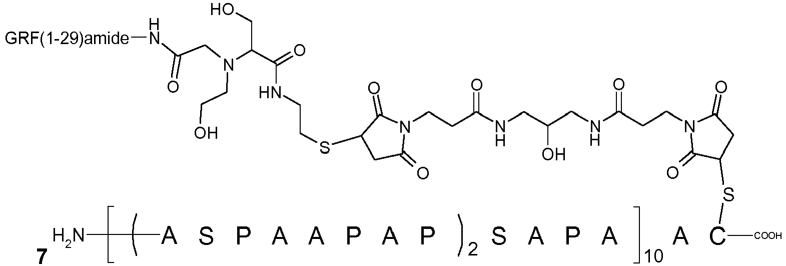

1.5 mg **2b** is dissolved in a solution of 2.5 mg of random coil protein [(ASPAAPAP)₂SAPA]₁₀AC in 0.5 mL PBS. pH is adjusted to approx. pH 7.5 by titration with 0.5 M phosphate buffer pH 7.5. Mixture is shaken for 15 min at RT. The pH of the mixture is adjusted to pH 4.5 with acetic acid and diluted with water in order to obtain a conductivity of 0.4 mS. **7** is purified by cation exchange chromatography.

### Example 8: NHS activated random coil protein carrier reagent

### Synthesis of acetylated random coil protein carrier 8a

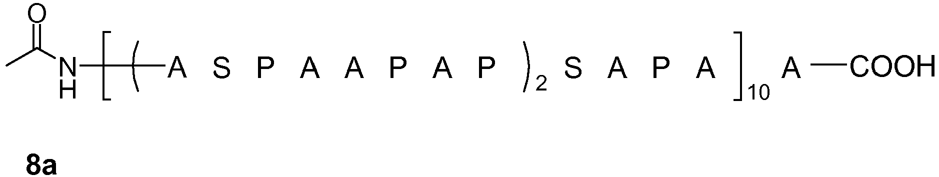

5 mg of random coil protein [(ASPAAPAP)₂SAPA]₁₀A in 1 mL 0.1 M sodium phosphate buffer pH 7.4 containing 0.01 % Tween 20 is reacted with of 5 µl acetic anydride at RT for 1 h. Excess reagent is removed by repeated ultrafiltration using a centrifugal filter device with a MWCO of 5 kD and PBS containing 0.01 % Tween 20 as washing buffer.

### Synthesis of NHS activated random coil protein carrier reagent 8b

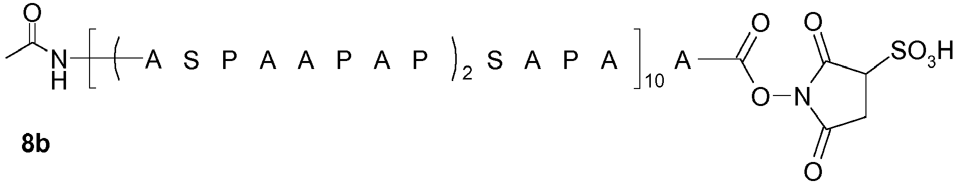

EDC (1.5 mg) and sulfo-NHS sodium salt (5 mg) are dissolved in a solution of 5 mg **8a** in 1 mL 0.1 M MES buffer pH 6.0 containing 0.01 % Tween 20. Mixture is allowed to react for 30 min at RT. Excess reagent is removed by repeated ultrafiltration at 4 °C using a centrifugal filter device with a MWCO of 5 kD and 0.1 M MES buffer pH 6.0 containing 0.01 % Tween 20 as washing buffer.

### Example 9: branched random coil protein carrier reagent (lysin core)

### Synthesis of building block 9a

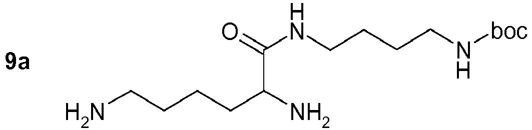

Fmoc-Lys(Fmoc)-OH (59 mg), PyBOP (52 mg) and N-Boc-1,4-butanediamine (19 mg) are dissolved in DMF (0.5 mL). DIPEA (30 µl) is added and mixture is stirred for 1 h. Piperidine (100 µl) is added and mixture is stirred for 1 h. Mixture is quenched with acetonitrile/water and acidified with acetic acid. **9a** is purified by RP-HPLC and lyophilized.

### Synthesis of branched random coil protein carrier 9b

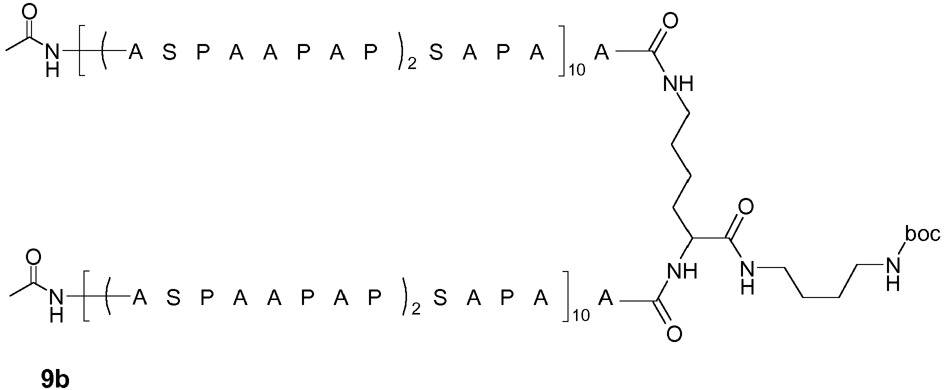

Building block **9a** (0.05 mg) is dissolved in a solution of NHS activated **8b** (5 mg) in 1 mL 0.1 M MES buffer pH 6.0 containing 0.01 % Tween 20. 0.5 M phosphate buffer pH 7.8 is titrated in order to obtain a pH of 7.4. Reaction is followed by SEC analysis and further NHS activated **8b** in MES buffer pH 6.0 and 0.5 M phosphate buffer pH 7.8 is added if necessary. Branched random coil protein carrier is purified by SEC, followed by a desalting and a lyophilization step.

### Synthesis of boc deprotected branched random coil protein carrier 9c

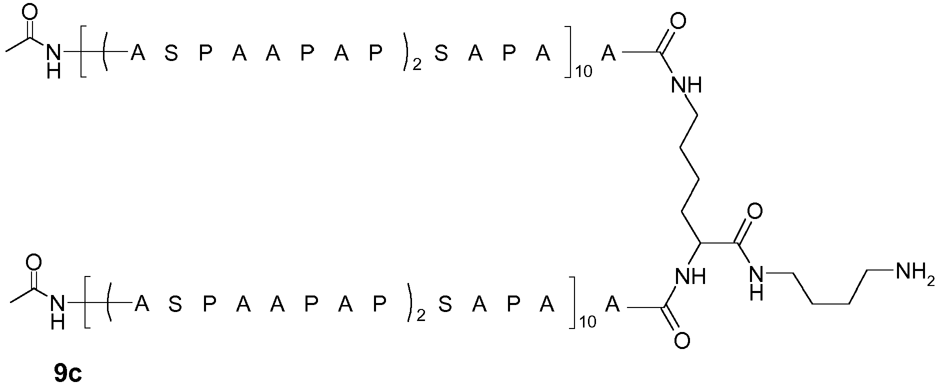

Lyophilized **9b** (5 mg) is dissolved in a mixture of HFIP (0.5 mL) and TFA (0.5 mL) and stirred at RT for 1 h. Volatiles are removed in a stream of nitrogen. The residue is taken up in PBS buffer containing 0.01 % Tween. The solution of **9c** is subjected to repeated ultrafiltration steps with PBS containing 0.01 % Tween by using a centrifugal filter device with a MWCO of 5 kD until a pH of 7.4 and a concentration of 5 mg/mL are obtained.

### Synthesis of branched random coil protein carrier reagent (lysin core) 9d

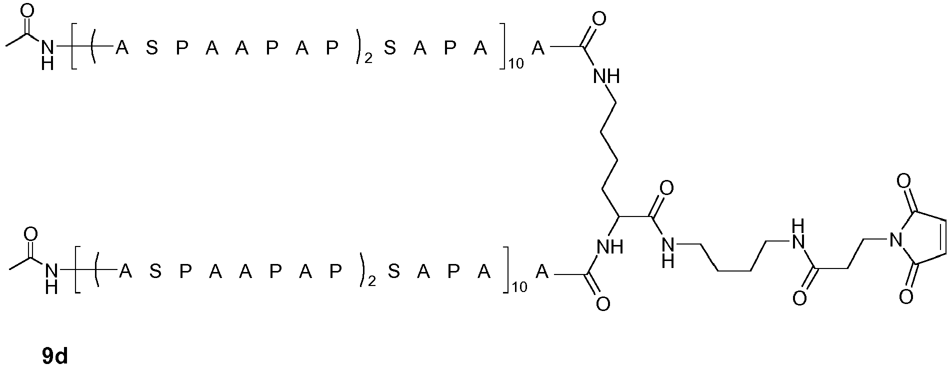

A solution of **9c** (5 mg in 1 mL PBS containing 0.01 % Tween 20) is mixed with DMSO (100 µl). A solution of 1 mg 3-(maleimido)propionic acid N-hydroxysuccinimide ester reagent in 50 µl DMSO is added and mixture is shaken at RT for 1 h. Excess reagent is removed by repeated ultrafiltration using a centrifugal filter device with a MWCO of 5 kD and 0.1 M MES pH 6.0 containing 0.01 % Tween 20 as washing buffer. Solution of **9d** is stored frozen until further use.

### Example 10: branched random coil protein carrier reagent (glutamic acid core)

### Synthesis of branched random coil protein carrier 10a

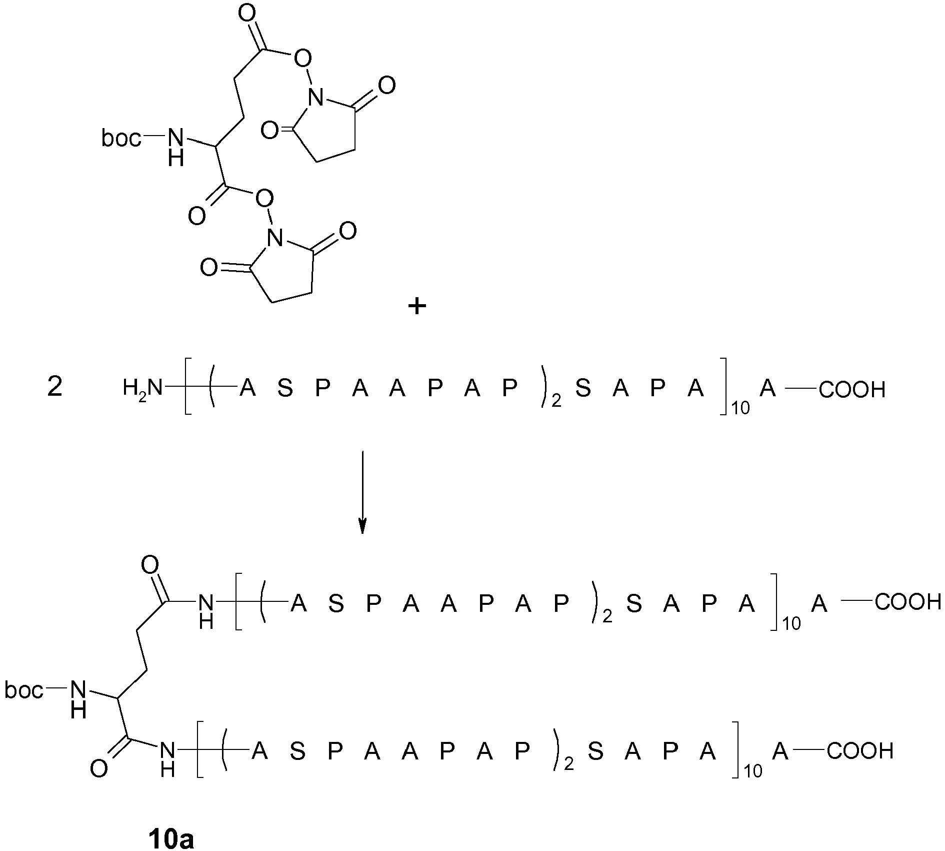

Building block Boc-Glu(OSu)-OSu (Koshi, Yoichiro et al, Journal of the American Chemical Society, 130(1), 245-251; 2008) (0.07 mg) in 50 µl DMSO is mixed with a solution of 5 mg of random coil protein [(ASPAAPAP)₂SAPA]₁₀A in 1 mL PBS containing 0.01 % Tween 20/DMSO (9/1, v/v) and shaken at RT for 3 h. Branched random coil protein carrier **10a** is purified by SEC, followed by a desalting and a lyophilization step.

### Synthesis of boc deprotected branched random coil protein carrier 10b

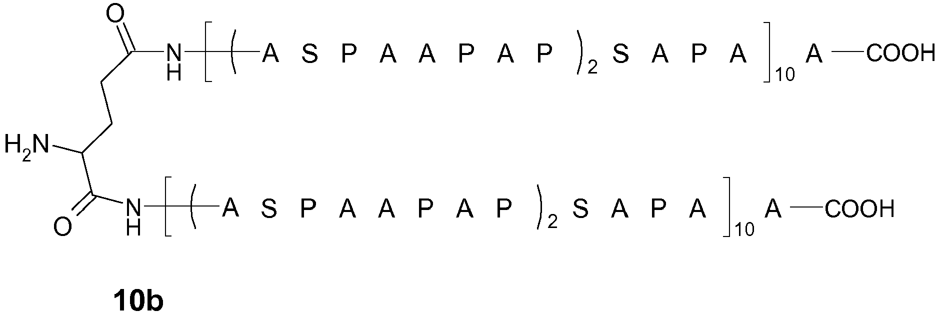

Lyophilized **10b** (5 mg) is dissolved in a mixture of HFIP (0.5 mL) and TFA (0.5 mL) and stirred at RT for 1 h. Volatiles are removed in a stream of nitrogen. The residue is taken up in PBS buffer containing 0.01 % Tween. The solution of **10b** is subjected to repeated ultrafiltration steps with PBS containing 0.01 % Tween by using a centrifugal filter device with a MWCO of 5 kD until a pH of 7.4 and a concentration of 5 mg/mL are obtained.

### Synthesis of branched random coil protein carrier reagent (glutamic acid core) 10c

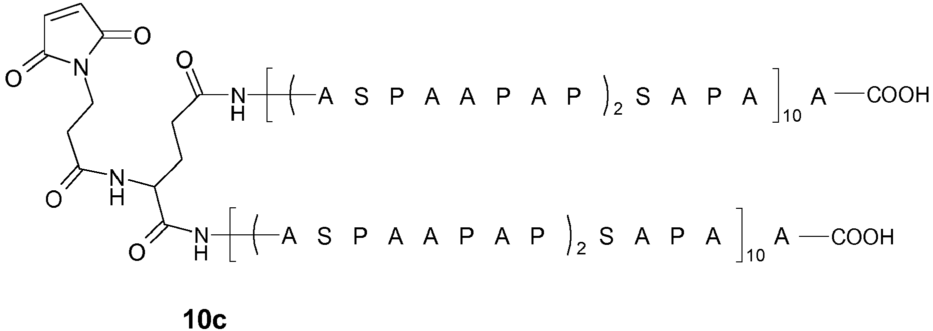

A solution of **10b** (5 mg in 1 mL PBS containing 0.01 % Tween 20) is mixed with DMSO (100 µl). A solution of 3-(maleimido)propionic acid N-hydroxysuccinimide ester reagent (1 mg) in 50 µl DMSO is added and mixture is shaken at RT for 1 h. Excess reagent is removed by repeated ultrafiltration using a centrifugal filter device with a MWCO of 5 kD and 0.1 M MES pH 6.0 containing 0.01 % Tween 20 as washing buffer. Solution of **10c** is stored frozen until further use.

### Example 11: Synthesis of a "branched" random coil protein linker GRF prodrug (not according to the invention)

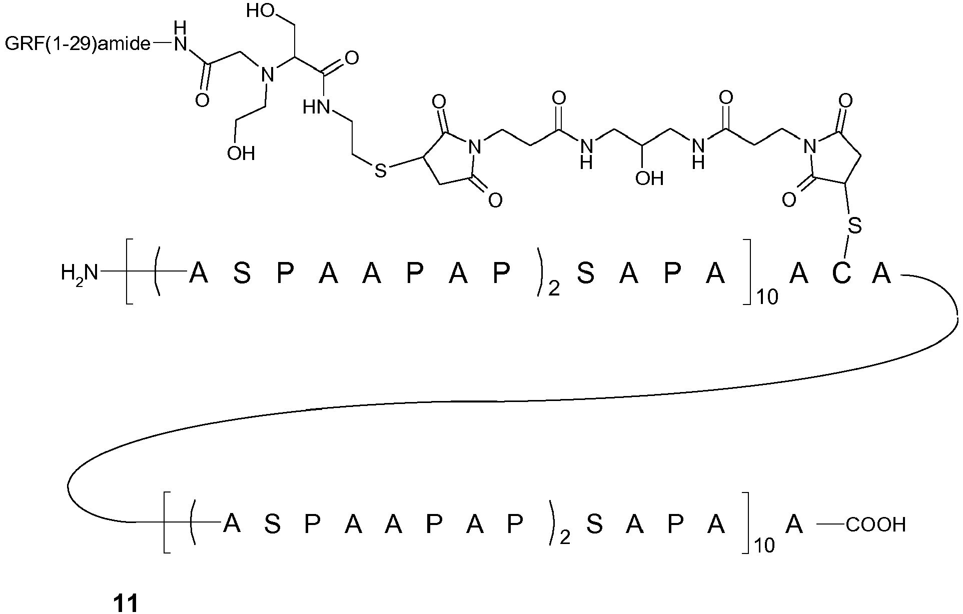

**2b** (1.5 mg) is dissolved in a solution of 5 mg of random coil protein [(ASPAAPAP)₂SAPA]₁₀ACA[(ASPAAPAP)₂SAPA]₁₀A in 1 mL PBS. pH is adjusted to approx. pH 7.4 by titration with 0.5 M phosphate buffer pH 7.5. Mixture is shaken for 15 min at RT. The pH of the mixture is adjusted to pH 4.5 with acetic acid and diluted with water in order to obtain a conductivity of 0.4 mS. **11** is purified by cation exchange chromatography.

### Example 12: Synthesis of N-terminal random coil protein linker BNP prodrug (not according to the invention)

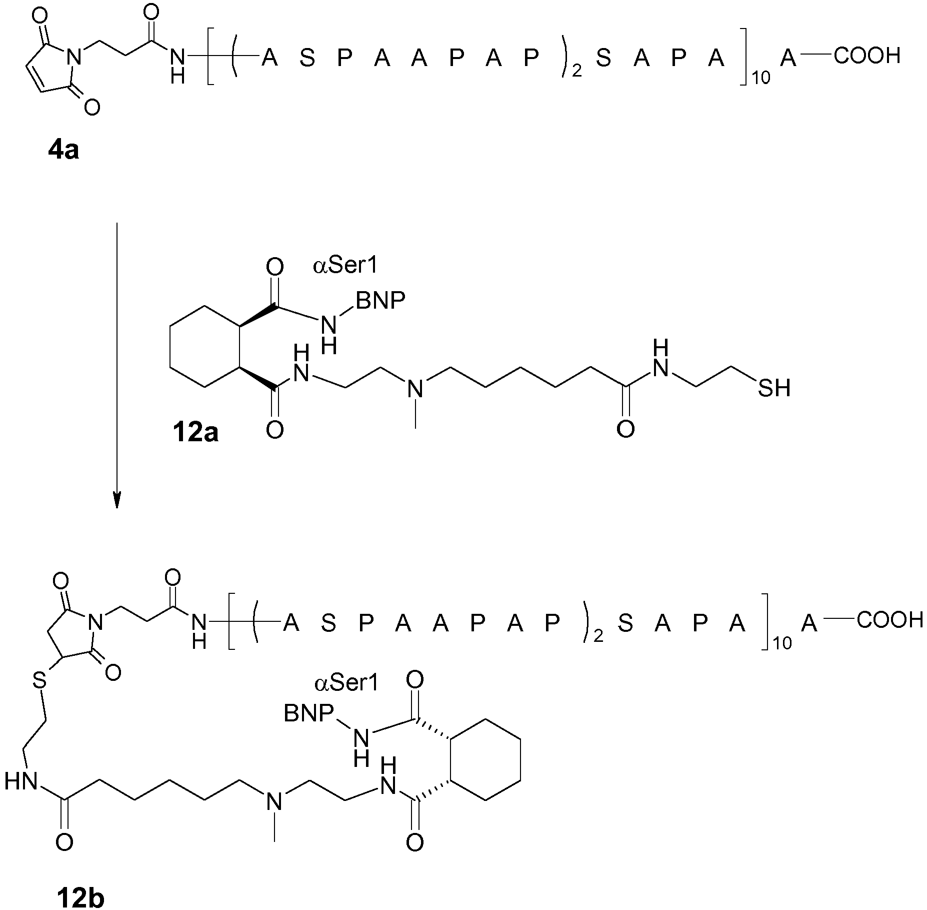

1.5 mg **4a** (for synthesis see WO-A 2009/095479) is dissolved in a solution of 2.5 mg of **4a** in 0.5 mL PBS. pH is adjusted to approx. pH 7.4 by titration with 0.5 M phosphate buffer pH 7.5. Mixture is shaken for 15 min at RT. 0.2 µL of mercaptoethanol is added and mixture is shaken for further 10 min. The pH of the mixture is adjusted to pH 4.5 with acetic acid and diluted with water in order to obtain a conductivity of 0.4 mS. **12b** is purified by cation exchange chromatography.

### Abbreviations:

- ACN: acetonitrile
- Boc: t-butyloxycarbonyl
- DCC: N,N'-dicyclohexylcarbodiimide
- DCM: dichloromethane
- DIEA: diisopropylethylamine
- DMAP: dimethylamino-pyridine
- DMSO: dimethylsulfoxide
- EDC: N-(3-Dimethylaminopropyl)-N'-ethylcarbodiimide
- eq: stoichiometric equivalent
- GRF: growth hormone releasing factor
- HFIP: 1,1,1,3,3,3-Hexafluoro-2-propanol
- HOBt: 1-hydroxybentotriazole
- LCMS: mass spectrometry-coupled liquid chromatography
- MS: mass spectrum
- MTBE: methyl *tert*.-butyl ether
- MW: molecular mass
- NHS: N-hydroxy succinimide
- NMP: N-methyl-2-pyrrolidone
- PEG: poly(ethylene glycol)
- rhGH: recombinant human Growth Hormone
- RP-HPLC: reversed-phase high performance liquid chromatography
- RT: room temperature
- Sulfo-NHS: N-hydroxy sulfosuccinimide
- TFA: trifluoroacetic acid

## Claims

1. A protein carrier reagent of formula (A) or (B):
PC-SP-LG (A),
(PC)ₚ-SP-LG (B),
wherein
each PC individually is a protein carrier moiety,
wherein a protein carrier moiety comprises, preferably consists of an amino acid sequence consisting of at least 100 amino acid residues, and
wherein the amino acid sequence of at least 100 amino acid residues, preferably the protein carrier moiety, is in random coil conformation as determined by circular dichroism measurements, and,
wherein the amino acid sequence of at least 100 amino acid residues, preferably the protein carrier moiety, comprises alanine, serine and proline residues,
SP is a spacer moiety comprising q branching points BP,
p is an integer of from 1 to 32, preferably of from 1 to 16, more preferably of from 1 to 10, even more preferably p is selected from 1, 2, 3, 4, 5, 6, 7, 8, 9, and 10,
q is an integer of from 0 to 32, preferably of from 0 to 16, more preferably of from 2 to 8, most preferably q is 3,
LG is a leaving group or an electrophile selected from formulas (a) to (f): 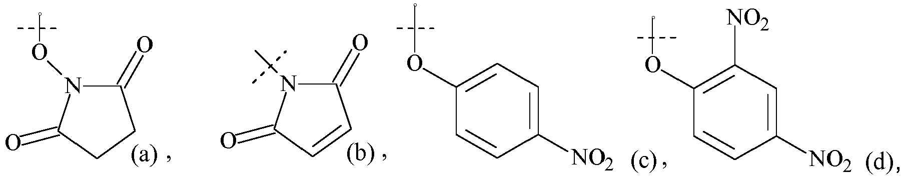 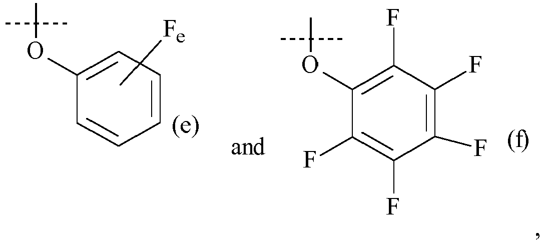 wherein
dashed lines indicate attachment to the rest of the molecule, and
e is 1, 2, 3 or 4.

2. The protein carrier reagent of claim 1, wherein a moiety PC comprises a plurality of amino acid repeats,
wherein said repeats consist of Ala, Ser, and Pro residues,
and wherein no more than 6 consecutive amino acid residues of the carrier moiety PC are identical.

3. The protein carrier reagent of claim 1 or 2, wherein said proline residues constitute more than 4 % and less than 40 % of the amino acids of the protein carrier moiety PC.

4. The protein carrier reagent of any one of claims 1 to 3, wherein the protein carrier moiety PC comprises at least one amino acid sequence selected from the group consisting of:
ASPAAPAPASPAAPAPSAPA (SEQ ID NO:9),
AAPASPAPAAPSAPAPAAPS (SEQ ID NO:10),
APSSPSPSAPSSPSPASPSS (SEQ ID NO:1 1),
SSPSAPSPSSPASPSPSSPA (SEQ ID NO:12),
AASPAAPSAPPAAASPAAPSAPPA (SEQ ID NO:13), and
ASAAAPAAASAAASAPSAAA (SEQ ID NO:14);
and circular permuted versions or multimers of these sequences.

5. The protein carrier reagent of any one of claims 1 to 4, wherein a moiety PC comprises, in particular consists of, an amino acid sequence of 100 to 3000 amino acid residues forming random coil conformation.

6. The protein carrier reagent of any one of claims 1 to 5, wherein PC has maximally 1500 amino acids.

7. The protein carrier reagent of any one of claims 1 to 6, wherein PC has maximally 900 amino acids.

8. The protein carrier reagent of any one of claims 1 to 7, wherein PC comprises further amino acids different from alanine, serine, and proline as minor constituents, which minor constituents are maximally 10% of the amino acids of PC.

9. The protein carrier reagent of any one of claims 1 to 8, wherein PC comprises no more than 4 identical consecutive amino acid residues.

10. The protein carrier reagent of any one of claims 1 to 9, wherein PC comprises more than 10% but less than 50% alanine residues.

11. The protein carrier reagent of any one of claims 1 to 10, wherein PC comprises more than 10% but less than 50% serine residues.

12. The protein carrier reagent of any one of claims 1 to 11, wherein PC comprises 35% proline residues, 50% alanine residues and 15% serine residues.

13. The protein carrier reagent of any one of claims 1 to 12, wherein LG is selected from formula (a) or (b).

14. The protein carrier reagent of any one of claims 1 to 13, wherein PC in formula (B) is connected via its N- or C-terminus or via one of its side chains to SP.

15. The protein carrier reagent of any one of claims 1 to 14, wherein SP is selected from the group consisting of C₁₋₅₀ alkyl, C₂₋₅₀ alkenyl and C₂₋₅₀ alkynyl, which moiety is optionally interrupted by one or more groups selected from the group consisting of -NH-, -N(C₁₋₄ alkyl)-, -O-, -S-, -C(O)-, -C(O)NH-, -C(O)N(C₁₋₄ alkyl)-, -O-C(O)-, -S(O)-, -S(O)₂-, 4- to 7-membered heterocyclyl, phenyl, naphthyl, at least 3-fold substituted carbocycles, at least 3-fold substituted heterocycles, a tertiary carbon atom, a quaternary carbon atom and a tertiary nitrogen atom, and which moiety is optionally substituted.

16. The protein carrier reagent of any one of claims 1 to 15, wherein the protein carrier reagent is selected from one of the following structures: 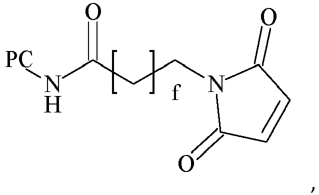 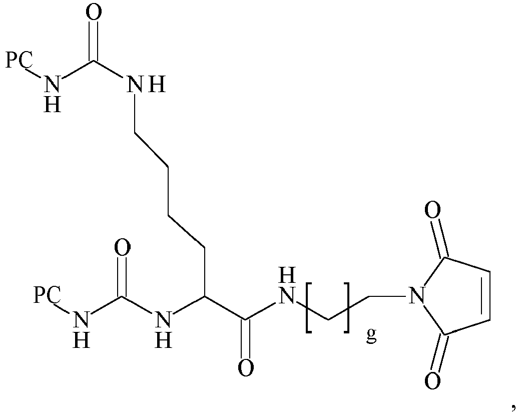 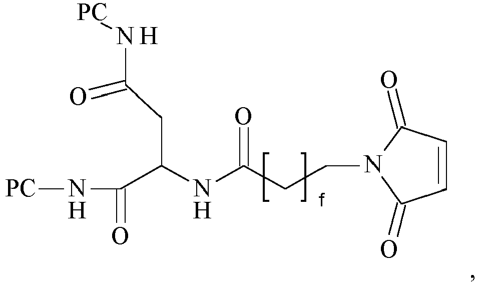 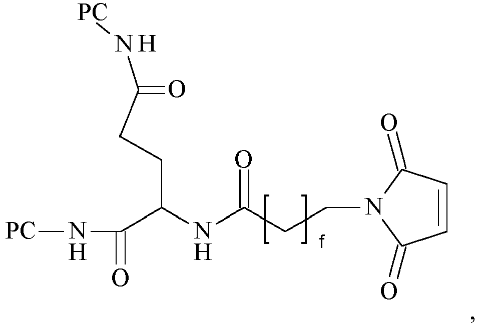 wherein
PC is attached to the rest of the molecule through its N-terminus which is shown as the amine (-NH-) adjacent to PC;
g is selected from 1, 2, 3, 4, 5, 6, 7, 8, 9, and 10, preferably, g is selected from 1, 2, 3, 4, 5, and 6, most preferably, g is 1, 2, or 3;
f is selected from 0, 1, 2, 3, 4, 5, 6, 7, and 8, preferably, f is selected from 0, 1, 2, and 3; 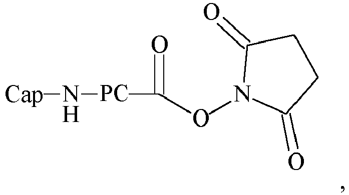 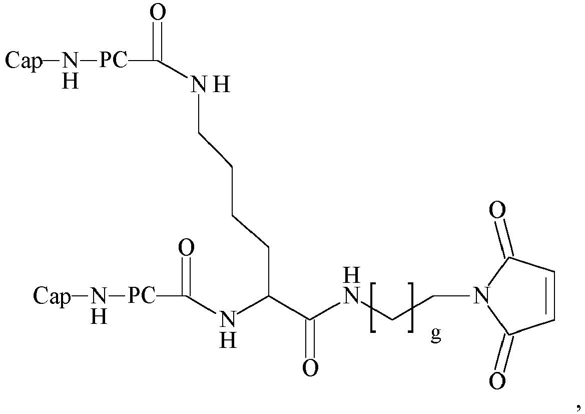
and 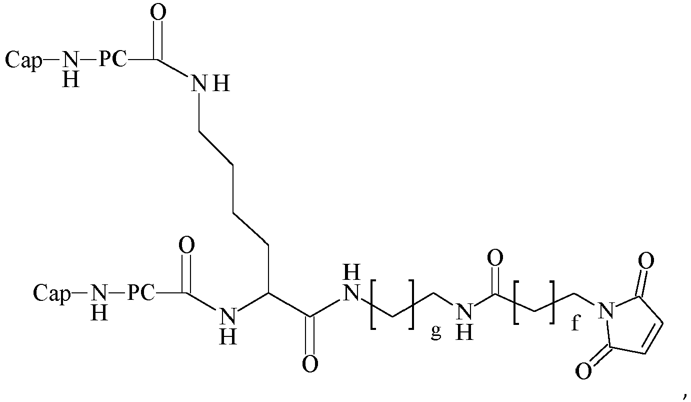 wherein
PC is attached to the rest of the molecule through its C-terminus which is shown as the -C(O)- adjacent to PC;
g is selected from 1, 2, 3, 4, 5, 6, 7, 8, 9, and 10, preferably, g is selected from 1, 2, 3, 4, 5, and 6, most preferably, g is 1, 2, or 3;
f is selected from 0, 1, 2, 3, 4, 5, 6, 7, and 8, preferably, f is selected from 0, 1, 2, and 3; and
Cap is a capping group blocking the N-terminal amino group which is shown as the -NH- adjacent to PC and is C₁₋₂₀ alkanoyl.

17. The protein carrier reagent of claim 16, wherein Cap is selected from the group consisting of formyl, acetyl, linear or branched propanoyl, butanoyl, pentanoyl, hexanoyl, heptanoyl, octanoyl, nonanoyl, and decanoyl.

## Patentansprüche

1. Ein Proteinträger-Reagenz der Formel (A) oder (B):
PC-SP-LG (A),
(PC)ₚ-SP-LG (B),
wobei
jedes PC individuell ein Proteinträger-Rest ist,
wobei ein Proteinträger-Rest eine Aminosäuresequenz bestehend aus mindestens 100 Aminosäureresten umfasst, bevorzugt daraus besteht, und
wobei die Aminosäuresequenz aus mindestens 100 Aminosäureresten, bevorzugt der Proteinträger-Rest, in einer "Random Coil"-Konformation vorliegt, wie durch Messung mit Circulardichroismus-Spektroskopie bestimmt, und
wobei die Aminosäuresequenz mindestens 100 Aminosäureresten, bevorzugt der Proteinträger-Rest, Alanin-, Serin- und Prolin-Reste umfasst,
SP ein Abstandshalter-Rest ist, welcher q Verzweigungspunkte BP umfasst,
p eine ganze Zahl von 1 bis 32 ist, bevorzugt von 1 bis 16, bevorzugter von 1 bis 10, p noch weiter bevorzugt ausgewählt ist aus 1, 2, 3, 4, 5, 6, 7, 8, 9 und 10,
q eine ganze Zahl von 0 bis 32 ist, bevorzugt von 0 bis 16, bevorzugter von 2 bis 8, q am meisten bevorzugt 3 ist,
LG eine Abgangsgruppe oder ein Elektrophil ist, ausgewählt aus den Formeln (a) bis (f): 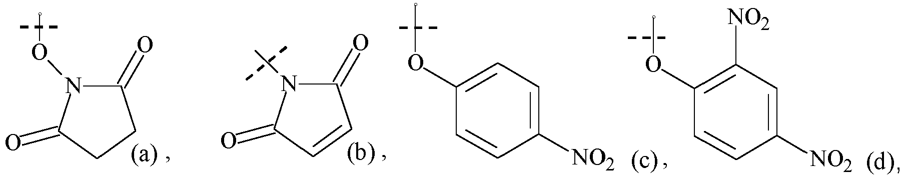 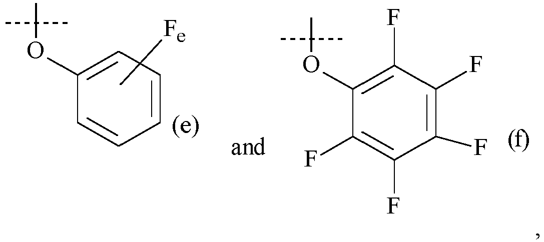 wobei
gestrichelte Linien die Anbindung an den Rest des Moleküls anzeigen, und
e 1, 2, 3 oder 4 ist.

2. Das Proteinträger-Reagenz nach Anspruch 1, wobei ein Rest PC eine Vielzahl von Aminosäure-Wiederholungen umfasst,
wobei besagte Wiederholungen aus Ala-, Ser- und Pro-Resten bestehen,
und wobei nicht mehr als 6 aufeinanderfolgende Aminosäurereste des Trägerrestes PC identisch sind.

3. Das Proteinträger-Reagenz nach Anspruch 1 oder 2, wobei besagte Prolin-Reste mehr als 4% und weniger als 40% der Aminosäuren des Proteinträger-Restes PC ausmachen.

4. Das Proteinträger-Reagenz nach einem der Ansprüche 1 bis 3, wobei der Proteinträger-Rest PC mindestens eine Aminosäuresequenz umfasst ausgewählt aus der Gruppe bestehend aus:
ASPAAPAPASPAAPAPSAPA (SEQ ID NO:9),
AAPASPAPAAPSAPAPAAPS (SEQ ID NO:10),
APSSPSPSAPSSPSPASPSS (SEQ ID NO:11),
SSPSAPSPSSPASPSPSSPA (SEQ ID NO:12),
AASPAAPSAPPAAASPAAPSAPPA (SEQ ID NO:13), und
ASAAAPAAASAAASAPSAAA (SEQ ID NO:14);
und zyklisch permutierte Versionen oder Multimere dieser Sequenzen.

5. Das Proteinträger-Reagenz nach einem der Ansprüche 1 bis 4, wobei ein Rest PC eine Aminosäuresequenz umfasst, die insbesondere aus 100 bis 3000 Aminosäureresten besteht, welche eine Random Coil-Konformation bilden.

6. Das Proteinträger-Reagenz nach einem der Ansprüche 1 bis 5, wobei PC maximal 1500 Aminosäuren hat.

7. Das Proteinträger-Reagenz nach einem der Ansprüche 1 bis 6, wobei PC maximal 900 Aminosäuren hat.

8. Das Proteinträger-Reagenz nach einem der Ansprüche 1 bis 7, wobei PC weitere Aminosäuren außer Alanin, Serin und Prolin als Nebenbestandteile umfasst, wobei diese Nebenbestandteile maximal 10% der Aminosäuren von PC ausmachen.

9. Das Proteinträger-Reagenz nach einem der Ansprüche 1 bis 8, wobei PC nicht mehr als 4 identische aufeinanderfolgende Aminosäurereste umfasst.

10. Das Proteinträger-Reagenz nach einem der Ansprüche 1 bis 9, wobei PC mehr als 10%, aber weniger als 50% Alanin-Reste umfasst.

11. Das Proteinträger-Reagenz nach einem der Ansprüche 1 bis 10, wobei PC mehr als 10%, aber weniger als 50% Serin-Reste umfasst.

12. Das Proteinträger-Reagenz nach einem der Ansprüche 1 bis 11, wobei PC 35% Prolin-Reste, 50% Alanin-Reste und 15% Serin-Reste umfasst.

13. Das Proteinträger-Reagenz nach einem der Ansprüche 1 bis 12, wobei LG ausgewählt ist aus Formel (a) oder (b).

14. Das Proteinträger-Reagenz nach einem der Ansprüche 1 bis 13, wobei PC in Formel (B) über seinen N- oder C-Terminus oder über eine seiner Seitenketten mit SP verbunden ist.

15. Das Proteinträger-Reagenz nach einem der Ansprüche 1 bis 14, wobei SP ausgewählt ist aus der Gruppe bestehend aus C₁₋₅₀-Alkyl, C₂₋₅₀-Alkenyl und C₂₋₅₀-Alkinyl, dessen Rest optional unterbrochen ist durch eine oder mehrere Gruppen, ausgewählt aus der Gruppe bestehend aus -NH-, -N(C₁₋₄-Alkyl)-, -O-, -S-, -C(O)-, -C(O)NH-, -C(O)N(C₁₋₄-Alkyl)-, -O-C(O)-, -S(O)-, -S(O)₂-, 4- bis 7-gliedrigem Heterocyclyl, Phenyl, Naphthyl, mindestens 3-fach substituierten Carbocyclen, mindestens 3-fach substituierten Heterocyclen, einem tertiären Kohlenstoffatom, einem quaternären Kohlenstoffatom und einem tertiärem Stickstoffatom, und dessen Rest optional substituiert ist.

16. Das Proteinträger-Reagenz nach einem der Ansprüche 1 bis 15, wobei das Proteinträger-Reagenz aus einer der folgenden Strukturen ausgewählt ist: 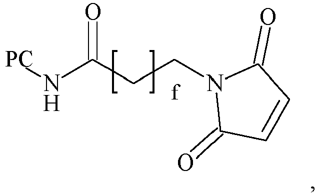 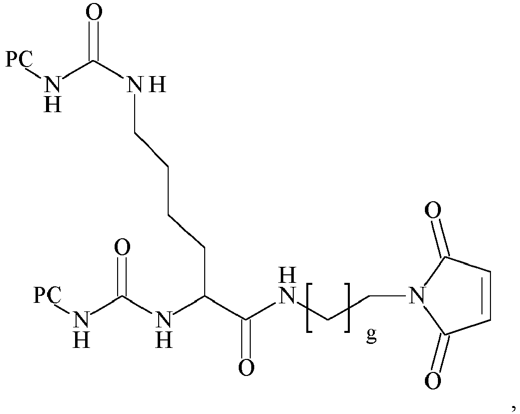 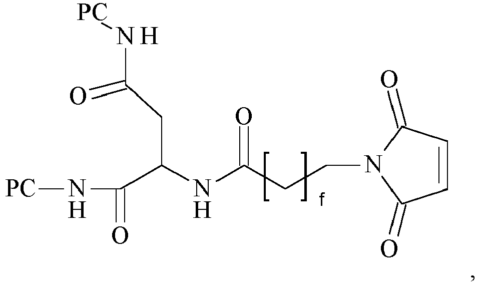 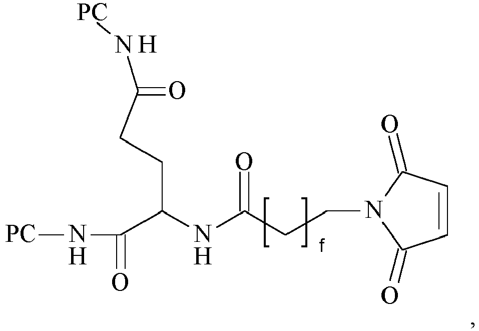 wobei
PC über seinen N-Terminus mit dem Rest des Moleküls verbunden ist, was durch das Amin (-NH-) benachbart zu PC dargestellt ist;
g ausgewählt ist aus 1, 2, 3, 4, 5, 6, 7, 8, 9 und 10, g bevorzugt ausgewählt ist aus 1, 2, 3, 4, 5 und 6, g am meisten bevorzugt 1, 2 oder 3 ist;
f ausgewählt ist aus 0, 1, 2, 3, 4, 5, 6, 7 und 8, f bevorzugt ausgewählt ist aus 0, 1, 2 und 3; 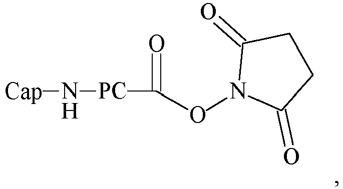 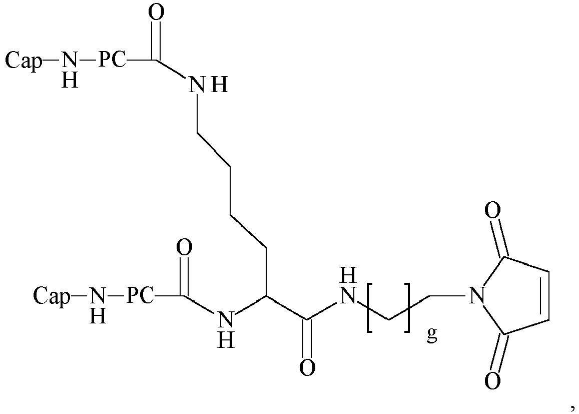
und 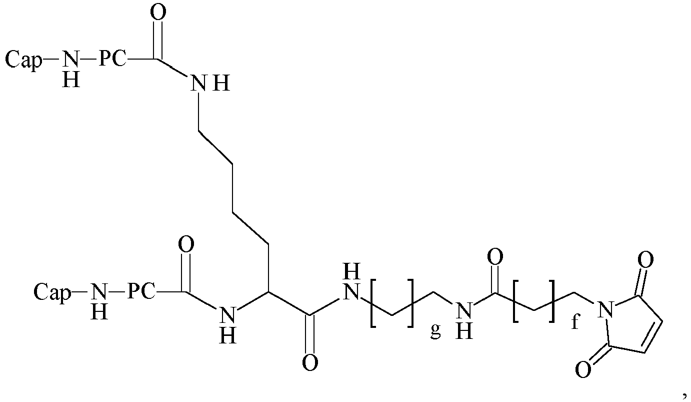 wobei
PC über seinen C-Terminus mit dem Rest des Moleküls verbunden ist, was durch das -C(O)- benachbart zu PC dargestellt ist;
g ausgewählt ist aus 1, 2, 3, 4, 5, 6, 7, 8, 9 und 10, g bevorzugt ausgewählt ist aus 1, 2, 3, 4, 5 und 6, g am meisten bevorzugt aus 1, 2 oder 3 ist;
f ausgewählt ist aus 0, 1, 2, 3, 4, 5, 6, 7 und 8, f bevorzugt ausgewählt ist aus 0, 1, 2 und 3; und
Cap eine Verkappungsgruppe ist, welche die N-terminale Amingruppe blockiert, was durch das -NH- benachbart zu PC dargestellt ist, und C₁₋₂₀-Alkanoyl ist.

17. Das Proteinträger-Reagenz nach Anspruch 16, wobei Cap ausgewählt ist aus der Gruppe bestehend aus Formyl, Acetyl, linearem oder verzweigtem Propanoyl, Butanoyl, Pentanoyl, Hexanoyl, Heptanoyl, Octanoyl, Nonanoyl und Decanoyl.

## Revendications

1. Réactif de support de protéine de formule (A) ou (B) :
PC-SP-LG (A),
(PC)ₚ-SP-LG (B),
dans lequel
chaque PC est individuellement un fragment de support de protéine,
dans lequel un fragment de support de protéine comprend, de préférence est constitué d'une séquence d'acides aminés constituée d'au moins 100 résidus d'acide aminé, et
dans lequel la séquence d'acides aminés d'au moins 100 résidus d'acide aminé, de préférence le fragment de support de protéine, est dans une conformation de pelote aléatoire comme déterminé par des mesures de dichroïsme circulaire, et,
dans lequel la séquence d'acides aminés d'au moins 100 résidus d'acide aminé, de préférence le fragment de support de protéine, comprend des résidus alanine, sérine et proline,
SP est un fragment d'espaceur comprenant q points de ramification BP,
p est un entier de 1 à 32, de préférence de 1 à 16, plus préférablement de 1 à 10,
encore plus préférablement p est choisi parmi 1, 2, 3, 4, 5, 6, 7, 8, 9 et 10,
q est un entier de 0 à 32, de préférence de 0 à 16, plus préférablement de 2 à 8, de manière préférée entre toutes q est 3,
LG est un groupe partant ou un électrophile choisi parmi les formules (a) à (f) : 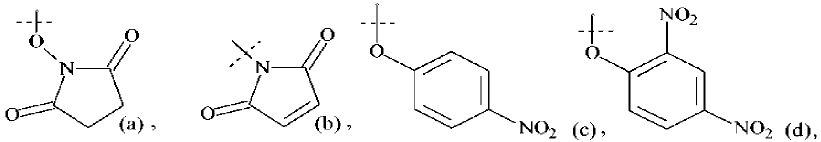 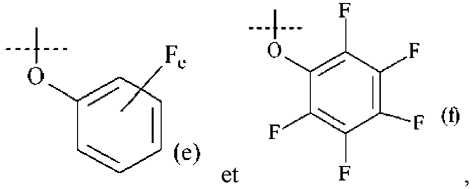
dans lesquelles
les traits pointillés indiquent la liaison au reste de la molécule, et
e est 1, 2, 3 ou 4.

2. Réactif de support de protéine selon la revendication 1, dans lequel un fragment PC comprend une pluralité de répétitions d'acide aminé,
dans lequel lesdites répétitions sont constituées de résidus Ala, Ser et Pro,
et dans lequel pas plus de 6 résidus d'acide aminé consécutifs du fragment de support PC sont identiques.

3. Réactif de support de protéine selon la revendication 1 ou 2, dans lequel lesdits résidus proline constituent plus de 4 % et moins de 40 % des acides aminés du fragment de support de protéine PC.

4. Réactif de support de protéine selon l'une quelconque des revendications 1 à 3, dans lequel le fragment de support de protéine PC comprend au moins une séquence d'acides aminés choisie dans le groupe constitué de :
ASPAAPAPASPAAPAPSAPA (SEQ ID NO : 9),
AAPASPAPAAPSAPAPAAPS (SEQ ID NO : 10),
APSSPSPSAPSSPSPASPSS (SEQ ID NO : 11),
SSPSAPSPSSPASPSPSSPA (SEQ ID NO : 12),
AASPAAPSAPPAAASPAAPSAPPA (SEQ ID NO : 13), et
ASAAAPAAASAAASAPSAAA (SEQ ID NO : 14) ;
et des versions de permutation circulaire ou des multimères de ces séquences.

5. Réactif de support de protéine selon l'une quelconque des revendications 1 à 4, dans lequel un fragment PC comprend, en particulier est constitué de, une séquence d'acides aminés de 100 à 3000 résidus d'acide aminé formant une conformation de pelote aléatoire.

6. Réactif de support de protéine selon l'une quelconque des revendications 1 à 5, dans lequel PC comporte au maximum 1500 acides aminés.

7. Réactif de support de protéine selon l'une quelconque des revendications 1 à 6, dans lequel PC comporte au maximum 900 acides aminés.

8. Réactif de support de protéine selon l'une quelconque des revendications 1 à 7, dans lequel PC comprend des acides aminés supplémentaires différents de l'alanine, la sérine, et la proline en tant que constituants mineurs, lesdits constituants mineurs représentant au maximum 10 % des acides aminés de PC.

9. Réactif de support de protéine selon l'une quelconque des revendications 1 à 8, dans lequel PC ne comprend pas plus de 4 résidus d'acide aminé consécutifs identiques.

10. Réactif de support de protéine selon l'une quelconque des revendications 1 à 9, dans lequel PC comprend plus de 10 % mais moins de 50 % de résidus alanine.

11. Réactif de support de protéine selon l'une quelconque des revendications 1 à 10, dans lequel PC comprend plus de 10 % mais moins de 50 % de résidus sérine.

12. Réactif de support de protéine selon l'une quelconque des revendications 1 à 11, dans lequel PC comprend 35 % de résidus proline, 50 % de résidus alanine et 15 % de résidus sérine.

13. Réactif de support de protéine selon l'une quelconque des revendications 1 à 12, dans lequel LG est choisi parmi la formule (a) ou (b).

14. Réactif de support de protéine selon l'une quelconque des revendications 1 à 13, dans lequel PC dans la formule (B) est lié par l'intermédiaire de son extrémité N- ou C-terminale ou par l'intermédiaire de l'une de ses chaînes latérales à SP.

15. Réactif de support de protéine selon l'une quelconque des revendications 1 à 14, dans lequel SP est choisi dans le groupe constitué d' alkyle en C₁₋₅₀, alcényle en C₂₋₅₀ et alcynyle en C₂₋₅₀, ledit fragment étant facultativement interrompu par un ou plusieurs groupes choisis dans le groupe constitué de -NH-, -N(alkyle en C₁₋₄)-, -O-, -S-, -C(O)-, -C(O)NH-, -C(O)N(alkyle en C₁₋₄)-, -O-C(O)-, -S(O)-, -S(O)₂-, hétérocyclyle de 4 à 7 chaînons, phényle, naphtyle, carbocycles substitués au moins 3 fois, hétérocycles substitués au moins 3 fois, un atome de carbone tertiaire, un atome de carbone quaternaire et un atome d'azote tertiaire, et ledit fragment étant facultativement substitué.

16. Réactif de support de protéine selon l'une quelconque des revendications 1 à 15, le réactif de support de protéine étant choisi parmi l'une des structures suivantes : 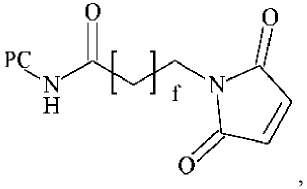 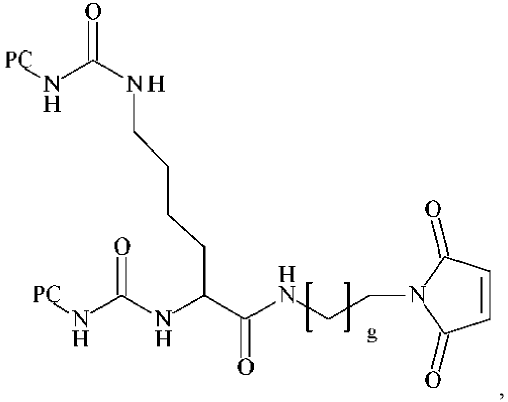 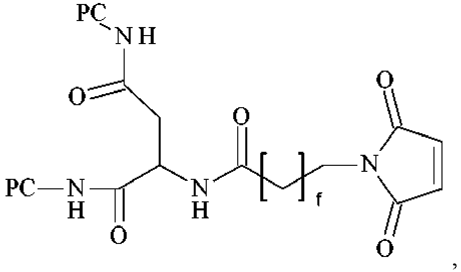 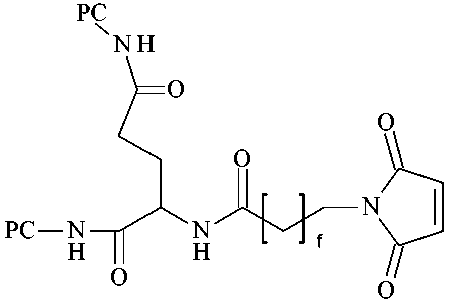 dans lequel
PC est lié au reste de la molécule par l'intermédiaire de son extrémité N-terminale qui est représentée comme étant l'amine (-NH-) adjacente à PC ;
g est choisi parmi 1, 2, 3, 4, 5, 6, 7, 8, 9 et 10, de préférence, g est choisi parmi 1, 2, 3, 4, 5 et 6, de manière préférée entre toutes, g est 1, 2 ou 3 ;
f est choisi parmi 0, 1, 2, 3, 4, 5, 6, 7 et 8, de préférence, f est choisi parmi 0, 1, 2, et 3 ; 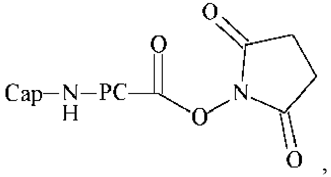 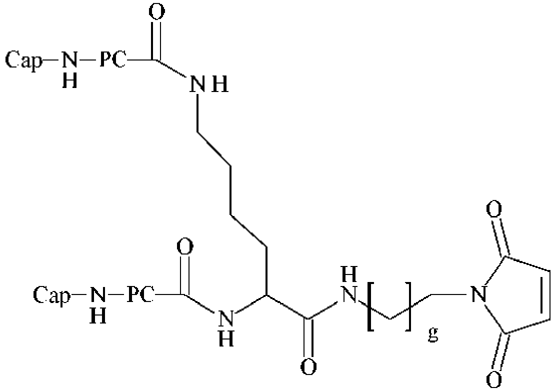 et 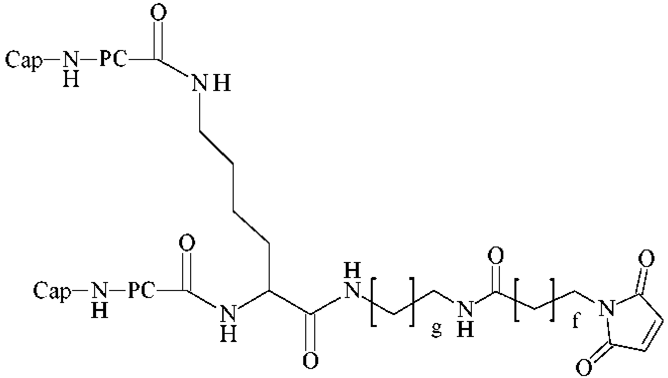
dans lequel
PC est lié au reste de la molécule par l'intermédiaire de son extrémité C-terminale qui est représentée comme étant le -C(O)- adjacent à PC ;
g est choisi parmi 1, 2, 3, 4, 5, 6, 7, 8, 9 et 10, de préférence, g est choisi parmi 1, 2, 3, 4, 5 et 6, de manière préférée entre toutes, g est 1, 2 ou 3 ;
f est choisi parmi 0, 1, 2, 3, 4, 5, 6, 7 et 8, de préférence, f est choisi parmi 0, 1, 2 et 3 ; et
Cap est un groupe de coiffe bloquant le groupe amino N-terminal qui est représenté comme étant le -NH- adjacent à PC et est alcanoyle en C₁₋₂₀.

17. Réactif de support de protéine selon la revendication 16, dans lequel Cap est choisi dans le groupe constitué de formyle, acétyle, propanoyle, butanoyle, pentanoyle, hexanoyle, heptanoyle, octanoyle, nonanoyle et décanoyle linéaires ou ramifiés.


### SEQUENCE LISTING

<110> Ascendis Pharma A/S
<120> Protein Carrier-Linked Prodrugs
<130> CP67815PC
<160> 15
<170> PatentIn version 3.5
<210> 1
   <211> 20
   <212> PRT
   <213> Artificial Sequence
<220>
   <223> polymer cassette
<400> 1
   Ala Ala Ala Ala Ser Ser Ala Ser Ser Ala Ser Ser Ser Ser Ser Ala
   1               5                   10                  15
   Ala Ala Ser Ala
               20
<210> 2
   <211> 20
   <212> PRT
   <213> Artificial Sequence
<220>
   <223> polymer cassette
<400> 2
   Ala Ala Ser Ala Ala Ala Ser Ser Ala Ala Ala Ser Ala Ala Ala Ala
   1               5                   10                  15
   Ser Ala Ser Ser
               20
<210> 3
   <211> 20
   <212> PRT
   <213> Artificial Sequence
<220>
   <223> polymer cassette
<400> 3
   Ala Ser Ala Ser Ala Ser Ala Ser Ala Ser Ala Ser Ser Ala Ala Ser
   1               5                   10                  15
   Ala Ala Ser Ala
                                 20
<210> 4
   <211> 20
   <212> PRT
   <213> Artificial Sequence
<220>
   <223> polymer cassette
<400> 4
   Ser Ala Ala Ser Ser Ser Ala Ser Ser Ser Ser Ala Ala Ser Ser Ala
   1               5                   10                  15
   Ser Ala Ala Ala
               20
<210> 5
   <211> 20
   <212> PRT
   <213> Artificial Sequence
<220>
   <223> polymer cassette
<400> 5
   Ser Ser Ser Ser Ala Ala Ser Ala Ala Ser Ala Ala Ala Ala Ala Ser
   1               5                   10                  15
   Ser Ser Ala Ser
               20
<210> 6
   <211> 20
   <212> PRT
   <213> Artificial Sequence
<220>
   <223> polymer cassette
<400> 6
   Ser Ser Ala Ser Ser Ser Ala Ala Ser Ser Ser Ala Ser Ser Ser Ser
   1               5                   10                  15
   Ala Ser Ala Ala
               20
<210> 7
   <211> 20
   <212> PRT
   <213> Artificial Sequence
<220>
   <223> polymer cassette
<400> 7
   Ser Ala Ser Ala Ser Ala Ser Ala Ser Ala Ser Ala Ala Ser Ser Ala
   1               5                   10                  15
   Ser Ser Ala Ser
               20
<210> 8
   <211> 20
   <212> PRT
   <213> Artificial Sequence
<220>
   <223> polymer cassette
<400> 8
   Ala Ser Ser Ala Ala Ala Ser Ala Ala Ala Ala Ser Ser Ala Ala Ser
   1               5                   10                  15
   Ala Ser Ser Ser
               20
<210> 9
   <211> 20
   <212> PRT
   <213> Artificial Sequence
<220>
   <223> polymer cassette
<400> 9
   Ala Ser Pro Ala Ala Pro Ala Pro Ala Ser Pro Ala Ala Pro Ala Pro
   1               5                   10                  15
   Ser Ala Pro Ala
               20
<210> 10
   <211> 20
   <212> PRT
   <213> Artificial Sequence
<220>
   <223> polymer cassette
<400> 10
   Ala Ala Pro Ala Ser Pro Ala Pro Ala Ala Pro Ser Ala Pro Ala Pro
   1               5                   10                  15
   Ala Ala Pro Ser
         20
<210> 11
   <211> 20
   <212> PRT
   <213> Artificial Sequence
<220>
   <223> polymer cassette
<400> 11
   Ala Pro Ser Ser Pro Ser Pro Ser Ala Pro Ser Ser Pro Ser Pro Ala
   1 5 10 15
   Ser Pro Ser Ser
   20
<210> 12
   <211> 20
   <212> PRT
   <213> Artificial Sequence
<220>
   <223> polymer cassette
<400> 12
   Ser Ser Pro Ser Ala Pro Ser Pro Ser Ser Pro Ala Ser Pro Ser Pro
   1 5 10 15
   Ser Ser Pro Ala
   20
<210> 13
   <211> 24
   <212> PRT
   <213> Artificial Sequence
<220>
   <223> polymer cassette
<400> 13
   Ala Ala Ser Pro Ala Ala Pro Ser Ala Pro Pro Ala Ala Ala Ser Pro
   1 5 10 15
   Ala Ala Pro Ser Ala Pro Pro Ala
   20
<210> 14
   <211> 20
   <212> PRT
   <213> Artificial Sequence
<220>
   <223> polymer cassette
<400> 14
   Ala Ser Ala Ala Ala Pro Ala Ala Ala Ser Ala Ala Ala Ser Ala Pro
   1 5 10 15
   Ser Ala Ala Ala
   20
<210> 15
   <211> 20
   <212> PRT
   <213> Artificial Sequence
<220>
   <223> polymer cassette
<400> 15
   Ser Ala Pro Ser Ser Pro Ser Pro Ser Ala Pro Ser Ser Pro Ser Pro
   1               5                   10                  15
   Ala Ser Pro Ser
               20